# EUROPEAN PATENT APPLICATION

(11) **EP 4 527 856 A1**
(43) Date of publication of application: **26.03.2025**
(21) Application number: 23807948.7
(22) Date of filing: 19.05.2023
(51) Int. Cl.: C07K 16/40, C07K 14/525, A61P 35/00, A61K 39/00

(54) **FUSION PROTEIN COMPRISING LIGHT PROTEIN AND ANTI-FAP ANTIBODY AND USES THEREOF**

(30) Priority: 20.05.2022 KR 20220062018
(71) Applicant: Kanaph Therapeutics Inc., Seoul 04348 (KR)
(72) Inventor: LEE, Dahea, Seoul 03938 (KR); CHANG, Jihoon, Seoul 04211 (KR); LEE, Byoung Chul, Seoul 06289 (KR)
(74) Representative: HGF
(86) International application number: PCT/KR2023/006839
(87) International publication number: WO 2023/224429

(57) **Abstract**

A fusion protein comprising a LIGHT protein and an antigen-binding domain that specifically binds to FAP may exhibit anti-cancer effects. In particular, the fusion protein efficiently targets cancer cells which overexpress FAP, and LIGHT may bind to a lymphotoxin beta receptor and HVEM to promote the formation of tertiary lymphoid structures and normalize blood vessels. Accordingly, the fusion protein enables effective treatment of cancer through efficient infiltration of immune cells into tumor tissue. Furthermore, it was confirmed that the fusion protein of the present invention can treat cancer more effectively when administered in combination with an anti-PD-1 antibody compared to when administered individually.

## Description

### Technical Field

The present invention relates to a novel fusion protein comprising a LIGHT protein, a fragment thereof, or a variant thereof; and an anti-FAP antibody, and an anti-cancer pharmaceutical composition comprising the same.

### Background Art

LIGHT protein, also known as TNFSF14, is a glycoprotein that exists as a homotrimer of the TNF superfamily. LIGHT protein is expressed on activated lymphocytes, NK cells, immature dendritic cells, monocytes, and granulocytes, and binds to and activates herpes virus entry mediator (HVEM/TNFRSF14) and lymphotoxin beta receptor (LTβR). By this mechanism, it co-stimulates Th1 immune response, enhances CD8+ T cell-mediated tumor immunity, activates T cells, and modulates allograft rejection. In particular, LIGHT protein is known to be involved in the activation of lymphoid cells, normalization of blood vessels, and inhibition of herpes virus infection.

Meanwhile, fibroblast activation protein alpha (FAPα) is a gelatinase expressed on activated fibroblasts. FAP is known to be expressed in more than 90% of cancer-related fibroblasts of various human cancers, including prostate cancer and pancreatic cancer. Accordingly, interest in developing immunotherapy targeting FAP-expressing cells has been rapidly increasing recently (Fang J. et.al., Mol. Ther. Oncolytics., 3:16007, 2016).

### Detailed Description of Invention

### Technical Problem

Accordingly, the present inventors conducted research to develop a novel fusion protein having an anti-cancer effect. As a result, it was found that the fusion protein comprising the LIGHT protein and the anti-FAP antibody had increased anti-cancer activity while having reduced systemic toxicity. Based on the above, the present inventors completed the present invention.

### Solution to Problem

In one aspect of the present invention, provided is a fusion protein comprising an antigen binding domain that specifically binds to FAP (fibroblast activation protein alpha); and a LIGHT protein, a fragment thereof, or a variant thereof.

In another aspect of the present invention, provided is a pharmaceutical composition for preventing or treating cancer, comprising the fusion protein as an active ingredient.

In another aspect of the present invention, provided is a method for preventing or treating cancer, comprising administering the pharmaceutical composition to a subject.

In another aspect of the present invention, provided is a use of the fusion protein for the prevention or treatment of cancer.

### Effects of Invention

A fusion protein comprising a LIGHT protein and an antigen binding domain that specifically binds to FAP may exhibit anti-cancer effects. In particular, the fusion protein may efficiently target cancer cells which overexpress FAP, promote the formation of tertiary lymphoid structures by binding a LIGHT protein to a lymphotoxin beta receptor and a herpes virus entry mediator, and normalize blood vessels. Accordingly, the fusion protein enables effective treatment of cancer through efficient infiltration of immune cells into tumor tissue. Furthermore, it was confirmed that the fusion protein of the present invention may treat cancer more effectively when administered in combination with an anti-PD-1 antibody compared to when administered individually.

### Brief Description of Drawings

Figure 1 shows the results of SDS-PAGE for T3.01, T3.02, T3.03, T3.04, T3.05, and T3.06 according to one embodiment.
Figure 2 shows the results of SDS-PAGE for T3.07, T3.08, T3.09, T3.10, T3.11, and T3.13 according to one embodiment.
Figure 3 shows the results of SDS-PAGE for T3.14, T3.15, T3.16, T3.17, and T3.18 according to one embodiment.
Figure 4 shows the results of SDS-PAGE for T3.01m, T3.02m, T3.05m, T3.06m, T3.07m, and T3.08m according to one embodiment.
Figure 5 shows the results of SDS-PAGE for T3.10m, T3.11m, T3.12m, T3.13m, T3.14m, and T3.19m according to one embodiment.
Figure 6 shows a schematic diagram of a) an anti-FAP/scLIGHT3 bispecific antibody, b) an anti-FAP/scLIGHT3 bispecific antibody in which LIGHT is bound to the C-terminus of Fc, c) an anti-FAP/scLIGHT2 bispecific antibody in which LIGHT is bound to the C-terminus of Fc, d) an anti-FAP/scLIGHT-LTβ2 bispecific antibody according to one embodiment.
Figures 7a to 7c are graphs showing the binding affinities of T3.01, T3.07, and T3.14 for recombinant human FAP (rhFAP) according to one embodiment through a surface plasmon resonance analysis method.
Figures 8a to 8e are graphs showing the binding affinities of T3.01, T3.07, T3.09, T3.14, and T3.18 for LIGHT receptor LTβR according to one embodiment through a surface plasmon resonance analysis method.
Figures 9a to 9e are graphs showing the binding affinities of T3.01, T3.07, T3.09, T3.14, and T3.18 for LIGHT receptor HVEM according to one embodiment through a surface plasmon resonance analysis method.
Figures 10a to 10e are graphs showing the binding affinities of T3.01, T3.07, T3.09, T3.14, and T3.18 for LIGHT receptor DcR3 according to one embodiment through a surface plasmon resonance analysis method.
Figure 11 is a graph showing the binding affinities of T3.10, T3.01, T3.05, T3.06, T3.14, and T3.18 for HEK293-hFAP cells and HEK293 cells through flow cytometry.
Figure 12 is a graph showing the binding affinities of T3.10m and T3.01m for B16F10-mFAP cells and B16F10 cells through flow cytometry.
Figure 13a is a graph showing the binding affinities of T3.10, T3.01, T3.07, T3.04, and T3.18 for A375 cells through flow cytometry.
Figure 13b is a graph showing the binding affinities of T3.10, T3.01, T3.07, T3.04, and T3.18 for A375 cells for each concentration as mean fluorescence intensity (MFI).
Figure 14a is a graph showing the binding affinities of T3.10, T3.01, T3.14, T3.16, T3.17, and T3.18 for herpes virus entry mediator (HVEM) through enzyme-linked immunoprecipitation assay.
Figure 14b is a graph showing the binding affinities of T3.10, T3.01, T3.14, T3.16, T3.17, and T3.18 for lymphotoxin beta receptor (LTβR) through enzyme-linked immunoprecipitation assay.
Figure 14c is a graph showing the binding affinities of T3.10, T3.05, T3.06, T3.04, and T3.14 for herpes virus entry mediator through enzyme-linked immunoprecipitation assay.
Figure 14d is a graph showing the binding affinities of T3.10, T3.05, T3.06, T3.04, and T3.14 for lymphotoxin beta receptor through enzyme-linked immunoprecipitation assay.
Figure 15a is a graph showing the results obtained by measuring the ability of A375 cell line to secrete cytokine IL-8 by rhLIGHT, T3.01, T3.04, T3.14, T3.15, and T3.10.
Figure 15b is a graph showing the results obtained by measuring the ability of A375 cell line to secrete cytokine IL-8 by rhLIGHT, T3.01, T3.07, T3.18, and T3.10.
Figure 16a is a graph measuring the ability of human T cells to secrete cytokine IFN-γ by T3.01, T3.04, T3.14, T3.15, and T3.10.
Figure 16b is a graph measuring the ability of human T cells to secrete cytokine IFN-γ by T3.01, T3.07, T3.04, T3.18, and T3.10.
Figure 17a is a graph confirming the LIGHT activity of TNF-α, T3.01m, and T3.10m through luminescence degree in NIH-3T3 NFκB reporter cells.
Figure 17b is a graph confirming the LIGHT activity of T3.10, T3.01, T3.04, T3.14, and T3.15 through luminescence degree in NIH-3T3 NFκB reporter cells.
Figure 18a is a graph measuring the tumor size of each subject in order to confirm the degree of tumor growth inhibition by T3.01m, anti-PD-1 antibody and T3.01m/anti-PD-1 antibody according to one embodiment in a mouse tumor model transplanted with FAP-overexpressing colorectal cancer CT26-mFAP cell line.
Figure 18b is a graph showing the distribution of immune cells in tumor by isolating immune cells from tumor tissues harvested from mice on day 12 after treatment with T3.01m, anti-PD-1 antibody and T3.01m/anti-PD-1 antibody according to one embodiment in a mouse tumor model transplanted with FAP-overexpressing colorectal cancer CT26-mFAP cell line through flow cytometry.
Figure 18c is a graph showing the CCR7 expression level of immune cells in tumor by isolating immune cells from tumor tissues harvested from mice on day 12 after treatment with T3.01m, anti-PD-1 antibody and T3.01m/anti-PD-1 antibody according to one embodiment in a mouse tumor model transplanted with FAP-overexpressing colorectal cancer CT26-mFAP cell line through flow cytometry.
Figure 18d shows the expression of MECA-79, a marker of high endothelial venules (HEV) in tumor tissues harvested from mice on day 12 after treatment with T3.01m, anti-PD-1 antibody and T3.01m/anti-PD-1 antibody according to one embodiment in a mouse tumor model transplanted with FAP-overexpressing colorectal cancer CT26-mFAP cell line through immunohistochemical staining.
Figure 18e is a graph showing the results quantifying the expression of CD3, CD19, and MECA-79, a marker of high endothelial venules, in tumor tissues harvested from mice on day 12 after treatment with T3.01m, anti-PD-1 antibody and T3.01m/anti-PD-1 antibody according to one embodiment in a mouse tumor model transplanted with FAP-overexpressing colorectal cancer CT26-mFAP cell line through immunohistochemical staining.
Figure 19a is a graph showing the results measuring the tumor size of each mouse in order to confirm the degree of dose-dependent tumor growth inhibition of T3.01m according to one embodiment in a mouse tumor model transplanted with FAP-overexpressing colorectal cancer CT26-mFAP cell line.
Figure 19b is a graph showing the results measuring the change in tumor size in order to confirm the degree of dose-dependent tumor growth inhibition of T3.01m according to one embodiment in a mouse tumor model transplanted with FAP-overexpressing colorectal cancer CT26-mFAP cell line.
Figure 19c is a graph showing the results measuring the weight of the mouse in order to confirm the side effects after treatment with T3.01m according to one embodiment in a mouse tumor model transplanted with FAP-overexpressing colorectal cancer CT26-mFAP cell line.
Figure 20a is a graph showing the results measuring the change in tumor size of each mouse after treatment with T3.01m, anti-PD-1 antibody and T3.01m/anti-PD-1 antibody according to one embodiment in a mouse model transplanted with FAP-overexpressing melanoma B16F10-mFAP cell line.
Figure 20b is a graph showing the results measuring the change in tumor size after treatment with T3.01m, anti-PD-1 antibody and T3.01m/anti-PD-1 antibody according to one embodiment in a mouse model transplanted with FAP-overexpressing melanoma B16F10-mFAP cell line.
Figure 20c is a graph showing the results measuring the weight of the mouse in order to confirm the side effects after treatment with T3.01m according to one embodiment in a mouse model transplanted with FAP-overexpressing melanoma B16F10-mFAP cell line.
Figure 20d is a graph showing the results measuring the weight of tumor tissue harvested from mice on day 12 after treatment with T3.01m, anti-PD-1 antibody and T3.01m/anti-PD-1 antibody according to one embodiment in a mouse model transplanted with FAP-overexpressing melanoma B16F10-mFAP cell line.
Figure 20e shows the expression of MECA-79, a marker of high endothelial venules, and the distribution of immune cells in tumor tissues harvested from mice on day 12 after treatment with T3.01m, anti-PD-1 antibody and T3.01m/anti-PD-1 antibody according to one embodiment in a mouse model transplanted with FAP-overexpressing melanoma B16F10-mFAP cell line through immunohistochemical staining.
Figure 20f is a graph showing the results quantifying the expression of MECA-79, a marker of high endothelial venules, and the distribution of immune cells in tumor tissues harvested from mice on day 12 after treatment with T3.01m, anti-PD-1 antibody and T3.01m/anti-PD-1 antibody according to one embodiment in a mouse model transplanted with FAP-overexpressing melanoma B16F10-mFAP cell line through immunohistochemical staining.
Figure 20g shows the expression of MECA-79, a marker of high endothelial venules, and the distribution of immune cells in tumor tissues harvested from mice on day 12 after treatment with T3.01m, anti-PD-1 antibody and T3.01m/anti-PD-1 antibody according to one embodiment in a mouse model transplanted with FAP-overexpressing melanoma B16F10-mFAP cell line through fluorescent immunohistochemical staining.
Figure 20h is a graph showing the results quantifying the expression of MECA-79, a marker of high endothelial venules, and the distribution of immune cells in tumor tissues harvested from mice on day 12 after treatment with T3.01m, anti-PD-1 antibody and T3.01m/anti-PD-1 antibody according to one embodiment in a mouse model transplanted with FAP-overexpressing melanoma B16F10-mFAP cell line through fluorescent immunohistochemical staining.
Figure 21 is a graph showing the tumor size after treatment by individual or simultaneous administration of T3.13m, T3.11m, T3.01m or anti-PD1 according to one embodiment in a mouse tumor model co-injected with FAP-overexpressing fibroblast NIH-3T3-mFAP and lung cancer cell line LLC1.
Figure 22 is a table showing an example of a heterotrimer comprising LIGHT and lymphotoxin (LT) protein.

### Mode for Carrying out the Invention

### Fusion protein comprising FAP antigen binding domainand LIGHT protein

In one aspect of the present invention, provided is a fusion protein comprising an antigen binding domainthat specifically binds to FAP (fibroblast activation protein alpha); and a LIGHT protein, a fragment thereof, or a variant thereof. Specifically, the fusion protein may comprise an immunoglobulin Fc region.

The fusion protein may be a fusion protein comprising a first monomer comprising an antigen binding domain that specifically binds to FAP; and a second monomer comprising a LIGHT protein, a fragment thereof, or a variant thereof.

In one embodiment, the first monomer may further comprise a LIGHT protein, a fragment thereof, or a variant thereof. At this time, the LIGHT protein, a fragment thereof, or a variant thereof may be linked to the N-terminus or C-terminus of the first monomer.

In one embodiment, the second monomer may further comprise an antigen binding domain that specifically binds to FAP. At this time, the antigen binding domain that specifically binds to FAP may be linked to the N-terminus or C-terminus of the second monomer.

### LIGHT protein, fragment thereof or variant thereof

As used herein, the term "LIGHT protein" is also known as TNFSF14 and is a glycoprotein that exists as a homotrimer. The LIGHT protein may bind to HVEM/TNFRSF14 and activate it to enhance an immune response. In addition, the LIGHT protein may bind to LTβ receptor/TNFRSF3 to enhance cytokine production, formation of lymph node tissue, and maintenance of lymph node structure and function. In the present specification, the LIGHT protein may refer to a monomer constituting a homotrimer, a fragment thereof, or a variant thereof. In addition, the LIGHT protein may be used in the sense of including a LIGHT monomer, a LIGHT dimer, or a LIGHT trimer.

In one embodiment, the LIGHT protein may be hydrophilic.

As used herein, the term "herpes virus entry mediator (HVEM)" is an infection mediating receptor of herpes simplex virus (HSV). The HSV glycoprotein binds to the HVEM receptor. The LIGHT protein may bind to HVEM/TNFRSF14 to enhance an immune response and promote T cell activation.

In the present specification, the LIGHT protein may include a monomer of the LIGHT protein, a fragment thereof, or a variant thereof. At this time, the LIGHT protein may be a monomer of the LIGHT protein comprising the amino acid sequence of SEQ ID NO: 77 or SEQ ID NO: 92, or a fragment thereof.

In addition, the LIGHT protein may comprise one amino acid selected from the group consisting of SEQ ID NO: 78 and SEQ ID NO: 93.

In addition, the variant of the LIGHT protein or a fragment thereof may have 1, 2, 3, 4, 5, 6, 7, 8, 9, 10, 11, or 12 substitutions in the amino acid sequence of SEQ ID NO: 77, SEQ ID NO: 78, SEQ ID NO: 92, or SEQ ID NO: 93. In addition, it may have about 85%, about 86%, about 87%, about 88%, about 89%, about 90%, about 91%, about 92%, about 93%, about 94%, about 95%, about 96%, about 97%, about 98%, about 99%, or about 100% identity to the amino acid sequence of SEQ ID NO: 78 or SEQ ID NO: 93.

The variant of the LIGHT protein or fragment thereof may comprise an amino acid sequence in which at least one amino acid selected from the group consisting of the 118th, 119th, 195th, 196th, 198th, and 226th to 231st amino acids in the amino acid sequence of SEQ ID NO: 78 is substituted with another amino acid.

In one embodiment, the variant of the LIGHT protein or a fragment thereof may be one in which (i) 226th to 231st amino acids; (ii) 118th to 119th amino acids; or (iii) 195th, 196th and 198th amino acids in the amino acid sequence of SEQ ID NO: 78 are substituted with another amino acid.

In one embodiment, the variant of the LIGHT protein or a fragment thereof may have at least one substitution selected from the group consisting of L118K, G119E, R195S, V196N, W198F, R226D, L227Y, R228T, D229K, G230E and T231D in the amino acid sequence of SEQ ID NO: 78.

In one embodiment, the variant of the LIGHT protein or a fragment thereof may have an amino acid substitution of (i) R226D/L227Y/R228T/D229K/G230E/T231D; (ii) L118K/G119E; or (iii) R195S/V196N/W198F in the amino acid sequence of SEQ ID NO: 78. At this time, the variant of the LIGHT protein or a fragment thereof may have the amino acid sequence of SEQ ID NO: 81, SEQ ID NO: 83, or SEQ ID NO: 85.

In addition, the variant of the LIGHT protein or a fragment thereof may comprise an amino acid sequence in which at least one amino acid selected from the group consisting of the 194th, 195th, 197th, and 225th to 230th amino acids in the amino acid sequence of SEQ ID NO: 93 is substituted with another amino acid.

In one embodiment, the variant of the LIGHT protein or a fragment thereof may be one in which (i) 194th, 195th, and 197th amino acids or (ii) 225th to 230th amino acids in the amino acid sequence of SEQ ID NO: 93 are substituted with another amino acid.

In one embodiment, the variant of the LIGHT protein or a fragment thereof may have at least one substitution selected from the group consisting of R194N, V195N, W197F, R225D, P226Y, R227T, D228K, G229E, and T230D in the amino acid sequence of SEQ ID NO: 93.

In one embodiment, the variant of the LIGHT protein or a fragment thereof may have an amino acid substitution of (i) R194N/V195N/W197F or (ii)
R225D/P226Y/R227T/D228K/G229E/T230D in the amino acid sequence of SEQ ID NO: 93. At this time, the variant of the LIGHT protein or a fragment thereof may have the amino acid sequence of SEQ ID NO: 96 or SEQ ID NO: 98.

The variant of the LIGHT protein includes a homomultimer of the LIGHT protein or a fragment thereof.

As used herein, the term "homomultimer" refers to a complex in which multiple proteins of the same type are bound. The protein may be a homodimer in which two proteins of the same type are linked, or a homotrimer in which three proteins of the same type are linked. In the present specification, a complex in which multiple non-identical LIGHT protein monomers are bound is also defined as a homomultimer. For example, a complex in which a LIGHT protein and a variant thereof are bound is also a homomultimer.

In one embodiment, a homomultimer of the LIGHT protein may be a homodimer or a homotrimer.

The homomultimer may be a homomultimer comprising a monomer composed of the amino acid sequence of SEQ ID NO: 78, SEQ ID NO: 81, SEQ ID NO: 83, SEQ ID NO: 85, SEQ ID NO: 93, SEQ ID NO: 96, or SEQ ID NO: 98. Specifically, it may be a homodimer or a homotrimer.

In one embodiment, the homomultimer may comprise at least one amino acid sequence selected from the group consisting of SEQ ID NO: 79, SEQ ID NO: 80, SEQ ID NO: 82, SEQ ID NO: 84, SEQ ID NO: 86, SEQ ID NO: 94, SEQ ID NO: 95, SEQ ID NO: 97, and SEQ ID NO: 99.

In addition, the LIGHT protein may be one in which an LT (lymphotoxin) protein or a variant thereof is additionally bound. Specifically, the LIGHT protein may be a heteromultimer in which LTα (lymphotoxin-α) or LTβ (lymphotoxin-β) protein is bound to the LIGHT protein.

As used herein, the term "LT (lymphotoxin) protein" is a member of the tumor necrosis factor (TNF) superfamily, which regulates the growth and function of lymphocytes and is expressed by various cells in the body. The LT protein may be composed of LTα (lymphotoxin-α) and/or LTβ (lymphotoxin-β). In addition, the LT protein may exist as a trimer. The trimer may be a homotrimer or a heterotrimer, and specifically, it may be LTα3, a homotrimer in which three LTα monomers are bound, or LTβ3, a homotrimer in which three LTβ monomers are bound. In addition, it may be LTα1LTβ2, a heterotrimer in which one LTα monomer and two LTβ monomers are bound. In addition, it may be LTα2LTβ1, a heterotrimer in which two LTα monomers and one LTβ monomer are bound.

In the present specification, the LT protein collectively refers to a LTα or LTβ protein monomer, a fragment thereof, or a variant thereof. In one embodiment, the LT protein may be an LTβ monomer protein.

In one embodiment, the LIGHT protein may be a homomultimer of the LIGHT protein, in which a LT protein or a fragment thereof is bound. At this time, the LT protein may be a monomer of LTα or LTβ protein, or may be a multimer comprising LTα and LTβ proteins.

In one embodiment, the LTβ protein may be a polypeptide having the amino acid sequence of SEQ ID NO: 87 or a fragment thereof. Specifically, the LTβ protein may have the amino acid sequence of SEQ ID NO: 88. In addition, in one embodiment, the LTβ protein may be a polypeptide having the amino acid sequence of SEQ ID NO: 289 or a fragment thereof. Specifically, the LTβ protein may have the amino acid sequence of SEQ ID NO: 290.

In one embodiment, the homodimer of the LT protein or a variant thereof may comprise the amino acid sequence of SEQ ID NO: 288 or SEQ ID NO: 291.

In one embodiment, the LIGHT protein may be one in which a homodimer of the LT protein is bound to a monomer of the LIGHT protein. At this time, the homomultimer of the LIGHT protein may comprise an amino acid sequence selected from the group consisting of SEQ ID NO: 78, SEQ ID NO: 81, SEQ ID NO: 83, SEQ ID NO: 85, SEQ ID NO: 93, SEQ ID NO: 96, and SEQ ID NO: 98, and the homodimer of the LT protein may comprise the amino acid sequence of SEQ ID NO: 288 or SEQ ID NO: 291.

The homomultimer may be linked by a peptide linker. The peptide linker comprises at least one amino acid sequence selected from the group consisting of SEQ ID NO: 120 to SEQ ID NO: 126.

In addition, the heterodimer is the same as described below.

### Heteromultimer of LIGHT protein and LT protein

In one aspect of the present invention, provided is a heteromultimer comprising a fragment of the LIGHT protein or a variant thereof and a fragment of the LT protein or a variant thereof. At this time, the LIGHT protein and the LT protein may be linked by a peptide linker.

The LIGHT protein, the LT protein, the fragment thereof, or the variant thereof, and the multimer are the same as described above.

The heteromultimer may comprise a LIGHT protein and an LTβ protein. Specifically, the heteromultimer may comprise a fragment of the LIGHT protein or a variant thereof comprising at least one amino acid sequence selected from the group consisting of SEQ ID NO: 78, SEQ ID NO: 81, SEQ ID NO: 83, SEQ ID NO: 85, SEQ ID NO: 93, SEQ ID NO: 96, and SEQ ID NO: 98; and a fragment of the LTβ protein or a variant thereof comprising the amino acid sequence of SEQ ID NO: 88 or SEQ ID NO: 290.

The heteromultimer may be a heterodimer comprising a LIGHT protein and an LT protein.

In addition, the heteromultimer may be a heterotrimer comprising a LIGHT protein and an LT protein. Specifically, the heterotrimer may comprise two LIGHT proteins and one LT protein; or one LIGHT protein and two LT proteins.

At this time, the heteromultimer may be composed of the following structural formula (A).
N'-Y1-[linker (1)]n-Y2-[linker (2)]m-(Y3)a-C' (A)
in the structural formula (A),
N' is the N-terminus of the fusion protein,
C' is the C-terminus of the fusion protein,
Y1, Y2, and Y3 are each a fragment of the LIGHT protein or a variant thereof; or a fragment of the LT protein or a variant thereof,
wherein Y1, Y2, and Y3 should comprise at least one fragment of the LIGHT protein or a variant thereof; and a fragment of the LT protein or a variant thereof,
the linkers (1) and (2) are peptide linkers, and
n, m, and a are each independently 0 or 1.

In one embodiment, the heteromultimer may be a heterodimer having a structure of structural formula (A) in which a and m are each 0 and n is 1. At this time, Y1 may be a fragment of the LIGHT protein or a variant thereof, and Y2 may be a fragment of the LT protein or a variant thereof.

In one embodiment, in the heteromultimer, Y1 may be a fragment of the LIGHT protein or a variant thereof, and Y2 may be a fragment of the LTβ protein or a variant thereof. At this time, in the heterodimer, Y1 may comprise the amino acid sequence of SEQ ID NO: 78, SEQ ID NO: 81, SEQ ID NO: 83, SEQ ID NO: 85, SEQ ID NO: 93, SEQ ID NO: 96, or SEQ ID NO: 98, and Y2 may comprise the amino acid sequence of SEQ ID NO: 88 or SEQ ID NO: 290.

In one embodiment, the heteromultimer may be a heterotrimer having a structure of structural formula (A) in which a, n, and m are each 1.

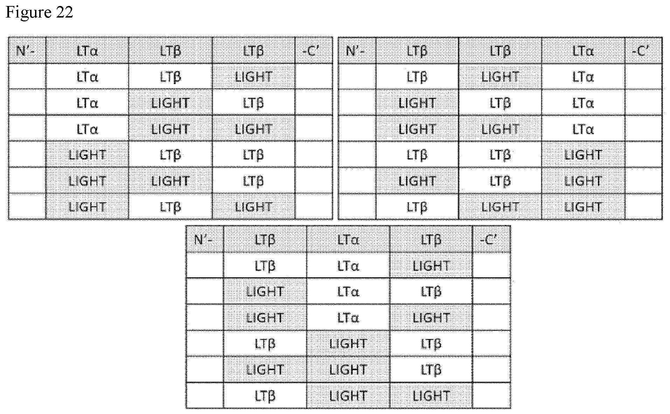

At this time, in the heterotrimer, Y1 to Y3 may be one of the following combinations (see Figure 22):
(i) Y1 and Y2 are a fragment of the LIGHT protein or a variant thereof, and Y3 is a fragment of the LT protein or a variant thereof;
(ii) Y1 is a fragment of the LIGHT protein or a variant thereof, and Y2 and Y3 are a fragment of the LT protein or a variant thereof;
(iii) Y1 is a fragment of the LT protein or a variant thereof, and Y2 and Y3 are a fragment of the LIGHT protein or a variant thereof;
(iv) Y1 and Y2 are a fragment of the LT protein or a variant thereof, and Y3 is a fragment of the LIGHT protein or a variant thereof;
(v) Y1 and Y3 are a fragment of the LIGHT protein or a variant thereof, and Y2 is a fragment of the LT protein or a variant thereof; or
(vi) Y1 and Y3 are a fragment of the LT protein or a variant thereof, and Y2 is a fragment of the LIGHT protein or a variant thereof.

In one embodiment, the heterotrimer may be one in which Y1 is a fragment of the LIGHT protein or a variant thereof, and Y2 and Y3 are a fragment of the LTβ protein or a variant thereof; or Y1 and Y2 are a fragment of the LTβ protein or a variant thereof, and Y3 is a fragment of the LIGHT protein or a variant thereof. At this time, the heterotrimer may comprise the amino acid sequence of SEQ ID NO: 89, SEQ ID NO: 90, SEQ ID NO: 91, or SEQ ID NO: 100.

### FAP and antigen binding domain that specifically binds to FAP

As used herein, the term "FAP (fibroblast activation protein)" refers to fibroblast activation protein, which is expressed on the cell surface and presented in tumor cells of various tumor types or in the tumor cell environment of various tumor types. FAP is known to be selectively overexpressed on the surface of cancer-associated fibroblasts (CAFs) in the tumor microenvironment.

In addition, FAP is a homodimer containing two N-glycosylated subunits having a C-terminal extracellular domain in which the enzyme catalytic domain is located, and the glycosylated form of FAP has both post-prolyl dipeptidyl peptidase and gelatinase activities.

Meanwhile, FAP is a prolyl endopeptidase, a membrane-bound gelatinase of about 170 kDa. The prolyl endopeptidase FAP may recognize and cleave a specific amino acid sequence. Accordingly, FAP is also referred to as FAPα, separase, or circulating antiplasmin-cleaving enzyme.

The amino acid sequence of the FAP may be described in GenBank accession No. AAC51668, and human FAP was first identified in cultured fibroblasts using monoclonal antibody (mAb) F19 (see International Patent Application Publication No. WO 93/05804). FAP is expressed in many cancers but has limited expression in normal tissues, making it a promising antigenic target for imaging, diagnosis, and therapy of a variety of cancers.

The FAP includes any variant, isoform, or homolog of human FAP, which is naturally expressed by cells or expressed on cells transfected with the FAP gene. The antibody of the present invention may bind to FAP present on the membrane or cell surface of cancer cells (tumor cells or cells of the tumor stroma) and may induce ADCC or other effector-mediated cancer cell death. In addition, it may be used to block FAP enzymatic activity (e.g., serine peptidase, gelatinase, collagenase activity), FAP-mediated ECM degradation, and FAP-mediated cell invasion or migration.

As used herein, the term "that specifically binds to" refers to binding that is measurably different from a non-specific interaction. Specifically binding may be determined by competing with a control molecule similar to a target that does not have binding activity.

As used herein, the term "antigen binding domain (epitope)" refers to a determinant that interacts with a specific antigen binding domain in a variable region of the antibody. The antigen binding domain is a group of molecules, such as amino acids or sugar side chains, which usually have specific structural characteristics as well as specific charge characteristics. The antigen binding domain that specifically binds to FAP may be a generic term for molecules capable of antigen-antibody binding specifically to FAP.

In addition, the antibody or fragment thereof may be used in any form as long as it contains an antigen binding domain capable of specifically binding to FAP.

The antigen binding domain may include other amino acids not directly involved in binding, or amino acids whose effects are blocked by amino acid residues of the antigen binding domain.

Specifically, the antigen binding domain may be an antibody that specifically binds to FAP, or a fragment thereof.

As used herein, the term "antibody" refers to a molecule containing an antigen binding domain, and an immunologically active fragment of an immunoglobulin molecule containing an antigen binding domain. The immunoglobulin molecule may be an immunoglobulin molecule of IgG, IgE, IgM, IgD, IgA, IgY or a subclass thereof. Antibodies include synthetic antibodies, monoclonal antibodies, single domain antibodies, single chain antibodies, recombinantly produced antibodies, multispecific antibodies (including bispecific antibodies), human antibodies, humanized antibodies, chimeric antibodies, intrabodies, scFv (including for example monospecific and bispecific, etc.), Fab fragment, F(ab') fragment, disulfide-linked Fv (sdFv), anti-idiotypic (anti-Id) antibodies, and an antigen binding fragment of the antibodies, but are not limited thereto.

As used herein, the term "antibody fragment" may be a portion of an antibody, such as F(ab')₂, F(ab)₂, Fab', Fab, Fv, scFv. Regardless of structure, an antibody fragment binds to the same antigen recognized by the intact antibody.

In one embodiment, the antigen binding domain that specifically binds to FAP may comprise a heavy chain variable region comprising HCDR1 of SEQ ID NO: 101, HCDR2 of SEQ ID NO: 102, and HCDR3 of SEQ ID NO: 103; and a light chain variable region comprising LCDR1 of SEQ ID NO: 104, LCDR2 of SEQ ID NO: 105, and LCDR3 of SEQ ID NO: 106. At this time, in one embodiment, the antigen binding domain that specifically binds to FAP may have a heavy chain variable region of SEQ ID NO: 107 and a light chain variable region of SEQ ID NO: 108.

In addition, in one embodiment, it may comprise a heavy chain variable region comprising HCDR1 of SEQ ID NO: 109, HCDR2 of SEQ ID NO: 110, and HCDR3 of SEQ ID NO: 111; and a light chain variable region comprising LCDR1 of SEQ ID NO: 112, LCDR2 of SEQ ID NO: 113, and LCDR3 of SEQ ID NO: 114. At this time, in one embodiment, the antigen binding domain that specifically binds to FAP may have a heavy chain variable region of SEQ ID NO: 115 and a light chain variable region of SEQ ID NO: 116.

In addition, the antigen binding domain that specifically binds to FAP may include a known anti-FAP antibody or a fragment thereof. The anti-FAP antibody or a fragment thereof may mean an antibody known to those of ordinary skill in the art without limitation.

The anti-FAP antibody or a fragment thereof may comprise at least one variable region selected from the group consisting of NG-641, AMG-506/MP-0310, 28H1 of RG-7827, 4B9 of RG-7827, OMTX-705, 3F2, 4G8, 3D9, 4B3, 19G1, 20G8, 5B8, 5F1, 14B3, 16F1, 16F8, O3C9, 29B11, O2D7, and 23C10.

In another example, as the antibody, an anti-FAP antibody or a fragment thereof disclosed in Korean Patent Application Publication No. KR 20200037826 A, US Patent Application Publication No. US 2020-0385488 A1, US Patent Registration No. US 3,924,445 B2, US Patent Registration No. US 9,011,847 B2, or US Patent Application Publication No. US 2017-007716 A1 may be used.

In one embodiment, the anti-FAP antibody may comprise a variable region of NG-641. Specifically, the antibody may comprise a heavy chain variable region comprising HCDR1 of SEQ ID NO: 127, HCDR2 of SEQ ID NO: 128, and HCDR3 of SEQ ID NO: 129; and a light chain variable region comprising LCDR1 of SEQ ID NO: 130, LCDR2 of SEQ ID NO: 131, and LCDR3 of SEQ ID NO: 132. In addition, the anti-FAP antibody may comprise a heavy chain variable region of SEQ ID NO: 248 and a light chain variable region of SEQ ID NO: 249.

In one embodiment, the anti-FAP antibody may comprise a variable region of 28H1. Specifically, the antibody may comprise a heavy chain variable region comprising HCDR1 of SEQ ID NO: 133, HCDR2 of SEQ ID NO: 134, and HCDR3 of SEQ ID NO: 135; and a light chain variable region comprising LCDR1 of SEQ ID NO: 136, LCDR2 of SEQ ID NO: 137, and LCDR3 of SEQ ID NO: 138. In addition, the anti-FAP antibody may comprise a heavy chain variable region of SEQ ID NO: 250 and a light chain variable region of SEQ ID NO: 251.

In one embodiment, the anti-FAP antibody may comprise a variable region of 4B9. Specifically, the antibody may comprise a heavy chain variable region comprising HCDR1 of SEQ ID NO: 139, HCDR2 of SEQ ID NO: 140, and HCDR3 of SEQ ID NO: 141; and a light chain variable region comprising LCDR1 of SEQ ID NO: 142, LCDR2 of SEQ ID NO: 143, and LCDR3 of SEQ ID NO: 144. In addition, the anti-FAP antibody may comprise a heavy chain variable region of SEQ ID NO: 252 and a light chain variable region of SEQ ID NO: 253.

In one embodiment, the anti-FAP antibody may comprise a variable region of OMTX-705. Specifically, the antibody may comprise a heavy chain variable region comprising HCDR1 of SEQ ID NO: 145, HCDR2 of SEQ ID NO: 146, and HCDR3 of SEQ ID NO: 147; and a light chain variable region comprising LCDR1 of SEQ ID NO: 148, LCDR2 of SEQ ID NO: 149, and LCDR3 of SEQ ID NO: 150. In addition, the anti-FAP antibody may comprise a heavy chain variable region of SEQ ID NO: 254 and a light chain variable region of SEQ ID NO: 255.

In one embodiment, the anti-FAP antibody may comprise a variable region of 3F2. Specifically, the antibody may comprise a heavy chain variable region comprising HCDR1 of SEQ ID NO: 151, HCDR2 of SEQ ID NO: 152, and HCDR3 of SEQ ID NO: 153; and a light chain variable region comprising LCDR1 of SEQ ID NO: 154, LCDR2 of SEQ ID NO: 155, and LCDR3 of SEQ ID NO: 156. In addition, the anti-FAP antibody may comprise a heavy chain variable region of SEQ ID NO: 256 and a light chain variable region of SEQ ID NO: 257.

In one embodiment, the anti-FAP antibody may comprise a variable region of 4G8. Specifically, the antibody may comprise a heavy chain variable region comprising HCDR1 of SEQ ID NO: 157, HCDR2 of SEQ ID NO: 158, and HCDR3 of SEQ ID NO: 159; and a light chain variable region comprising LCDR1 of SEQ ID NO: 160, LCDR2 of SEQ ID NO: 161, and LCDR3 of SEQ ID NO: 162. In addition, the anti-FAP antibody may comprise a heavy chain variable region of SEQ ID NO: 258 and a light chain variable region of SEQ ID NO: 259.

In one embodiment, the anti-FAP antibody may comprise a variable region of 3D9. Specifically, the antibody may comprise a heavy chain variable region comprising HCDR1 of SEQ ID NO: 163, HCDR2 of SEQ ID NO: 164, and HCDR3 of SEQ ID NO: 165; and a light chain variable region comprising LCDR1 of SEQ ID NO: 166, LCDR2 of SEQ ID NO: 167, and LCDR3 of SEQ ID NO: 168. In addition, the anti-FAP antibody may comprise a heavy chain variable region of SEQ ID NO: 260 and a light chain variable region of SEQ ID NO: 261.

In one embodiment, the anti-FAP antibody may comprise a variable region of 4B3. Specifically, the antibody may comprise a heavy chain variable region comprising HCDR1 of SEQ ID NO: 169, HCDR2 of SEQ ID NO: 170, and HCDR3 of SEQ ID NO: 171; and a light chain variable region comprising LCDR1 of SEQ ID NO: 172, LCDR2 of SEQ ID NO: 173, and LCDR3 of SEQ ID NO: 174. In addition, the anti-FAP antibody may comprise a heavy chain variable region of SEQ ID NO: 262 and a light chain variable region of SEQ ID NO: 263.

In one embodiment, the anti-FAP antibody may comprise a variable region of 19G1. Specifically, the antibody may comprise a heavy chain variable region comprising HCDR1 of SEQ ID NO: 175, HCDR2 of SEQ ID NO: 176, and HCDR3 of SEQ ID NO: 177; and a light chain variable region comprising LCDR1 of SEQ ID NO: 178, LCDR2 of SEQ ID NO: 179, and LCDR3 of SEQ ID NO: 180. In addition, the anti-FAP antibody may comprise a heavy chain variable region of SEQ ID NO: 264 and a light chain variable region of SEQ ID NO: 265.

In one embodiment, the anti-FAP antibody may comprise a variable region of 20G8. Specifically, the antibody may comprise a heavy chain variable region comprising HCDR1 of SEQ ID NO: 181, HCDR2 of SEQ ID NO: 182, and HCDR3 of SEQ ID NO: 183; and a light chain variable region comprising LCDR1 of SEQ ID NO: 184, LCDR2 of SEQ ID NO: 185, and LCDR3 of SEQ ID NO: 186. In addition, the anti-FAP antibody may comprise a heavy chain variable region of SEQ ID NO: 266 and a light chain variable region of SEQ ID NO: 267.

In one embodiment, the anti-FAP antibody may comprise a variable region of 5B8. Specifically, the antibody may comprise a heavy chain variable region comprising HCDR1 of SEQ ID NO: 187, HCDR2 of SEQ ID NO: 188, and HCDR3 of SEQ ID NO: 189; and a light chain variable region comprising LCDR1 of SEQ ID NO: 190, LCDR2 of SEQ ID NO: 191, and LCDR3 of SEQ ID NO: 192. In addition, the anti-FAP antibody may comprise a heavy chain variable region of SEQ ID NO: 268 and a light chain variable region of SEQ ID NO: 269.

In one embodiment, the anti-FAP antibody may comprise a variable region of 5F1. Specifically, the antibody may comprise a heavy chain variable region comprising HCDR1 of SEQ ID NO: 193, HCDR2 of SEQ ID NO: 194, and HCDR3 of SEQ ID NO: 195; and a light chain variable region comprising LCDR1 of SEQ ID NO: 196, LCDR2 of SEQ ID NO: 197, and LCDR3 of SEQ ID NO: 198. In addition, the anti-FAP antibody may comprise a heavy chain variable region of SEQ ID NO: 270 and a light chain variable region of SEQ ID NO: 271.

In one embodiment, the anti-FAP antibody may comprise a variable region of 14B3. Specifically, the antibody may comprise a heavy chain variable region comprising HCDR1 of SEQ ID NO: 199, HCDR2 of SEQ ID NO: 200, and HCDR3 of SEQ ID NO: 201; and a light chain variable region comprising LCDR1 of SEQ ID NO: 202, LCDR2 of SEQ ID NO: 203, and LCDR3 of SEQ ID NO: 204. In addition, the anti-FAP antibody may comprise a heavy chain variable region of SEQ ID NO: 272 and a light chain variable region of SEQ ID NO: 273.

In one embodiment, the anti-FAP antibody may comprise a variable region of 16F1. Specifically, the antibody may comprise a heavy chain variable region comprising HCDR1 of SEQ ID NO: 205, HCDR2 of SEQ ID NO: 206, and HCDR3 of SEQ ID NO: 207; and a light chain variable region comprising LCDR1 of SEQ ID NO: 208, LCDR2 of SEQ ID NO: 209, and LCDR3 of SEQ ID NO: 210. In addition, the anti-FAP antibody may comprise a heavy chain variable region of SEQ ID NO: 274 and a light chain variable region of SEQ ID NO: 275.

In one embodiment, the anti-FAP antibody may comprise a variable region of 16F8. Specifically, the antibody may comprise a heavy chain variable region comprising HCDR1 of SEQ ID NO: 211, HCDR2 of SEQ ID NO: 212, and HCDR3 of SEQ ID NO: 213; and a light chain variable region comprising LCDR1 of SEQ ID NO: 214, LCDR2 of SEQ ID NO: 215, and LCDR3 of SEQ ID NO: 216. In addition, the anti-FAP antibody may comprise a heavy chain variable region of SEQ ID NO: 276 and a light chain variable region of SEQ ID NO: 277.

In one embodiment, the anti-FAP antibody may comprise a variable region of O3C9. Specifically, the antibody may comprise a heavy chain variable region comprising HCDR1 of SEQ ID NO: 217, HCDR2 of SEQ ID NO: 218, and HCDR3 of SEQ ID NO: 219; and a light chain variable region comprising LCDR1 of SEQ ID NO: 220, LCDR2 of SEQ ID NO: 221, and LCDR3 of SEQ ID NO: 222. In addition, the anti-FAP antibody may comprise a heavy chain variable region of SEQ ID NO: 278 and a light chain variable region of SEQ ID NO: 279.

In one embodiment, the anti-FAP antibody may comprise a variable region of 22A3. Specifically, the antibody may comprise a heavy chain variable region comprising HCDR1 of SEQ ID NO: 223, HCDR2 of SEQ ID NO: 224, and HCDR3 of SEQ ID NO: 225; and a light chain variable region comprising LCDR1 of SEQ ID NO: 226, LCDR2 of SEQ ID NO: 227, and LCDR3 of SEQ ID NO: 228. In addition, the anti-FAP antibody may comprise a heavy chain variable region of SEQ ID NO: 280 and a light chain variable region of SEQ ID NO: 281.

In one embodiment, the anti-FAP antibody may comprise a variable region of 29B11. Specifically, the antibody may comprise a heavy chain variable region comprising HCDR1 of SEQ ID NO: 229, HCDR2 of SEQ ID NO: 230, and HCDR3 of SEQ ID NO: 231; and a light chain variable region comprising LCDR1 of SEQ ID NO: 232, LCDR2 of SEQ ID NO: 233, and LCDR3 of SEQ ID NO: 234. In addition, the anti-FAP antibody may comprise a heavy chain variable region of SEQ ID NO: 282 and a light chain variable region of SEQ ID NO: 283.

In one embodiment, the anti-FAP antibody may comprise a variable region of O2D7. Specifically, the antibody may comprise a heavy chain variable region comprising HCDR1 of SEQ ID NO: 235, HCDR2 of SEQ ID NO: 236, and HCDR3 of SEQ ID NO: 237; and a light chain variable region comprising LCDR1 of SEQ ID NO: 238, LCDR2 of SEQ ID NO: 239, and LCDR3 of SEQ ID NO: 240. In addition, the anti-FAP antibody may comprise a heavy chain variable region of SEQ ID NO: 284 and a light chain variable region of SEQ ID NO: 285.

In one embodiment, the anti-FAP antibody may comprise a variable region of 23C10. Specifically, the antibody may comprise a heavy chain variable region comprising HCDR1 of SEQ ID NO: 241, HCDR2 of SEQ ID NO: 242, and HCDR3 of SEQ ID NO: 243; and a light chain variable region comprising LCDR1 of SEQ ID NO: 244, LCDR2 of SEQ ID NO: 245, and LCDR3 of SEQ ID NO: 246. In addition, the anti-FAP antibody may comprise a heavy chain variable region of SEQ ID NO: 286 and a light chain variable region of SEQ ID NO: 287.

In one embodiment, the anti-FAP antibody may be AMG-506/MP-0310. Specifically, the antibody may comprise the amino acid sequence of SEQ ID NO: 247.

The anti-FAP antibody specifically binds to FAP present on cancer cells, and any antibody may be used as long as it may induce the death of cancer cells.

### Structure of the first monomer

The first monomer comprises an antigen binding domain that specifically binds to FAP. At this time, the first monomer may further comprise a LIGHT protein, a fragment thereof, or a variant thereof.

In one embodiment, the first monomer may comprise a LIGHT protein, a fragment thereof, or a variant thereof; and an Fc region fragment or a variant thereof. At this time, the LIGHT protein, a fragment thereof, or a variant thereof may be bound to the C-terminus or the N-terminus of the first monomer.

In one embodiment, the first monomer may further comprise an antigen binding domain that specifically binds to FAP. At this time, the antigen binding domain that specifically binds to FAP may be bound to the C-terminus or the N-terminus of the first monomer. Preferably, it may be bound to the N-terminus.

The first monomer may comprise a structure of the following structural formula (I) or (II).
N'-X-[linker (3)]q-Fc domain-[linker (4)]r-(Y)c-C' (I)
N'-(Y)d-[linker (3)]q-Fc domain-[linker (4)]r-X-C' (II)
in the structural formulas (I) and (II),
N' is the N-terminus of the fusion protein,
C' is the C-terminus of the fusion protein,
X is the antigen binding domain that specifically binds to FAP,
Y is the LIGHT protein, a fragment thereof, or a variant thereof,
the linkers (3) and (4) are peptide linkers, and
c, d, q, and r are each independently 0 or 1.

At this time, the first monomer may comprise at least one sequence number selected from the group consisting of SEQ ID NO: 1, SEQ ID NO: 2, SEQ ID NO: 4, SEQ ID NO: 9 SEQ ID NO: 10, SEQ ID NO: 11, SEQ ID NO: 12, SEQ ID NO: 13, SEQ ID NO: 16, SEQ ID NO: 22, SEQ ID NO: 23, SEQ ID NO: 25, SEQ ID NO: 29, SEQ ID NO: 30, SEQ ID NO: 31, and SEQ ID NO: 35.

In addition, the first monomer may comprise a monomer structure of an antibody, and specifically, may comprise a heavy chain consisting of the amino acid sequence of SEQ ID NO: 75 and a light chain consisting of the amino acid sequence of SEQ ID NO: 2; or a heavy chain consisting of the amino acid sequence of SEQ ID NO: 76 and a light chain consisting of the amino acid sequence of SEQ ID NO: 23.

In one embodiment, the first monomer may be composed of an amino acid sequence of SEQ ID NO: 1 and SEQ ID NO: 2; SEQ ID NO: 2 and SEQ ID NO: 4; SEQ ID NO: 2 and SEQ ID NO: 9; SEQ ID NO: 2 and SEQ ID NO: 10; SEQ ID NO: 2 and SEQ ID NO: 11; SEQ ID NO: 2 and SEQ ID NO: 12; SEQ ID NO: 2 and SEQ ID NO: 13; SEQ ID NO: 2 and SEQ ID NO: 16; SEQ ID NO: 22 and SEQ ID NO: 23; SEQ ID NO: 23 and SEQ ID NO: 25; SEQ ID NO: 23 and SEQ ID NO: 29; SEQ ID NO: 23 and SEQ ID NO: 30; SEQ ID NO: 23 and SEQ ID NO: 31, or SEQ ID NO: 23 and SEQ ID NO: 35.

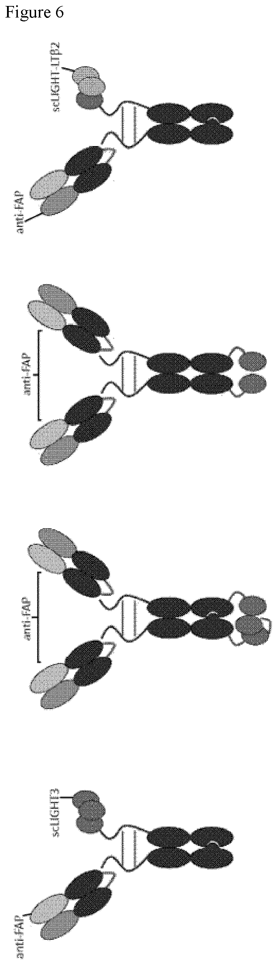

In one embodiment, the first monomer may have a structure of structural formula (I), in which q may be 1, and r and c may be each 0. At this time, the first monomer has the left monomer structure of a of Figure 6. In addition, the first monomer may comprise an amino acid sequence of SEQ ID NO: 1 and SEQ ID NO: 2; SEQ ID NO: 2 and SEQ ID NO: 4; SEQ ID NO: 22 and SEQ ID NO: 23; or SEQ ID NO: 23 and SEQ ID NO: 25.

In one embodiment, the first monomer may have a structure of structural formula (I), in which q may be 1, and r and c may be each 1. At this time, the first monomer has the left monomer structure of b of Figure 6 or c of Figure 6. In addition, the first monomer may comprise an amino acid sequence of SEQ ID NO: 2 and SEQ ID NO: 9; SEQ ID NO: 2 and 10; SEQ ID NO: 2 and SEQ ID NO: 11; SEQ ID NO: 2 and SEQ ID NO: 12; SEQ ID NO: 2 and 13; SEQ ID NO: 23 and SEQ ID NO: 29; SEQ ID NO: 23 and SEQ ID NO: 30; or SEQ ID NO: 23 and SEQ ID NO: 31.

### Structure of the second monomer

The second monomer comprises a LIGHT protein, a fragment thereof, or a variant thereof. The second monomer may further comprise an antigen binding domain that specifically binds to FAP.

The second monomer may comprise the following structural formula (III) or (IV).
N'-(X)e-[linker (5)]s-Fc domain-[linker (6)]t-Y'-C' (III)
N'-Y'-[linker (5)]s-Fc domain-[linker (6)]t-(X)f-C' (IV)
in the structural formulas (III) and (IV),
N' is the N-terminus of the fusion protein,
C' is the C-terminus of the fusion protein,
X is the antigen binding domain that specifically binds to FAP,
Y' is the LIGHT protein, a fragment thereof, or a variant thereof; or a heteromultimer of the LIGHT protein and the LTβ protein,
the linkers (5) and (6) are peptide linkers, and
e, f, s, and t are each independently 0 or 1.

At this time, the second monomer may comprise at least one sequence number selected from the group consisting of SEQ ID NO: 2, SEQ ID NO: 3, SEQ ID NO: 5, SEQ ID NO: 6, SEQ ID NO: 7, SEQ ID NO: 8, SEQ ID NO: 10, SEQ ID NO: 11, SEQ ID NO: 13, SEQ ID NO: 16, SEQ ID NO: 17, SEQ ID NO: 18, SEQ ID NO: 19, SEQ ID NO: 20, SEQ ID NO: 21, SEQ ID NO: 23, SEQ ID NO: 24, SEQ ID NO: 26, SEQ ID NO: 27, SEQ ID NO: 28, SEQ ID NO: 30, SEQ ID NO: 31, SEQ ID NO: 35, SEQ ID NO: 36, and SEQ ID NO: 37.

Specifically, the second monomer may be composed of SEQ ID NO: 3; SEQ ID NO: 5; SEQ ID NO: 6; SEQ ID NO: 7; SEQ ID NO: 8; SEQ ID NO: 2 and SEQ ID NO: 9; SEQ ID NO: 2 and SEQ ID NO: 10; SEQ ID NO: 2 and SEQ ID NO: 11; SEQ ID NO: 2 and SEQ ID NO: 12; SEQ ID NO: 2 and SEQ ID NO: 13; SEQ ID NO: 17; SEQ ID NO: 18; SEQ ID NO: 19; SEQ ID NO: 20; SEQ ID NO: 21; SEQ ID NO: 24; SEQ ID NO: 26; SEQ ID NO: 27; SEQ ID NO: 28; SEQ ID NO: 23 and SEQ ID NO: 29; SEQ ID NO: 23 and SEQ ID NO: 30; SEQ ID NO: 23 and SEQ ID NO: 31; SEQ ID NO: 36; or SEQ ID NO: 37.

In one embodiment, the second monomer has a structure of structural formula (III), in which s may be 1, e and t may be each 1, and Y' may be a monomer of the LIGHT protein or a fragment thereof. At this time, it may have the right monomer structure of b of Figure 6 or c of Figure 6. At this time, the second monomer may comprise an amino acid sequence of SEQ ID NO: 2 and SEQ ID NO: 9; SEQ ID NO: 2 and SEQ ID NO: 10; SEQ ID NO: 2 and SEQ ID NO: 11; SEQ ID NO: 2 and SEQ ID NO: 12; SEQ ID NO: 2 and SEQ ID NO: 13; SEQ ID NO: 23 and SEQ ID NO: 29; SEQ ID NO: 23 and SEQ ID NO: 30, or SEQ ID NO: 23 and SEQ ID NO: 31.

In one embodiment, the second monomer has a structure of structural formula (III), in which s may be 1, e and t may be each 1, and Y' may be a homodimer of the LIGHT protein fragment. At this time, it may have the left monomer structure of b of Figure 6. At this time, the second monomer may comprise an amino acid sequence of SEQ ID NO: 2 and SEQ ID NO: 9; SEQ ID NO: 2 and SEQ ID NO: 12; or SEQ ID NO: 23 and SEQ ID NO: 29.

In one embodiment, the second monomer has a structure of structural formula (IV), in which s may be 1, t and f may be each 0, and Y' may be the LIGHT protein, a fragment thereof, or a variant thereof. At this time, it may have the right monomer structure of a of Figure 6. In addition, the second monomer may comprise an amino acid sequence of SEQ ID NO: 3; SEQ ID NO: 5; SEQ ID NO: 6; SEQ ID NO: 7; SEQ ID NO: 8; SEQ ID NO: 18; SEQ ID NO: 24; SEQ ID NO: 26; SEQ ID NO: 27; or SEQ ID NO: 28.

In one embodiment, the second monomer has a structure of structural formula (IV), in which s may be 1, t and f may be each 0, and Y' may be a heteromultimer of the LIGHT protein and the LTβ protein. At this time, it may have the right monomer structure of d of Figure 6. In addition, the second monomer may comprise an amino acid sequence of SEQ ID NO: 17; SEQ ID NO: 19; SEQ ID NO: 20; SEQ ID NO: 21; SEQ ID NO: 36; or SEQ ID NO: 37.

### Peptide linker

As used herein, the term "peptide linker" refers to a peptide used to provide a physicochemical distance or to connect between domains in a fusion protein. The linker may comprise a hinge region of an immunoglobulin.

The peptide linkers (1) to (6) refer to peptide linkers composed of amino acids. Specifically, the peptide linkers (1) to (6) may be composed of 3 to 80 consecutive amino acids, 1 to 30 consecutive amino acids, 3 to 20 consecutive amino acids, or 3 to 15 consecutive amino acids. The peptide linker may include (G4S)n, GGGS(G4S)n, GGS(G3S)n, or GGSG(G3S)n (wherein n is an integer from 0 to 10). At this time, n may be each 0, 1, 2, 3, 4, 5, 6, 7, 8, 9, or 10.

The peptide linker (1) or (2) may be composed of one amino acid sequence among the amino acid sequences consisting of SEQ ID NO: 120 to SEQ ID NO: 126. In one embodiment, the peptide linker (1) or (2) may comprise the amino acid sequence of SEQ ID NO: 122, SEQ ID NO: 124, or SEQ ID NO: 125.

The peptide linker (3) or (5) may comprise at least one cysteine. Specifically, it may comprise one, two, or three cysteines. In addition, the peptide linker (3) or (5) may be derived from a hinge of an immunoglobulin, and may further comprise (G4S)n, GGGS(G4S)n, GGS(G3S)n, or GGSG(G3S)n (wherein n is an integer from 0 to 10). Specifically, the peptide linker (3) or (5) may comprise a hinge region comprising the amino acid sequence of SEQ ID NO: 117, SEQ ID NO: 118, or SEQ ID NO: 119.

In addition, the peptide linker (3) may be composed of a hinge region, and the peptide linker (5) further may comprise a hinge region and an amino acid sequence of (G4S)n, GGGS(G4S)n, GGS(G3S)n, or GGSG(G3S)n (wherein n is an integer from 0 to 10). At this time, (G4S)n, GGGS(G4S)n, or GGS(G3S)n may be one amino acid sequence selected from the group consisting of SEQ ID NO: 120 to SEQ ID NO: 126. In one embodiment, the peptide linker (3) may comprise the amino acid sequence of SEQ ID NO: 117, SEQ ID NO: 118, or SEQ ID NO: 119, and the peptide linker (5) may comprise the amino acid sequence of SEQ ID NO: 117, SEQ ID NO: 118, or SEQ ID NO: 119; and SEQ ID NO: 123 or SEQ ID NO: 125.

In addition, the peptide linker (4) or (6) may be composed of 1 to 30 consecutive amino acids, 3 to 20 consecutive amino acids, or 3 to 15 consecutive amino acids. The peptide linker may include (G4S)n, GGGS(G4S)n, GGS(G3S)n, or GGSG(G3S)n (wherein n is an integer from 0 to 10). At this time, in (G4S)n, n may be each 0, 1, 2, 3, 4, 5, 6, 7, 8, 9, or 10. In one embodiment, in addition, the peptide linker (4) or (6) may comprise the amino acid sequence of SEQ ID NO: 120 or SEQ ID NO: 126.

### Fc region or fragment thereof

At this time, the above-described immunoglobulin fragment may be an Fc region of an immunoglobulin. The Fc region of an immunoglobulin may be a wild-type Fc domain as well as an Fc domain variant. Then, the Fc region may be an Fc region of IgG, IgA, IgE, IgD or IgM. Specifically, it may be derived from IgG1 or IgG2a.

As used herein, the term "Fc domain variant" may refer to a form which is different from the wild-type Fc domain in terms of glycosylation pattern, has a high glycosylation as compared with the wild-type Fc domain, has a low glycosylation as compared with the wild-type Fc domain, or has a deglycosylated form. In addition, an aglycosylated Fc domain is included therein. The Fc domain or a variant thereof may be adapted to have an adjusted number of sialic acids, fucosylations, or glycosylations, through culture conditions or genetic modification of a host.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc regions of immunoglobulin IgG, IgA, IgE, IgD, or IgM. In addition, the Fc domain variant may be in a form in which some amino acids of the Fc domain are substituted with other amino acids.

In one embodiment, the Fc domain may be an IgG1 or IgG2a Fc domain. At this time, the IgG1 Fc domain may comprise the amino acid sequence of SEQ ID NO: 292, and the IgG2a Fc domain may comprise the amino acid sequence of SEQ ID NO: 293.

The "amino acid" introduced by the substitution and/or addition may be any one selected from the group consisting of lysine (K), alanine (A), arginine (R), asparagine (N), aspartic acid (D), cysteine (C), glutamine (Q), glutamic acid (E), glycine (G), histidine (H), isoleucine (I), leucine (L), methionine (M), phenylalanine (F), proline (P), serine (S), threonine (T), tryptophan (W), tyrosine (Y), and valine (V).

In addition, the Fc region may comprise a knob structure or a hole structure.

As used herein, the term "knob-into-hole" is a design strategy for manufacturing an antibody that specifically binds to different regions, such as a bispecific antibody, a multispecific antibody, or a heterodimeric antibody. In general, this technique involves introducing a knob at the interface of a first polypeptide (for example, a first CH3 domain of a first antibody heavy chain) and a corresponding hole at the interface of a second polypeptide (for example, a second CH3 domain of a second antibody heavy chain), so that the knob may be positioned within the hole to promote heterodimer formation and hinder homodimer formation.

A 'knob' is constructed by replacing small amino acid side chains from the interface of a first polypeptide (for example, a first CH3 domain of a first antibody heavy chain) with larger side chains (for example, arginine, phenylalanine, tyrosine or tryptophan). A complementary 'hole' of the same or similar size in the knob is produced by replacing large amino acid side chains from the interface of a second polypeptide (for example, a second CH3 domain of a second antibody heavy chain) with smaller side chains (for example, alanine, serine, valine, or threonine). The knob and hole may be produced by altering a nucleic acid encoding a polypeptide, for example, by site-specific mutagenesis or by peptide synthesis.

In one embodiment, the Fc region comprising a knob or hole may comprise an amino acid sequence selected from the group consisting of SEQ ID NO: 34 and SEQ ID NO: 294 to SEQ ID NO: 296.

The knob structure or the hole structure may be a form in which the 366th, 368th, and/or 407th amino acid in the wild-type IgG1 are substituted with other amino acids. Specifically, the knob structure may be a form in which the amino acid sequence of SEQ ID NO: 292 or SEQ ID NO: 297 is substituted with T366W, and the hole structure may be a form in which the amino acid sequence of SEQ ID NO: 292 or SEQ ID NO: 297 is substituted with T366S, L368A, and Y407V.

In one embodiment, the knob structure or the hole structure may be a form in which the 321st, 323rd, and 362nd amino acids in the wild-type IgG2a are substituted with other amino acids. Specifically, the knob structure may be a form in which the amino acid sequence of SEQ ID NO: 293 or SEQ ID NO: 298 is substituted with T321W, and the hole structure may be a form in which the amino acid sequence of SEQ ID NO: 293 or SEQ ID NO: 298 is substituted with T321S, M323A, and Y362V.

The Fc domain variant may include a DANG mutation or an NG mutation. At this time, the "DANG mutation" refers to the D265A/N297G mutation for removing an effector function in human IgG1 or mouse IgG2a.

The effector functions mediated by the Fc region in an IgG molecule include C1q binding, complement dependent cytotoxicity, Fc receptor binding, antibody dependent cell mediated cytotoxicity (ADCC), phagocytosis, downregulation of cell surface receptors (for example, B cell receptor, BCR) and the like. In general, these effector functions need to the binding of the Fc region with a binding domain (for example, an antibody variable domain).

The effector function may be changed by the substitution of the amino acid sequence of the non-mutated Fc region, and the Fc region in which the effector function is changed may be designed for example, by modifying C1q binding and/or FcR binding, thereby changing CDC activity and/or ADCC activity. That is, the 'DANG mutation' means that the effector function mediated by the Fc region is removed from the IgG molecule so that an unwanted effector fuction does not occur during antibody production.

In one embodiment, the Fc region of the IgG1 comprising the DANG mutation may comprise the D265A/N297G mutated DANG mutation in the amino acid sequence of SEQ ID NO: 292. Specifically, the Fc region of the IgG1 may comprise the amino acid sequence of SEQ ID NO: 297.

In addition, the Fc region of the IgG2a comprising the DANG mutation may comprise the D219A/N254G mutated DANG mutation in the amino acid sequence of SEQ ID NO: 293. Specifically, the Fc region of the IgG2a comprising the DANG mutation may comprise the amino acid sequence of SEQ ID NO: 298.

In one embodiment, the IgG1-derived Fc region may comprise both the DANG mutation and the knob-into-hole structure. Specifically, the IgG1-derived Fc region may comprise the amino acid sequence of SEQ ID NO: 15 or SEQ ID NO: 14.

In one embodiment, the IgG2a-derived Fc region may comprise both the DANG mutation and the knob-into-hole structure. Specifically, the IgG2a-derived Fc region may comprise the amino acid sequence of SEQ ID NO: 32 or SEQ ID NO: 33.

### Structure of fusion protein

The fusion protein may be an antibody. Specifically, the antibody may be an antibody having a homodimeric structure. In addition, the fusion protein may be a heterodimeric antibody comprising a knob-into-hole structure.

The fusion protein may comprise structural formula (I) and structural formula (III); or structural formula (I) and structural formula (IV).

In one embodiment, the first monomer or the second monomer may have one of the following structures:

### Examples of the first monomer:

(i) structural formula (I), in which q, r, and c are 1; or
(ii) structural formula (I), in which q is 1, and r and c are 0.

### Examples of the second monomer:

(iii) structural formula (III), in which s, e, and t are 1, and Y' is the LIGHT protein, a fragment thereof, or a variant thereof;
(iv) structural formula (IV), in which s is 1, t and f are 0, and Y' is the LIGHT protein, a fragment thereof, or a variant thereof; or
(v) structural formula (IV), in which s is 1, t and f are 0, and Y' is a heteromultimer of the LIGHT protein and the LTβ protein.

In the structural formulas (I) to (VI), Y or Y' may have the following structures.

### Examples of LIGHT protein, fragment thereof, or variant thereof:

(vi) a monomer of the LIGHT protein or a variant thereof;
(vii) a homodimer of (vi) above; or
(viii) a homotrimer of (vi) above.

### Examples of heteromultimer of LIGHT protein and LTβ protein:

(ix) structural formula (A), in which n, m, and a are 1, Y1 is a monomer of the LIGHT protein or a variant thereof, and Y2 and Y3 are a monomer of LTβ protein.

In one embodiment, the fusion protein may comprise a first monomer comprising (i) above; and a second monomer comprising (iii) above (b and c of Figure 6). At this time, the fusion protein may comprise at least one amino acid sequence selected from the group consisting of SEQ ID NO: 2, SEQ ID NO: 9, SEQ ID NO: 10, SEQ ID NO: 11, SEQ ID NO: 12, SEQ ID NO: 13, SEQ ID NO: 23, SEQ ID NO: 29, SEQ ID NO: 30, and SEQ ID NO: 31.

Specifically, the fusion protein may comprise SEQ ID NO: 2, SEQ ID NO: 9 and SEQ ID NO: 10; SEQ ID NO: 2 and SEQ ID NO: 11; SEQ ID NO: 2, SEQ ID NO: 12 and SEQ ID NO: 13; SEQ ID NO: 23, SEQ ID NO: 29 and SEQ ID NO: 30; or SEQ ID NO: 23 and SEQ ID NO: 31.

In one embodiment, the fusion protein may comprise a first monomer comprising (ii) above; and a second monomer comprising (iv) above (a of Figure 6). At this time, the fusion protein may comprise at least one amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 18, SEQ ID NO: 22, SEQ ID NO: 23, SEQ ID NO: 24, SEQ ID NO: 25, SEQ ID NO: 26, SEQ ID NO: 27, and SEQ ID NO: 28.

Specifically, the fusion protein may comprise SEQ ID NO: 1, SEQ ID NO: 2 and SEQ ID NO: 3; SEQ ID NO: 2, SEQ ID NO: 4 and SEQ ID NO: 5; SEQ ID NO: 1, SEQ ID NO: 2 and SEQ ID NO: 5; SEQ ID NO: 1, SEQ ID NO: 2 and SEQ ID NO: 6; SEQ ID NO: 1, SEQ ID NO: 2 and SEQ ID NO: 7; SEQ ID NO: 1, SEQ ID NO: 2 and SEQ ID NO: 8; SEQ ID NO: 1, SEQ ID NO: 2, SEQ ID NO: 18; SEQ ID NO: 22, SEQ ID NO: 23 and SEQ ID NO: 24; SEQ ID NO: 23, SEQ ID NO: 25 and SEQ ID NO: 26; SEQ ID NO: 22, SEQ ID NO: 23 and SEQ ID NO: 27; or SEQ ID NO: 22, SEQ ID NO: 23 and SEQ ID NO: 28.

In one embodiment, the fusion protein may comprise a first monomer comprising (ii) above; and a second monomer comprising (v) above (d of Figure 6). At this time, the fusion protein may comprise at least one amino acid sequence selected from the group consisting of SEQ ID NO: 1, SEQ ID NO: 2, SEQ ID NO: 17, SEQ ID NO: 19, SEQ ID NO: 20, SEQ ID NO: 21, SEQ ID NO: 22, SEQ ID NO: 23, SEQ ID NO: 36, and SEQ ID NO: 37.

Specifically, the fusion protein may comprise SEQ ID NO: 1, SEQ ID NO: 2 and SEQ ID NO: 17; SEQ ID NO: 1, SEQ ID NO: 2 and SEQ ID NO: 19; SEQ ID NO: 1, SEQ ID NO: 2 and SEQ ID NO: 20; SEQ ID NO: 1, SEQ ID NO: 2 and SEQ ID NO: 21; SEQ ID NO: 22, SEQ ID NO: 23 and SEQ ID NO: 36; or SEQ ID NO: 22, SEQ ID NO: 23 and SEQ ID NO: 37.

The multispecific fusion protein herein may be in a chemically modified form. In one embodiment, the fusion protein may be chemically modified by glycosylation, acetylation, PEGylation, phosphorylation, amidation, derivatization with known protecting/blocking groups, proteolytic cleavage and/or binding to cellular ligands or other proteins. Many of these chemical modifications may be performed by known techniques.

### Polynucleotide encoding fusion protein

In another aspect of the present invention, provided is a polynucleotide encoding the first monomer or a polynucleotide encoding the second monomer.

In addition, provided is a polynucleotide encoding the first monomer consisting of the structural formula (I) or the structural formula (II); or a polynucleotide encoding the second monomer consisting of the structural formula (III) or the structural formula (IV).

In one embodiment, a polypeptide of the first monomer may comprise an amino acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to a polypeptide composed of SEQ ID NO: 1 and SEQ ID NO: 2; SEQ ID NO: 2 and SEQ ID NO: 4; SEQ ID NO: 2 and SEQ ID NO: 9; SEQ ID NO: 2 and SEQ ID NO: 10; SEQ ID NO: 2 and SEQ ID NO: 11; SEQ ID NO: 2 and SEQ ID NO: 12; SEQ ID NO: 2 and SEQ ID NO: 13; SEQ ID NO: 2 and SEQ ID NO: 16; SEQ ID NO: 22 and SEQ ID NO: 23; SEQ ID NO: 23 and SEQ ID NO: 25; SEQ ID NO: 23 and SEQ ID NO: 29; SEQ ID NO: 23 and SEQ ID NO: 30; SEQ ID NO: 23 and SEQ ID NO: 31; or SEQ ID NO: 23 and SEQ ID NO: 35.

In one embodiment, a polynucleotide encoding the first monomer may have at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to a polynucleotide composed of SEQ ID NO: 38 and SEQ ID NO: 39; SEQ ID NO: 39 and SEQ ID NO: 41; SEQ ID NO: 39 and SEQ ID NO: 46; SEQ ID NO: 39 and SEQ ID NO: 47; SEQ ID NO: 39 and SEQ ID NO: 48; SEQ ID NO: 39 and SEQ ID NO: 49; SEQ ID NO: 39 and SEQ ID NO: 50; SEQ ID NO: 39 and SEQ ID NO: 53; SEQ ID NO: 59 and SEQ ID NO: 60; SEQ ID NO: 60 and SEQ ID NO: 62; SEQ ID NO: 60 and SEQ ID NO: 63; SEQ ID NO: 60 and SEQ ID NO: 66; SEQ ID NO: 60 and SEQ ID NO: 67; SEQ ID NO: 60 and SEQ ID NO: 68; or SEQ ID NO: 60 and SEQ ID NO: 72.

In one embodiment, a polypeptide of the second monomer may comprise an amino acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to a polypeptide composed of SEQ ID NO: 3; SEQ ID NO: 5; SEQ ID NO: 6; SEQ ID NO: 7; SEQ ID NO: 8; SEQ ID NO: 2 and SEQ ID NO: 9; SEQ ID NO: 2 and SEQ ID NO: 10; SEQ ID NO: 2 and SEQ ID NO: 11; SEQ ID NO: 2 and SEQ ID NO: 12; SEQ ID NO: 2 and SEQ ID NO: 13; SEQ ID NO: 2 and SEQ ID NO: 16; SEQ ID NO: 17; SEQ ID NO: 18; SEQ ID NO: 19; SEQ ID NO: 20; SEQ ID NO: 21; SEQ ID NO: 24; SEQ ID NO: 26; SEQ ID NO: 27; SEQ ID NO: 28; SEQ ID NO: 23 and SEQ ID NO: 29; SEQ ID NO: 23 and SEQ ID NO: 30; SEQ ID NO: 23 and SEQ ID NO: 31; SEQ ID NO: 23 and SEQ ID NO: 35; SEQ ID NO: 36; or SEQ ID NO: 37.

In one embodiment, a polynucleotide encoding the second monomer may have at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to a polynucleotide composed of SEQ ID NO: 40; SEQ ID NO: 42; SEQ ID NO: 43; SEQ ID NO: 44; SEQ ID NO: 45; SEQ ID NO: 39 and SEQ ID NO: 47; SEQ ID NO: 39 and SEQ ID NO: 48; SEQ ID NO: 39 and SEQ ID NO: 49; SEQ ID NO: 39 and SEQ ID NO: 50; SEQ ID NO: 39 and SEQ ID NO: 53; SEQ ID NO: 54; SEQ ID NO: 55; SEQ ID NO: 56; SEQ ID NO: 57; SEQ ID NO: 58; SEQ ID NO: 61; SEQ ID NO: 63; SEQ ID NO: 64; SEQ ID NO: 65; SEQ ID NO: 60 and SEQ ID NO: 66; SEQ ID NO: 60 and SEQ ID NO: 67; SEQ ID NO: 60 and SEQ ID NO: 68; SEQ ID NO: 60 and SEQ ID NO: 72; SEQ ID NO: 73; or SEQ ID NO: 74.

The polynucleotide may further comprise a nucleic acid encoding a signal sequence or a leader sequence. As used herein, the term "signal sequence" refers to a signal peptide that directs secretion of a target protein. The signal peptide is translated and then cleaved in a host cell. Specifically, the signal sequence is an amino acid sequence that initiates transportation of a protein across the endoplasmic reticulum (ER) membrane.

The signal sequences are well known in the art for their characteristics. Such signal sequences typically contain 16 to 30 amino acid residues, and may contain more or fewer amino acid residues than such amino acid residues. A typical signal peptide is composed of three regions, that is, an N-terminal region, a central hydrophobic region, and a more polar C-terminal region. The central hydrophobic region contains 4 to 12 hydrophobic residues that cause the signal sequence to be immobilized during transportation of an immature polypeptide through the membrane lipid bilayer.

After initiation, signal sequences are cleaved in the lumen of ER by cellular enzymes, commonly known as signal peptidases. Then, the signal sequence may be a secretory signal sequence of tPa (tissue plasminogen activator), HSV gDs (signal sequence of Herpes simplex virus glycoprotein D), or a growth hormone. Preferably, a secretory signal sequence used in higher eukaryotic cells including mammals and the like may be used. In addition, a wild-type signal sequence may be used, or a signal sequence that has been substituted with a codon having high expression frequency in a host cell may be used.

### Vector loaded with polynucleotide

In another aspect of the present invention, provided is a vector comprising the polynucleotide.

The vector may be introduced into a host cell to be recombined with and inserted into the genome of the host cell. Alternatively, the vector is understood as nucleic acid means containing a polynucleotide sequence which is autonomously replicable as an episome. The vectors include linear nucleic acids, plasmids, phagemids, cosmids, RNA vectors, viral vectors, and analogs thereof. Examples of the viral vector include, but are not limited to, retroviruses, adenoviruses, and adeno-associated viruses.

Specifically, the vector may include plasmid DNA, phage DNA, and the like; and commercially developed plasmids (pUC18, pBAD, pIDTSAMRT-AMP, and the like), E. *coli*-derived plasmids (pYG601BR322, pBR325, pUC118, pUC119, and the like), *Bacillus subtilis*-derived plasmids (pUB110, pTP5, and the like), yeast-derived plasmids (YEp13, YEp24, YCp50, and the like), phage DNA (Charon4A, Charon21A, EMBL3, EMBL4, λgt10, λgt11, λZAP, and the like), animal viral vectors (retroviruses, adenoviruses, vaccinia viruses, and the like), insect viral vectors (baculoviruses and the like). Since the vector exhibits different expression levels and modification of a protein depending on a host cell, it is preferred to select and use a host cell which is most suitable for the purpose.

As used herein, the term "gene expression" or "expression" of a target protein is understood to mean transcription of DNA sequences, translation of mRNA transcripts, and secretion of fusion protein products or fragments thereof. A useful expression vector may be RcCMV (Invitrogen, Carlsbad) or a variant thereof. Expression vectors may contain a human cytomegalovirus (CMV) promoter for promoting continuous transcription of a target gene in mammalian cells, and a bovine growth hormone polyadenylation signal sequence for increasing the stability level of RNA after transcription.

### Transformed cell expressing a fusion protein

In another aspect of the present invention, provided is a transformed cell into which the vector has been introduced.

Host cells for the transformed cell may include, but are not limited to, prokaryotic cells, eukaryotic cells, and cells of mammalian, plant, insect, fungal, or cellular origin. As an example of the prokaryotic cells, *E. coli* may be used. In addition, as an example of the eukaryotic cells, yeast may be used. In addition, for the mammalian cells, CHO cells, F2N cells, CSO cells, BHK cells, Bowes melanoma cells, HeLa cells, 911 cells, AT1080 cells, A549 cells, HEK 293 cells, HEK293T cells, or the like may be used. However, the mammalian cells are not limited thereto, and any cells which are known to those skilled in the art to be usable as mammalian host cells may be used.

In addition, for the introduction of an expression vector into the host cell, CaCl₂ precipitation, Hanahan method whose efficiency has been increased by using a reducing agent such as dimethyl sulfoxide (DMSO) in CaCl₂ precipitation, electroporation, calcium phosphate precipitation, protoplast fusion, agitation using silicon carbide fiber, agrobacteria-mediated transformation, transformation using PEG, dextran sulfate-, lipofectamine-, or dry/inhibition-mediated transformation, or the like may be used.

As described above, for optimization of properties of a fusion protein as a therapeutic agent or for any other purpose, glycosylation pattern of the fusion protein (for example, sialic acids, fucosylations, glycosylations) may be adjusted by manipulating, through methods known to those skilled in the art, glycosylation-related genes possessed by host cells.

### Method of producing a fusion protein

In another aspect of the present invention, provided is a method of producing a fusion protein, comprising the steps of: i) culturing the transformed cell; and ii) recovering a fusion protein comprising an antigen binding domain that specifically binds to FAP; and a LIGHT protein, a fragment thereof, or a variant thereof.

A method of culturing the transformed cells may be carried out using methods well known in the art. Specifically, the culture may be carried out in a batch process, or carried out continuously in a fed batch or repeated fed batch process.

### Use of a fusion protein

In another aspect of the present invention, provided is a pharmaceutical composition for preventing or treating cancer, comprising the fusion protein as an active ingredient.

In another aspect of the present invention, provided is a method for preventing or treating cancer, comprising administering the pharmaceutical composition to a subject.

In another aspect of the present invention, provided is a use of the fusion protein according to any one of claims 1 to 26 for the prevention or treatment of cancer.

At this time, the cancer may be any one selected from the group consisting of gastric cancer, liver cancer, lung cancer, colorectal cancer, breast cancer, prostate cancer, skin cancer, bone cancer, multiple myeloma, glioma, ovarian cancer, pancreatic cancer, cervical cancer, thyroid cancer, laryngeal cancer, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer, and lymphoma.

In one embodiment, the pharmaceutical composition may further comprise an immune checkpoint inhibitor targeting PD-1. For example, the immune checkpoint inhibitor may be an anti-PD-1 antibody.

As used herein, the term "immune checkpoint inhibitor" is a substance that inhibits the activity of an immune checkpoint protein, which inhibits the differentiation, proliferation, and activity of immune cells, and is known to eliminate cancer cells by preventing them from exerting functions to evade the immune system.

As used herein, the term "PD-1 (programmed cell death protein 1)" refers to CD279, a protein expressed on the surface of activated T cells. It reacts with PD-L1 (B7-H1) and PD-L2 (B7-DC), proteins on the surface of cancer cells, and inhibits T-cell activation, growth factors, and cytokine production mediated by TCR (T cell receptor) and CD28, thereby inducing negative signal transduction. PD-1 inhibitors include, for example, pembrolizumab (Keytruda^{®}), nivolumab (Opdivo^{®}), cemiplimab (Libtayo^{®}), JTX-4014, spartalizumab, camrelizumab, scintillimab, tislelizumab, toripalimab, dostalimab, INCMGA00012, AMP-224, and AMP-514.

The preferred dosage of the pharmaceutical composition varies depending on the patient's condition and body weight, the severity of the disease, the drug form, and the route and period of administration, but may be appropriately selected by those of ordinary skill in the art. In the pharmaceutical composition for treating or preventing tumor of the present invention, the active ingredient may be included in any amount (effective amount) depending on the purpose, formulation, mixing purpose, and the like, as long as it may exhibit tumor treatment activity or exhibit a therapeutic effect, especially against cancer. The typical effective amount will be determined within the range of 0.001 wt% to 20.0 wt% based on the total weight of the composition. Here, the term "effective amount" refers to an amount of the active ingredient capable of inducing an effect of improving or treating the condition of a disease, and in particular, inducing an effect of improving or treating the condition of cancer. Such an effective amount may be experimentally determined within the normal capabilities of those of ordinary skill in the art

As used herein, the term "treatment" may be used to mean both therapeutic treatment and preventive treatment. At this time, prevention may be used to mean alleviating or reducing a pathological condition or disease in a subject. In one embodiment, the term "treatment" includes all applications or any form of medication for treating a disease in mammals, including humans. In addition, the above term includes inhibiting or slowing down the progression of a disease; restoring or repairing damaged or defective functions, thereby partially or completely alleviating a disease; or stimulating an inefficient process; or alleviating a serious disease.

Pharmacokinetic parameters such as bioavailability and underlying parameters such as clearance rate may also affect efficacy. Therefore, "enhanced efficacy" (for example, improved efficacy) may be due to enhanced pharmacokinetic parameters and enhanced efficacy, and may be measured by comparing parameters such as clearance rate and treatment or improvement of tumor in test animals or human subjects.

Here, the term "therapeutically effective amount" or "pharmaceutically effective amount" refers to an amount of a compound or composition effective for preventing or treating a target disease, which is an amount that is sufficient to treat the disease with a reasonable benefit/risk ratio applicable to medical treatment and that does not cause side effects. The level of the effective amount may be determined according to factors including the health condition of the patient, the type and severity of the disease, the activity of the drug, the sensitivity to the drug, administration method, administration time, the route of administration and excretion rate, treatment period, the drug to be combined or concurrently used, and other factors well known in the medical field. In one embodiment, a therapeutically effective amount refers to an amount of a drug effective for treating cancer.

At this time, the pharmaceutical composition may further comprise a pharmaceutically acceptable carrier. The pharmaceutically acceptable carrier may be any carrier as long as it is non-toxic material suitable for delivery to a patient. Distilled water, alcohol, fats, waxes and inert solids may be included as a carrier. In addition, a pharmacologically acceptable adjuvant (buffering agent, dispersing agent) may be included in the pharmaceutical composition.

Specifically, the pharmaceutical composition may be prepared as a parenteral formulation according to the route of administration by a conventional method known in the art, including a pharmaceutically acceptable carrier in addition to the active ingredient. Here, "pharmaceutically acceptable" means that it does not inhibit the activity of the active ingredient and does not have toxicity beyond what the application (prescription) target may adapt.

When the pharmaceutical composition is prepared as a parenteral formulation, it may be formulated in the form of an injection, a transdermal preparation, a nasal inhalant, and a suppository together with suitable carriers according to methods known in the art. When it is formulated as an injection, as a suitable carrier, sterile water, ethanol, polyol such as glycerol or propylene glycol, or a mixture thereof may be used, and Ringer's solution, PBS (Phosphate Buffered Saline) containing triethanolamine, or sterile water for injection, an isotonic solution such as 5% dextrose, and the like may be preferably used. Methods for formulating pharmaceutical compositions are known in the art, and specific references may be made to literature [Remington's Pharmaceutical Sciences (19th ed., 1995)], etc. The above literature is considered a part of the present specification.

The preferred dosage of the pharmaceutical composition may be in the range of 0.01 µg/kg to 10 g/kg per day, or in the range of 0.01 mg/kg to 1 g/kg per day, depending on the patient's condition, body weight, sex, and age, the severity of the patient, and the route of administration. The administration may be performed once a day or divided into several times per day. This dosage should not be construed as limiting the scope of the present invention in any way.

The subject to which the pharmaceutical composition may be applied (prescribed) is a mammal and a human, and particularly, the subject is preferably a human. In addition to the active ingredient, the pharmaceutical composition of the present invention may further comprise any compound or natural extract, which is known to have a therapeutic effect on tumor.

At this time, the subject may be a subject suffering from cancer. In addition, the subject may be a mammal, preferably a human.

The route of administration, dosage, and frequency of administration of the fusion protein or the fusion protein dimer may vary depending on the patient's condition and the presence or absence of side effects, and thus the fusion protein or the fusion protein dimer may be administered to a subject in various ways and amounts. The optimal administration method, dosage, and frequency of administration may be selected in an appropriate range by those skilled in the art. In addition, the fusion protein or the fusion protein dimer may be administered in combination with other drugs or physiologically active substances whose therapeutic effect is known with respect to a disease to be treated, or may be formulated in the form of combination preparations with other drugs.

Hereinafter, the present invention will be described in more detail by way of the following examples. However, the following examples are intended only to illustrate the present invention, and the scope of the present invention is not limited thereto.

### Preparation Example 1. Overview of anti-FAP/scLIGHT3 IgG1 DANG, human fusion protein

**[Table 1]**

| PROT EIN | Format | Description | Corresponding sequence |
|---|---|---|---|
| T3.01 | K&H | anti-hu FAP/hu scLIGHT3, hu IgG1 DANG | seq1, seq2, seq3 |
| T3.02 | K&H | anti-hu FAP/hu scLIGHT3, hu IgG1 | seq2, seq4, seq5 |
| T3.03 | K&H | anti-hu FAP/hu scLIGHT3, hu IgG1 knob DANG, K&H | seq1, seq2, seq5 |
| T3.04 | K&H | anti-hu FAP/hu scLIGHT3(GGGGS4), hu IgG1 DANG | seq1, seq2, seq6 |
| T3.05 | K&H | anti-hu FAP/hu scLIGHT3(226-231), hu IgG1 DANG | seq1, seq2, seq7 |
| T3.06 | K&H | anti-hu FAP/hu scLIGHT3(195-198), hu IgG1 DANG | seq1, seq2, seq8 |
| T3.07 | K&H | anti-hu FAP-hu scLIGHT2/anti-hu FAP-hu LIGHT, hu IgG1 DANG (2+1) | seq2, seq9, seq10 |
| T3.08 | Fc-fusion | anti-hu FAP-hu LIGHT, hu IgG1 DANG (2+1) | seq2, seq11 |
| T3.09 | K&H | anti-hu FAP-hu scLIGHT2/anti-hu FAP-hu LIGHT, hu IgG1 (2+1) | seq2, seq12, seq13 |
| T3.10 | K&H | anti-hu FAP/null, hu IgG1 DANG | seq1, seq2, seq14 |
| T3.11 | K&H | null/hu scLIGHT3, hu IgG1 DANG | seq3, seq15 |
| T3.13 | Mab | anti-hu FAP, hu IgG1 DANG | seq2, seq16 |
| T3.14 | K&H | anti-hu FAP/hu scLIGHT-LTb2, hu IgG1 DANG | seq1, seq2, seq17 |
| T3.15 | K&H | anti-hu FAP/hu scLIGHT-LIGHT(L118K, G119E)2, hu IgG1 DANG | seq1, seq2, seq18 |
| T3.16 | K&H | anti-hu FAP/hu scLIGHT(226-231)-LTb2, hu IgG1 DANG | seq1, seq2, seq19 |
| T3.17 | K&H | anti-hu FAP/hu scLIGHT(195-198)-LTb2, hu IgG1 DANG | seq1, seq2, seq20 |
| T3.18 | K&H | anti-hu FAP/hu scLIGHT(GGSG)-LTb2, hu IgG1 DANG | seq1, seq2, seq21 |

| | | | |
|---|---|---|---|
| [seq1] is composed of a heavy chain variable region sequence of a human anti-FAP antibody, a human IgG1 CH1 region sequence, and a human IgG1 Fc in which an effector function is removed by DANG mutations (D265A and N297G) and a knob structure is formed by a T366W mutation. [seq2] is composed of a light chain sequence of a human anti-FAP antibody. [seq3] is composed of a homotrimer composed of a human LIGHT protein soluble form region (91-240) and a linker GGSG, a linker GGGS, and a human IgG1 Fc DANG in which a hole structure is formed by T366S, L368A, and Y407V mutations. [seq4] is composed of a heavy chain variable region sequence of a human anti-FAP antibody, a human IgG1 CH1 region sequence, and a human IgG1 Fc knob. [seq5] is composed of a homotrimer composed of a human LIGHT protein soluble form region and a linker GGSG, a linker GGGS, and a human IgG1 Fc hole. [seq6] is composed of a homotrimer composed of a human LIGHT protein soluble form region (91-240) and a linker GGGGSGGGGSGGGGSGGGGS, and a human IgG1 Fc hole DANG. [seq7] is composed of a homotrimer in which the amino acids 226-231 in a human LIGHT protein soluble form region are mutated to weaken the binding affinity for the HVEM receptor, a linker GGGS, and a human IgG1 Fc hole DANG. [seq8] is composed of a homotrimer in which the amino acids 195-198 in a human LIGHT protein soluble form region are mutated to weaken the binding affinity for the DcR3 receptor, a linker GGGS, and a human IgG1 Fc hole DANG. [seq9] comprises a heavy chain variable region sequence of a human anti-FAP antibody, a human IgG1 CH1 region sequence, a human IgG1 Fc knob DANG, a linker GGGGSGGGGS, and a homodimer composed of a human LIGHT protein soluble form region and a linker GGSG. [seq10] is composed of a heavy chain variable region sequence of a human anti-FAP antibody, a human IgG1 CH1 region sequence, a human IgG1 Fc hole DANG, a linker GGGGSGGGGS, and a human LIGHT protein soluble form region sequence. [seq11] is composed of a heavy chain variable region sequence of a human anti-FAP antibody, a human IgG1 CH1 region sequence, a human IgG1 Fc DANG, a linker GGGGSGGGGS, and a human LIGHT protein soluble form region sequence. [seq12] comprises a heavy chain variable region sequence of a human anti-FAP antibody, a human IgG1 CH1 region sequence, a human IgG1 Fc knob, a linker GGGGSGGGGS, and a homodimer composed of a human LIGHT protein soluble form region and a linker GGSG. [seq13] is composed of a heavy chain variable region sequence of a human anti-FAP antibody, a human IgG1 CH1 region sequence, a human IgG1 Fc hole, a linker GGGGSGGGGS, and a human LIGHT protein soluble form region sequence. [seq14] is composed of a human IgG1 Fc hole DANG. [seq15] is composed of a human IgG1 Fc knob DANG. [seq16] is composed of a heavy chain variable region sequence of a human anti-FAP antibody, a human IgG1 CH1 region sequence, and a human IgG1 Fc DANG. [seq17] is composed of a human LIGHT protein soluble form region sequence, a linker GGGGSGGGGSGGGGS, a heterotrimer composed of the amino acids 87-243 of a human lymphotoxin beta protein extracellular domain region and a linker GGSG, a linker GGGS, and a human IgG1 Fc hole DANG. [seq18] is composed of a heterotrimer composed of a human LIGHT protein soluble form region sequence of a wild type or in which amino acid 118 leucine (L) and 119 glycine (G) are mutated to lysine (K) and glutamic acid (E) and a linker GGSG, a linker GGGS, and a human IgG1 Fc hole DANG. [seq19] is composed of a heterotrimer composed of a human LIGHT protein soluble form region sequence in which the amino acids 226-231 are mutated to weaken the binding affinity for the HVEM receptor, a linker GGGGSGGGGSGGGGS, a sequence of the amino acids 87-243 of a human lymphotoxin beta protein extracellular domain region, and a linker GGSG, a linker GGGS, and a human IgG1 Fc hole DANG. [seq20] is composed of a heterotrimer composed of a human LIGHT protein soluble form region sequence in which the amino acids 195-198 are mutated to weaken the binding affinity for the DcR3 receptor, a linker GGGGSGGGGSGGGGS, a sequence of the amino acids 87-243 of a human lymphotoxin beta protein extracellular domain region, and a linker GGSG, a linker GGGS, and a human IgG1 Fc hole DANG. [seq21] is composed of a heterotrimer composed of a human LIGHT protein soluble form region sequence, a sequence of the amino acids 87-243 of a human lymphotoxin beta protein extracellular domain region, and a linker GGSG, a linker GGGS, and a human IgG1 Fc hole DANG. T3.01 (seq1, seq2, seq3) is a bispecific antibody in which a human anti-FAP antibody sequence and a human LIGHT homotrimer sequence form a knob-into-hole structure, and an effector function is removed. T3.02 (seq2, seq4, seq5) is a bispecific antibody in which a human anti-FAP antibody sequence and a human LIGHT homotrimer sequence form a knob-into-hole structure. T3.03 (seq1, seq2, seq5) is a bispecific antibody in which a human anti-FAP antibody sequence and a human LIGHT homotrimer sequence form a knob-into-hole structure, and some of the effector functions are removed. T3.04 (seq1, seq2, seq6) is a bispecific antibody in which a human anti-FAP antibody sequence and a human LIGHT homotrimer sequence with a modified linker length form a knob-into-hole structure, and an effector function is removed. T3.05 (seq1, seq2, seq7) is a bispecific antibody in which a human anti-FAP antibody sequence and a human LIGHT homotrimer sequence with a weakened binding affinity for the HVEM receptor form a knob-into-hole structure, and an effector function is removed. T3.06 (seq1, seq2, seq8) is a bispecific antibody in which a human anti-FAP antibody sequence and a human LIGHT homotrimer sequence with a weakened binding affinity for the DcR3 receptor form a knob-into-hole structure, and an effector function is removed. T3.07 (seq2, seq9, seq10) is a bispecific antibody in which a human anti-FAP antibody sequence in which a human LIGHT homodimer sequence is linked to the C-terminus of the heavy chain and a human anti-FAP antibody sequence in which a human LIGHT sequence is linked to the C-terminus of the heavy chain form a knob-into-hole structure, and an effector function is removed. T3.08 (seq2, seq11) is an Fc fusion protein that has a human anti-FAP antibody sequence in which a human LIGHT sequence is linked to the C-terminus of the heavy chain and in which an effector function is removed. T3.09 (seq2, seq12, seq13) is a bispecific antibody in which a human anti-FAP antibody sequence in which a human LIGHT homodimer sequence is linked to the C-terminus of the heavy chain and a human anti-FAP antibody sequence in which a human LIGHT sequence is linked to the C-terminus of the heavy chain form a knob-into-hole structure. T3.10 (seq1, seq2, seq14) is an antibody of a knob-into-hole structure having only a human anti-FAP antibody sequence in which an effector function is removed. T3.11 (seq3, seq15) is an antibody of a knob-into-hole structure having only a human LIGHT homotrimer sequence in which an effector function is removed. T3.13 (seq2, seq16) is a human anti-FAP antibody in which an effector function is removed. T3.14 (seq1, seq2, seq17) is a bispecific antibody in which a heterotrimer sequence composed of a human anti-FAP antibody sequence, a human LIGHT, and a human lymphotoxin beta forms a knob-into-hole structure, and an effector function is removed. T3.15 (seq1, seq2, seq18) is a bispecific antibody in which a heterotrimer sequence composed of a human anti-FAP antibody sequence, a human LIGHT, and a human LIGHT in which some amino acids are mutated forms a knob-into-hole structure, and an effector function is removed. T3.16 (seq1, seq2, seq19) is a bispecific antibody in which a heterotrimer sequence composed of a human anti-FAP antibody sequence, a human LIGHT with a weakened binding affinity for the HVEM receptor, and a human lymphotoxin beta forms a knob-into-hole structure, and an effector function is removed. T3.17 (seq1, seq2, seq20) is a bispecific antibody in which a heterotrimer sequence composed of a human anti-FAP antibody sequence, a human LIGHT with a weakened binding affinity for the DcR3 receptor, and a human lymphotoxin beta forms a knob-into-hole structure, and an effector function is removed. T3.18 (seq1, seq2, seq21) is a bispecific antibody in which a heterotrimer sequence composed of a human anti-FAP antibody sequence, a human LIGHT with a modified linker length, and a human lymphotoxin beta forms a knob-into-hole structure, and an effector function is removed. | | | |

**[seq1] anti-hu FAP HC hu IgG1 Fc knob DANG** 
QVQLVQSGAEVKKPGASVKVSCKTSRYTFTEYTIHWVRQAPGQRLEWIGGINPNNGI
PNYNQKFKGRVTITVDTSASTAYMELSSLRSEDTAVYYCARRRIAYGYDEGHAMDY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEK
TISKAKGQPREPQVYTLPPSRDELTKNQVSLWCLVKGFYPSDIAVEWESNGQPENNY
KTTPPVLDSDGSFFLYSKLTVDKSRWQQGNVFSCSVMHEALHNHYTQKSLSLSPGK
**[seq2] anti-hu FAP LC** 
DIVMTQSPDSLAVSLGERATINCKSSQSLLYSRNQKNYLAWYQQKPGQPPKLLIFWA
STRESGVPDRFSGSGFGTDFTLTISSLQAEDVAVYYCQQYFSYPLTFGQGTKVEIKRT
VAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
**[seq3] hu scLIGHT3-hu IgG1 Fc hole DANG** 
EVNPAAHLTGANSSLTGSGGPLLWETQLGLAFLRGLSYHDGALVVTKAGYYYIYSK
VQLGGVGCPLGLASTITHGLYKRTPRYPEELELLVSQQSPCGRATSSSRVWWDSSFL
GGVVHLEAGEKVVVRVLDERLVRLRDGTRSYFGAFMVGGSGEVNPAAHLTGANSS
LTGSGGPLLWETQLGLAFLRGLSYHDGALVVTKAGYYYIYSKVQLGGVGCPLGLAS
TITHGLYKRTPRYPEELELLVSQQSPCGRATSSSRVWWDSSFLGGVVHLEAGEKVVV
RVLDERLVRLRDGTRSYFGAFMVGGSGEVNPAAHLTGANSSLTGSGGPLLWETQLG
LAFLRGLSYHDGALVVTKAGYYYIYSKVQLGGVGCPLGLASTITHGLYKRTPRYPEE
LELLVSQQSPCGRATSSSRVWWDSSFLGGVVHLEAGEKVVVRVLDERLVRLRDGTR
SYFGAFMVGGSGGGGSDKTHTCPPCPAPELLGGPSVFLFPPKPKDTLMISRTPEVTCV
VVAVSHEDPEVKFNWYVDGVEVHNAKTKPREEQYGSTYRVVSVLTVLHQDWLNG
KEYKCKVSNKALPAPIEKTISKAKGQPREPQVYTLPPSRDELTKNQVSLSCAVKGFYP
SDIAVEWESNGQPENNYKTTPPVLDSDGSFFLVSKLTVDKSRWQQGNVFSCSVMHE
ALHNHYTQKSLSLSPGK
**[seq4] anti-hu FAP HC hu IgG1 Fc knob** 
QVQLVQSGAEVKKPGASVKVSCKTSRYTFTEYTIHWVRQAPGQRLEWIGGINPNNGI
PNYNQKFKGRVTITVDTSASTAYMELSSLRSEDTAVYYCARRRIAYGYDEGHAMDY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEK
TISKAKGQPREPQVYTLPPSRDELTKNQVSLWCLVKGFYPSDIAVEWESNGQPENNY
KTTPPVLDSDGSFFLYSKLTVDKSRWQQGNVFSCSVMHEALHNHYTQKSLSLSPGK
**[seq5] hu scLIGHT3-hu IgG1 Fc hole** 
EVNPAAHLTGANSSLTGSGGPLLWETQLGLAFLRGLSYHDGALVVTKAGYYYIYSK
VQLGGVGCPLGLASTITHGLYKRTPRYPEELELLVSQQSPCGRATSSSRVWWDSSFL
GGVVHLEAGEKVVVRVLDERLVRLRDGTRSYFGAFMVGGSGEVNPAAHLTGANSS
LTGSGGPLLWETQLGLAFLRGLSYHDGALVVTKAGYYYIYSKVQLGGVGCPLGLAS
TITHGLYKRTPRYPEELELLVSQQSPCGRATSSSRVWWDSSFLGGVVHLEAGEKVVV
RVLDERLVRLRDGTRSYFGAFMVGGSGEVNPAAHLTGANSSLTGSGGPLLWETQLG
LAFLRGLSYHDGALVVTKAGYYYIYSKVQLGGVGCPLGLASTITHGLYKRTPRYPEE
LELLVSQQSPCGRATSSSRVWWDSSFLGGVVHLEAGEKVVVRVLDERLVRLRDGTR
SYFGAFMVGGSGGGGSDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLSCAVKGFYP
SDIAVEWESNGQPENNYKTTPPVLDSDGSFFLVSKLTVDKSRWQQGNVFSCSVMHE
ALHNHYTQKSLSLSPGK
**[seq6] hu scLIGHT3(GGGGS4)-hu IgG1 Fc hole DANG** 
EVNPAAHLTGANSSLTGSGGPLLWETQLGLAFLRGLSYHDGALVVTKAGYYYIYSK
VQLGGVGCPLGLASTITHGLYKRTPRYPEELELLVSQQSPCGRATSSSRVWWDSSFL
GGVVHLEAGEKVVVRVLDERLVRLRDGTRSYFGAFMVGGGGSGGGGSGGGGSGG
 
GGSEVNPAAHLTGANSSLTGSGGPLLWETQLGLAFLRGLSYHDGALVVTKAGYYYI
YSKVQLGGVGCPLGLASTITHGLYKRTPRYPEELELLVSQQSPCGRATSSSRVWWDS
SFLGGVVHLEAGEKVVVRVLDERLVRLRDGTRSYFGAFMVGGGGSGGGGSGGGGS
GGGGSEVNPAAHLTGANSSLTGSGGPLLWETQLGLAFLRGLSYHDGALVVTKAGY
YYIYSKVQLGGVGCPLGLASTITHGLYKRTPRYPEELELLVSQQSPCGRATSSSRVW
WDSSFLGGVVHLEAGEKVVVRVLDERLVRLRDGTRSYFGAFMVGGGGSGGGGSGG
GGSGGGGSDKTHTCPPCPAPELLGGPSVFLFPPKPKDTLMISRTPEVTCVVVAVSHED
PEVKFNWYVDGVEVHNAKTKPREEQYGSTYRVVSVLTVLHQDWLNGKEYKCKVS
NKALPAPIEKTISKAKGQPREPQVYTLPPSRDELTKNQVSLSCAVKGFYPSDIAVEWE
SNGQPENNYKTTPPVLDSDGSFFLVSKLTVDKSRWQQGNVFSCSVMHEALHNHYTQ
KSLSLSPGK
**[seq7] hu scLIGHT(226-231)3-hu IgG1 Fc hole DANG** 
EVNPAAHLTGANSSLTGSGGPLLWETQLGLAFLRGLSYHDGALVVTKAGYYYIYSK
VQLGGVGCPLGLASTITHGLYKRTPRYPEELELLVSQQSPCGRATSSSRVWWDSSFL
GGVVHLEAGEKVVVRVLDERLVDYTKEDRSYFGAFMVGGSGEVNPAAHLTGANSS
LTGSGGPLLWETQLGLAFLRGLSYHDGALVVTKAGYYYIYSKVQLGGVGCPLGLAS
TITHGLYKRTPRYPEELELLVSQQSPCGRATSSSRVWWDSSFLGGVVHLEAGEKVVV
RVLDERLVDYTKEDRSYFGAFMVGGSGEVNPAAHLTGANSSLTGSGGPLLWETQLG
LAFLRGLSYHDGALVVTKAGYYYIYSKVQLGGVGCPLGLASTITHGLYKRTPRYPEE
LELLVSQQSPCGRATSSSRVWWDSSFLGGVVHLEAGEKVVVRVLDERLVDYTKEDR
SYFGAFMVGGSGGGGSDKTHTCPPCPAPELLGGPSVFLFPPKPKDTLMISRTPEVTCV
VVAVSHEDPEVKFNWYVDGVEVHNAKTKPREEQYGSTYRVVSVLTVLHQDWLNG
KEYKCKVSNKALPAPIEKTISKAKGQPREPQVYTLPPSRDELTKNQVSLSCAVKGFYP
SDIAVEWESNGQPENNYKTTPPVLDSDGSFFLVSKLTVDKSRWQQGNVFSCSVMHE
ALHNHYTQKSLSLSPGK
**[seq8] hu scLIGHT(195-198)3-hu IgG1 Fc hole DANG** 
EVNPAAHLTGANSSLTGSGGPLLWETQLGLAFLRGLSYHDGALVVTKAGYYYIYSK
VQLGGVGCPLGLASTITHGLYKRTPRYPEELELLVSQQSPCGRATSSSSNWFDSSFLG
GVVHLEAGEKVVVRVLDERLVRLRDGTRSYFGAFMVGGSGEVNPAAHLTGANSSL
TGSGGPLLWETQLGLAFLRGLSYHDGALVVTKAGYYYIYSKVQLGGVGCPLGLAST
ITHGLYKRTPRYPEELELLVSQQSPCGRATSSSSNWFDSSFLGGVVHLEAGEKVVVR
VLDERLVRLRDGTRSYFGAFMVGGSGEVNPAAHLTGANSSLTGSGGPLLWETQLGL
AFLRGLSYHDGALVVTKAGYYYIYSKVQLGGVGCPLGLASTITHGLYKRTPRYPEEL
ELLVSQQSPCGRATSSSSNWFDSSFLGGVVHLEAGEKVVVRVLDERLVRLRDGTRSY
FGAFMVGGSGGGGSDKTHTCPPCPAPELLGGPSVFLFPPKPKDTLMISRTPEVTCVVV
AVSHEDPEVKFNWYVDGVEVHNAKTKPREEQYGSTYRVVSVLTVLHQDWLNGKE
YKCKVSNKALPAPIEKTISKAKGQPREPQVYTLPPSRDELTKNQVSLSCAVKGFYPSD
IAVEWESNGQPENNYKTTPPVLDSDGSFFLVSKLTVDKSRWQQGNVFSCSVMHEAL
HNHYTQKSLSLSPGK
**[seq9] anti-hu FAP HC hu IgG1 Fc knob DANG-hu scLIGHT2** 
QVQLVQSGAEVKKPGASVKVSCKTSRYTFTEYTIHWVRQAPGQRLEWIGGINPNNGI
PNYNQKFKGRVTITVDTSASTAYMELSSLRSEDTAVYYCARRRIAYGYDEGHAMDY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEK
TISKAKGQPREPQVYTLPPSRDELTKNQVSLWCLVKGFYPSDIAVEWESNGQPENNY
KTTPPVLDSDGSFFLYSKLTVDKSRWQQGNVFSCSVMHEALHNHYTQKSLSLSPGK
GGGGSGGGGSEVNPAAHLTGANSSLTGSGGPLLWETQLGLAFLRGLSYHDGALVVT
KAGYYYIYSKVQLGGVGCPLGLASTITHGLYKRTPRYPEELELLVSQQSPCGRATSSS
RVWWDSSFLGGVVHLEAGEKVVVRVLDERLVRLRDGTRSYFGAFMVGGSGEVNPA
AHLTGANSSLTGSGGPLLWETQLGLAFLRGLSYHDGALVVTKAGYYYIYSKVQLGG
VGCPLGLASTITHGLYKRTPRYPEELELLVSQQSPCGRATSSSRVWWDSSFLGGVVH
LEAGEKVVVRVLDERLVRLRDGTRSYFGAFMV
**[seq10] anti-hu FAP HC hu IgG1 Fc hole DANG-hu LIGHT** 
QVQLVQSGAEVKKPGASVKVSCKTSRYTFTEYTIHWVRQAPGQRLEWIGGINPNNGI
PNYNQKFKGRVTITVDTSASTAYMELSSLRSEDTAVYYCARRRIAYGYDEGHAMDY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEK
TISKAKGQPREPQVYTLPPSRDELTKNQVSLSCAVKGFYPSDIAVEWESNGQPENNY
KTTPPVLDSDGSFFLVSKLTVDKSRWQQGNVFSCSVMHEALHNHYTQKSLSLSPGK
GGGGSGGGGSEVNPAAHLTGANSSLTGSGGPLLWETQLGLAFLRGLSYHDGALVVT
KAGYYYIYSKVQLGGVGCPLGLASTITHGLYKRTPRYPEELELLVSQQSPCGRATSSS
RVWWDSSFLGGVVHLEAGEKVVVRVLDERLVRLRDGTRSYFGAFMV
**[seq11] anti-hu FAP HC hu IgG1 Fc DANG-hu LIGHT** 
QVQLVQSGAEVKKPGASVKVSCKTSRYTFTEYTIHWVRQAPGQRLEWIGGINPNNGI
PNYNQKFKGRVTITVDTSASTAYMELSSLRSEDTAVYYCARRRIAYGYDEGHAMDY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEK
TISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGG
GGGSGGGGSEVNPAAHLTGANSSLTGSGGPLLWETQLGLAFLRGLSYHDGALVVTK
AGYYYIYSKVQLGGVGCPLGLASTITHGLYKRTPRYPEELELLVSQQSPCGRATSSSR
VWWDSSFLGGVVHLEAGEKVVVRVLDERLVRLRDGTRSYFGAFMV
**[seq12] anti-hu FAP HC hu IgG1 Fc knob-hu scLIGHT2** 
QVQLVQSGAEVKKPGASVKVSCKTSRYTFTEYTIHWVRQAPGQRLEWIGGINPNNGI
PNYNQKFKGRVTITVDTSASTAYMELSSLRSEDTAVYYCARRRIAYGYDEGHAMDY
 
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEK
TISKAKGQPREPQVYTLPPSRDELTKNQVSLWCLVKGFYPSDIAVEWESNGQPENNY
KTTPPVLDSDGSFFLYSKLTVDKSRWQQGNVFSCSVMHEALHNHYTQKSLSLSPGG
GGGSGGGGSEVNPAAHLTGANSSLTGSGGPLLWETQLGLAFLRGLSYHDGALVVTK
AGYYYIYSKVQLGGVGCPLGLASTITHGLYKRTPRYPEELELLVSQQSPCGRATSSSR
VWWDSSFLGGVVHLEAGEKVVVRVLDERLVRLRDGTRSYFGAFMVGGSGEVNPA
AHLTGANSSLTGSGGPLLWETQLGLAFLRGLSYHDGALVVTKAGYYYIYSKVQLGG
VGCPLGLASTITHGLYKRTPRYPEELELLVSQQSPCGRATSSSRVWWDSSFLGGVVH
LEAGEKVVVRVLDERLVRLRDGTRSYFGAFMV
**[seq13] anti-hu FAP HC hu IgG1 Fc hole-hu LIGHT** 
QVQLVQSGAEVKKPGASVKVSCKTSRYTFTEYTIHWVRQAPGQRLEWIGGINPNNGI
PNYNQKFKGRVTITVDTSASTAYMELSSLRSEDTAVYYCARRRIAYGYDEGHAMDY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEK
TISKAKGQPREPQVYTLPPSRDELTKNQVSLSCAVKGFYPSDIAVEWESNGQPENNY
KTTPPVLDSDGSFFLVSKLTVDKSRWQQGNVFSCSVMHEALHNHYTQKSLSLSPGG
GGGSGGGGSEVNPAAHLTGANSSLTGSGGPLLWETQLGLAFLRGLSYHDGALVVTK
AGYYYIYSKVQLGGVGCPLGLASTITHGLYKRTPRYPEELELLVSQQSPCGRATSSSR
VWWDSSFLGGVVHLEAGEKVVVRVLDERLVRLRDGTRSYFGAFMV
**[seq14] hu IgG1 Fc hole DANG** 
DKTHTCPPCPAPELLGGPSVFLFPPKPKDTLMISRTPEVTCVVVAVSHEDPEVKFNWY
VDGVEVHNAKTKPREEQYGSTYRVVSVLTVLHQDWLNGKEYKCKVSNKALPAPIE
KTISKAKGQPREPQVYTLPPSRDELTKNQVSLSCAVKGFYPSDIAVEWESNGQPENN
YKTTPPVLDSDGSFFLVSKLTVDKSRWQQGNVFSCSVMHEALHNHYTQKSLSLSPG
K
**[seq15] hu IgG1 Fc knob DANG** 
DKTHTCPPCPAPELLGGPSVFLFPPKPKDTLMISRTPEVTCVVVAVSHEDPEVKFNWY
VDGVEVHNAKTKPREEQYGSTYRVVSVLTVLHQDWLNGKEYKCKVSNKALPAPIE
KTISKAKGQPREPQVYTLPPSRDELTKNQVSLWCLVKGFYPSDIAVEWESNGQPENN
YKTTPPVLDSDGSFFLYSKLTVDKSRWQQGNVFSCSVMHEALHNHYTQKSLSLSPG
K
**[seq16] anti-hu FAP HC hu IgG1 Fc DANG** 
QVQLVQSGAEVKKPGASVKVSCKTSRYTFTEYTIHWVRQAPGQRLEWIGGINPNNGI
PNYNQKFKGRVTITVDTSASTAYMELSSLRSEDTAVYYCARRRIAYGYDEGHAMDY
WGQGTLVTVSSASTKGPSVFPLAPSSKSTSGGTAALGCLVKDYFPEPVTVSWNSGAL
 
TSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEK
TISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK
**[seq17] hu scLIGHT-LTb2-hu IgG1 Fc hole DANG** 
EVNPAAHLTGANSSLTGSGGPLLWETQLGLAFLRGLSYHDGALVVTKAGYYYIYSK
VQLGGVGCPLGLASTITHGLYKRTPRYPEELELLVSQQSPCGRATSSSRVWWDSSFL
GGVVHLEAGEKVVVRVLDERLVRLRDGTRSYFGAFMVGGGGSGGGGSGGGGSLPA
AHLIGAPLKGQGLGWETTKEQAFLTSGTQFSDAEGLALPQDGLYYLYCLVGYRGRA
PPGGGDPQGRSVTLRSSLYRAGGAYGPGTPELLLEGAETVTPVLDPARRQGYGPLW
YTSVGFGGLVQLRRGERVYVNISHPDMVDFARGKTFFGAVMVGGSGLPAAHLIGAP
LKGQGLGWETTKEQAFLTSGTQFSDAEGLALPQDGLYYLYCLVGYRGRAPPGGGDP
QGRSVTLRSSLYRAGGAYGPGTPELLLEGAETVTPVLDPARRQGYGPLWYTSVGFG
GLVQLRRGERVYVNISHPDMVDFARGKTFFGAVMVGGSGGGGSDKTHTCPPCPAPE
LLGGPSVFLFPPKPKDTLMISRTPEVTCVVVAVSHEDPEVKFNWYVDGVEVHNAKT
KPREEQYGSTYRVVSVLTVLHQDWLNGKEYKCKVSNKALPAPIEKTISKAKGQPREP
QVYTLPPSRDELTKNQVSLSCAVKGFYPSDIAVEWESNGQPENNYKTTPPVLDSDGS
FFLVSKLTVDKSRWQQGNVFSCSVMHEALHNHYTQKSLSLSPGK
**[seq18] hu scLIGHT-LIGHT(L118K, G119E)2-hu IgG1 Fc hole DANG** 
EVNPAAHLTGANSSLTGSGGPLLWETQLGLAFLRGLSYHDGALVVTKAGYYYIYSK
VQLGGVGCPLGLASTITHGLYKRTPRYPEELELLVSQQSPCGRATSSSRVWWDSSFL
GGVVHLEAGEKVVVRVLDERLVRLRDGTRSYFGAFMVGGSGEVNPAAHLTGANSS
LTGSGGPLLWETQKELAFLRGLSYHDGALVVTKAGYYYIYSKVQLGGVGCPLGLAS
TITHGLYKRTPRYPEELELLVSQQSPCGRATSSSRVWWDSSFLGGVVHLEAGEKVVV
RVLDERLVRLRDGTRSYFGAFMVGGSGEVNPAAHLTGANSSLTGSGGPLLWETQKE
LAFLRGLSYHDGALVVTKAGYYYIYSKVQLGGVGCPLGLASTITHGLYKRTPRYPEE
LELLVSQQSPCGRATSSSRVWWDSSFLGGVVHLEAGEKVVVRVLDERLVRLRDGTR
SYFGAFMVGGSGGGGSDKTHTCPPCPAPELLGGPSVFLFPPKPKDTLMISRTPEVTCV
VVAVSHEDPEVKFNWYVDGVEVHNAKTKPREEQYGSTYRVVSVLTVLHQDWLNG
KEYKCKVSNKALPAPIEKTISKAKGQPREPQVYTLPPSRDELTKNQVSLSCAVKGFYP
SDIAVEWESNGQPENNYKTTPPVLDSDGSFFLVSKLTVDKSRWQQGNVFSCSVMHE
ALHNHYTQKSLSLSPGK
**[seq19] hu scLIGHT(226-231)-LTb2-hu IgG1 Fc hole DANG** 
EVNPAAHLTGANSSLTGSGGPLLWETQLGLAFLRGLSYHDGALVVTKAGYYYIYSK
VQLGGVGCPLGLASTITHGLYKRTPRYPEELELLVSQQSPCGRATSSSRVWWDSSFL
GGVVHLEAGEKVVVRVLDERLVDYTKEDRSYFGAFMVGGGGSGGGGSGGGGSLPA
AHLIGAPLKGQGLGWETTKEQAFLTSGTQFSDAEGLALPQDGLYYLYCLVGYRGRA
PPGGGDPQGRSVTLRSSLYRAGGAYGPGTPELLLEGAETVTPVLDPARRQGYGPLW
YTSVGFGGLVQLRRGERVYVNISHPDMVDFARGKTFFGAVMVGGSGLPAAHLIGAP
LKGQGLGWETTKEQAFLTSGTQFSDAEGLALPQDGLYYLYCLVGYRGRAPPGGGDP
 
QGRSVTLRSSLYRAGGAYGPGTPELLLEGAETVTPVLDPARRQGYGPLWYTSVGFG
GLVQLRRGERVYVNISHPDMVDFARGKTFFGAVMVGGSGGGGSDKTHTCPPCPAPE
LLGGPSVFLFPPKPKDTLMISRTPEVTCVVVAVSHEDPEVKFNWYVDGVEVHNAKT
KPREEQYGSTYRVVSVLTVLHQDWLNGKEYKCKVSNKALPAPIEKTISKAKGQPREP
QVYTLPPSRDELTKNQVSLSCAVKGFYPSDIAVEWESNGQPENNYKTTPPVLDSDGS
FFLVSKLTVDKSRWQQGNVFSCSVMHEALHNHYTQKSLSLSPGK
**[seq20] hu scLIGHT(195-198)-LTb2-hu IgG1 Fc hole DANG** 
EVNPAAHLTGANSSLTGSGGPLLWETQLGLAFLRGLSYHDGALVVTKAGYYYIYSK
VQLGGVGCPLGLASTITHGLYKRTPRYPEELELLVSQQSPCGRATSSSSNWFDSSFLG
GVVHLEAGEKVVVRVLDERLVRLRDGTRSYFGAFMVGGGGSGGGGSGGGGSLPAA
HLIGAPLKGQGLGWETTKEQAFLTSGTQFSDAEGLALPQDGLYYLYCLVGYRGRAP
PGGGDPQGRSVTLRSSLYRAGGAYGPGTPELLLEGAETVTPVLDPARRQGYGPLWY
TSVGFGGLVQLRRGERVYVNISHPDMVDFARGKTFFGAVMVGGSGLPAAHLIGAPL
KGQGLGWETTKEQAFLTSGTQFSDAEGLALPQDGLYYLYCLVGYRGRAPPGGGDP
QGRSVTLRSSLYRAGGAYGPGTPELLLEGAETVTPVLDPARRQGYGPLWYTSVGFG
GLVQLRRGERVYVNISHPDMVDFARGKTFFGAVMVGGSGGGGSDKTHTCPPCPAPE
LLGGPSVFLFPPKPKDTLMISRTPEVTCVVVAVSHEDPEVKFNWYVDGVEVHNAKT
KPREEQYGSTYRVVSVLTVLHQDWLNGKEYKCKVSNKALPAPIEKTISKAKGQPREP
QVYTLPPSRDELTKNQVSLSCAVKGFYPSDIAVEWESNGQPENNYKTTPPVLDSDGS
FFLVSKLTVDKSRWQQGNVFSCSVMHEALHNHYTQKSLSLSPGK
**[seq21] hu scLIGHT-LTb2(GGSG)-hu IgG1 Fc hole DANG**

EVNPAAHLTGANSSLTGSGGPLLWETQLGLAFLRGLSYHDGALVVTKAGYYYIYSK
VQLGGVGCPLGLASTITHGLYKRTPRYPEELELLVSQQSPCGRATSSSRVWWDSSFL
GGVVHLEAGEKVVVRVLDERLVRLRDGTRSYFGAFMVGGSGLPAAHLIGAPLKGQ
GLGWETTKEQAFLTSGTQFSDAEGLALPQDGLYYLYCLVGYRGRAPPGGGDPQGRS
VTLRSSLYRAGGAYGPGTPELLLEGAETVTPVLDPARRQGYGPLWYTSVGFGGLVQ
LRRGERVYYVNISHPDMVDFARGKTFFGAVMVGGSGLPAAHLIGAPLKGQGLGWETT
KEQAFLTSGTQFSDAEGLALPQDGLYYLYCLVGYRGRAPPGGGDPQGRSVTLRSSL
YRAGGAYGPGTPELLLEGAETVTPVLDPARRQGYGPLWYTSVGFGGLVQLRRGERV
YYVNISHPDMVDFARGKTFFGAVMVGGSGGGGSDKTHTCPPCPAPELLGGPSVFLFPP
KPKDTLMISRTPEVTCVVVAVSHEDPEVKFNWYVDGVEVHNAKTKPREEQYGSTYR
VVSVLTVLHQDWLNGKEYKCKVSNKALPAPIEKTISKAKGQPREPQVYTLPPSRDEL
TKNQVSLSCAVKGFYPSDIAVEWESNGQPENNYKTTPPVLDSDGSFFLVSKLTVDKS
RWQQGNVFSCSVMHEALHNHYTQKSLSLSPGK

### Preparation Example 2. Overview of anti-FAP/scLIGHT3 IgG2a DANG, mouse fusion protein

**[Table 2]**

| PROTEI N | Format | Description | Corresponding sequence |
|---|---|---|---|
| T3.01m | K&H | anti-mu FAP/mu scLIGHT3, mu IgG2a DANG | seq22, seq23, seq24 |
| T3.02m | K&H | anti-mu FAP/mu scLIGHT3, mu IgG2a | seq23, seq25, seq26 |
| T3.05m | K&H | anti-mu FAP/mu scLIGHT3(225-230), mu IgG2a DANG | seq22, seq23, seq27 |
| T3.06m | K&H | anti-mu FAP/mu scLIGHT3(194-197), mu IgG2a DANG | seq22, seq23, seq28 |
| T3.07m | K&H | anti-mu FAP-mu scLIGHT2/anti-mu FAP-mu LIGHT, mu IgG2a DANG (2+1) | seq23, seq29, seq30 |
| T3.08m | K&H | anti-mu FAP-mu LIGHT, mu IgG2a DANG (2+1) | seq23, seq31 |
| T3.10m | K&H | anti-mu FAP/null, mu IgG2a DANG | seq22, seq23, seq32 |
| T3.11m | K&H | null/mu scLIGHT3, mu IgG2a DANG | seq24, seq33 |
| T3.12m | K&H | null/mu scLIGHT3, mu IgG2a | seq26, seq34 |
| T3.13m | Mab | anti-mu FAP, mu IgG2a DANG | seq23, seq35 |
| T3.14m | K&H | anti-mu FAP/mu scLIGHT-LTb2, mu IgG2a DANG | seq22, seq23, seq36 |
| T3.19m | K&H | anti-mu FAP/mu scLIGHT-LTb2, mu IgG2a knob DANG | seq22, seq23, seq37 |

| | | | |
|---|---|---|---|
| [seq22] is composed of a heavy chain variable region sequence of a mouse anti-FAP antibody (anti-FAP), a mouse IgG2a CH1 region sequence, and a mouse IgG2a Fc in which an effector function is removed by DANG mutations (D219A and N254G) and a knob structure is formed by a T321W mutation. [seq23] is composed of a light chain variable sequence of a mouse anti-FAP antibody. [seq24] is composed of a homotrimer composed of a mouse LIGHT protein soluble form region (91-239) and a linker GGSG, a linker GGGS, and a mouse IgG2a Fc DANG in which a hole structure is formed by T321S, M323A, and Y362V mutations. [seq25] is composed of a heavy chain variable region sequence of a mouse anti-FAP antibody, a mouse IgG2a CH1 region sequence, and a mouse IgG2a Fc knob. [seq26] is composed of a homotrimer composed of a mouse LIGHT protein soluble form region and a linker GGSG, a linker GGGS, and a mouse IgG2a Fc hole. [seq27] is composed of a homotrimer in which the amino acids 225-230 in a mouse LIGHT protein soluble form region are mutated to weaken the binding affinity for the HVEM receptor, a linker GGGS, and a mouse IgG2a Fc hole DANG. [seq28] is composed of a homotrimer in which the amino acids 194-197 in a mouse LIGHT protein soluble form region are mutated to weaken the binding affinity for the DcR3 receptor, a linker GGGS, and a mouse IgG2a Fc hole DANG. [seq29] comprises a heavy chain variable region sequence of a mouse anti-FAP antibody, a mouse IgG2a CH1 region sequence, a mouse IgG2a Fc knob DANG, a linker GGGGSGGGGS, and a homodimer composed of a mouse LIGHT protein soluble form region and a linker GGSG. [seq30] is composed of a heavy chain variable region sequence of a mouse anti-FAP antibody, a mouse IgG2a CH1 region sequence, a mouse IgG2a Fc hole DANG, a linker GGGGSGGGGS, and a mouse LIGHT protein soluble form region sequence. [seq31] is composed of a heavy chain variable region sequence of a mouse anti-FAP antibody, a mouse IgG2a CH1 region sequence, a mouse IgG2a Fc DANG, a linker GGGGSGGGGS, and a mouse LIGHT protein soluble form region sequence. [seq32] is composed of a mouse IgG2a Fc hole DANG. [seq33] is composed of a mouse IgG2a Fc knob DANG. [seq34] is composed of a mouse IgG2a Fc knob. [seq35] is composed of a heavy chain variable region sequence of a mouse anti-FAP antibody, a mouse IgG2a CH1 region sequence, and a mouse IgG2a Fc DANG. [seq36] is composed of a mouse LIGHT protein soluble form region sequence, a linker GGGGSGGGGSGGGGS, a heterotrimer composed of the amino acids 153-305 of a mouse lymphotoxin beta protein extracellular domain region and a linker GGSG, a linker GGGS, and a mouse IgG2a Fc hole DANG. [seq37] is composed of a mouse LIGHT protein soluble form region sequence, a linker GGGGSGGGGSGGGGS, a heterotrimer composed of the amino acids 153-305 of a mouse lymphotoxin beta protein extracellular domain region and a linker GGSG, a linker GGGS, and a mouse IgG2a Fc hole. T3.01m (seq22, seq23, seq24) is a bispecific antibody in which a mouse anti-FAP antibody sequence and a mouse LIGHT homotrimer sequence form a knob-into-hole structure, and an effector function is removed. T3.02m (seq23, seq25, seq26) is a bispecific antibody in which a mouse anti-FAP antibody sequence and a mouse LIGHT homotrimer sequence form a knob-into-hole structure. T3.05m (seq22, seq23, seq27) is a bispecific antibody in which a mouse anti-FAP antibody sequence and a mouse LIGHT homotrimer sequence with a weakened binding affinity for the HVEM receptor form a knob-into-hole structure, and an effector function is removed. T3.06m (seq22, seq23, seq28) is a bispecific antibody in which a mouse anti-FAP antibody sequence and a mouse LIGHT homotrimer sequence with a weakened binding affinity for the DcR3 receptor form a knob-into-hole structure, and an effector function is removed. T3.07m (seq23, seq29, seq30) is a bispecific antibody in which a mouse anti-FAP antibody sequence in which a mouse LIGHT homodimer sequence is linked to the C-terminus of the heavy chain and a mouse anti-FAP antibody sequence in which a mouse LIGHT sequence is linked to the C-terminus of the heavy chain form a knob-into-hole structure, and an effector function is removed. T3.08m (seq23, seq31) is an Fc fusion protein that has a mouse anti-FAP antibody sequence in which a mouse LIGHT sequence is linked to the C-terminus of the heavy chain and in which an effector function is removed. T3.10m (seq22, seq23, seq32) is an antibody of a knob-into-hole structure having only a mouse anti-FAP antibody sequence in which an effector function is removed. T3.11m (seq24, seq33) is an antibody of a knob-into-hole structure having only a mouse LIGHT homotrimer sequence in which an effector function is removed. T3.12m (seq26, seq34) is an antibody of a knob-into-hole structure having only a mouse LIGHT homotrimer sequence. T3.13m (seq23, seq35) is a mouse anti-FAP antibody in which an effector function is removed. T3.14m (seq22, seq23, seq36) is a bispecific antibody in which a heterotrimer sequence composed of a mouse anti-FAP antibody sequence, a mouse LIGHT, and a mouse lymphotoxin beta forms a knob-into-hole structure, and an effector function is removed. T3.19m (seq22, seq23, seq37) is a bispecific antibody in which a heterotrimer sequence composed of a mouse anti-FAP antibody sequence, a mouse LIGHT, and a mouse lymphotoxin beta forms a knob-into-hole structure, and some of the effector functions are removed. | | | |

**[seq22] anti-mu FAP HC mu IgG2a Fc knob DANG** 
QVQLQQSGAELARPGASVNLSCKASGYTFTNNGINWLKQRTGQGLEWIGEIYPRSTN
TLYNEKFKGKATLTADRSSNTAYMELRSLTSEDSAVYFCARTLTAPFAFWGQGTLV
TVSAAKTTAPSVYPLAPVCGDTTGSSVTLGCLVKGYFPEPVTLTWNSGSLSSGVHTF
PAVLQSDLYTLSSSVTVTSSTWPSQSITCNVAHPASSTKVDKKIEPRGPTIKPCPPCKC
PAPNLLGGPSVFIFPPKIKDVLMISLSPIVTCVVVAVSEDDPDVQISWFVNNVEVHTA
QTQTHREDYGSTLRVVSALPIQHQDWMSGKEFKCKVNNKDLPAPIERTISKPKGSVR
APQVYVLPPPEEEMTKKQVTLWCMVTDFMPEDIYVEWTNNGKTELNYKNTEPVLD
SDGSYFMYSKLRVEKKNWVERNSYSCSVVHEGLHNHHTTKSFSRTPGK
**[seq23] anti-mu FAP LC** 
QIVLTQSPAIMSASPGEKVTMTCSASSGVNFMHWYQQKSGTSPKRWIFDTSKLASGV
PARFSGSGSGTSYSLTISSMEAEDAATYYCQQWSFNPPTFGGGTKLEIKRADAAPTVS
IFPPSSEQLTSGGASVVCFLNNFYPKDINVKWKIDGSERQNGVLNSWTDQDSKDSTY
SMSSTLTLTKDEYERHNSYTCEATHKTSTSPIVKSFNRNEC
**[seq24] mu scLIGHT3-mu IgG2a Fc hole DANG** 
NPAAHLTGANASLIGIGGPLLWETRLGLAFLRGLTYHDGALVTMEPGYYYVYSKVQ
LSGVGCPQGLANGLPITHGLYKRTSRYPKELELLVSRRSPCGRANSSRVWWDSSFLG
GVVHLEAGEEVVVRVPGNRLVRPRDGTRSYFGAFMVGGSGNPAAHLTGANASLIGI
 
GGPLLWETRLGLAFLRGLTYHDGALVTMEPGYYYVYSKVQLSGVGCPQGLANGLPI
THGLYKRTSRYPKELELLVSRRSPCGRANSSRVWWDSSFLGGVVHLEAGEEVVVRV
PGNRLVRPRDGTRSYFGAFMVGGSGNPAAHLTGANASLIGIGGPLLWETRLGLAFLR
GLTYHDGALVTMEPGYYYVYSKVQLSGVGCPQGLANGLPITHGLYKRTSRYPKELE
LLVSRRSPCGRANSSRVWWDSSFLGGVVHLEAGEEVVVRVPGNRLVRPRDGTRSYF
GAFMVGGSGGGGSEPRGPTIKPCPPCKCPAPNLLGGPSVFIFPPKIKDVLMISLSPIVTC
VVVAVSEDDPDVQISWFVNNVEVHTAQTQTHREDYGSTLRVVSALPIQHQDWMSG
KEFKCKVNNKDLPAPIERTISKPKGSVRAPQVYVLPPPEEEMTKKQVTLSCAVTDFM
PEDIYVEWTNNGKTELNYKNTEPVLDSDGSYFMVSKLRVEKKNWVERNSYSCSVV
HEGLHNHHTTKSFSRTPGK
**[seq25] anti-mu FAP HC mu IgG2a Fc knob** 
QVQLQQSGAELARPGASVNLSCKASGYTFTNNGINWLKQRTGQGLEWIGEIYPRSTN
TLYNEKFKGKATLTADRSSNTAYMELRSLTSEDSAVYFCARTLTAPFAFWGQGTLV
TVSAAKTTAPSVYPLAPAKTTAPSVYPLAPVCGDTTGSSVTLGCLVKGYFPEPVTLT
WNSGSLSSGVHTFPAVLQSDLYTLSSSVTVTSSTWPSQSITCNVAHPASSTKVDKKIE
PRGPTIKPCPPCKCPAPNLLGGPSVFIFPPKIKDVLMISLSPIVTCVVVDVSEDDPDVQI
SWFVNNVEVHTAQTQTHREDYNSTLRVVSALPIQHQDWMSGKEFKCKVNNKDLPA
PIERTISKPKGSVRAPQVYVLPPPEEEMTKKQVTLWCMVTDFMPEDIYVEWTNNGKT
ELNYKNTEPVLDSDGSYFMYSKLRVEKKNWVERNSYSCSVVHEGLHNHHTTKSFSR
TPGK
**[seq26] mu scLIGHT3-mu IgG2a Fc hole** 
NPAAHLTGANASLIGIGGPLLWETRLGLAFLRGLTYHDGALVTMEPGYYYVYSKVQ
LSGVGCPQGLANGLPITHGLYKRTSRYPKELELLVSRRSPCGRANSSRVWWDSSFLG
GVVHLEAGEEVVVRVPGNRLVRPRDGTRSYFGAFMVGGSGNPAAHLTGANASLIGI
GGPLLWETRLGLAFLRGLTYHDGALVTMEPGYYYVYSKVQLSGVGCPQGLANGLPI
THGLYKRTSRYPKELELLVSRRSPCGRANSSRVWWDSSFLGGVVHLEAGEEVVVRV
PGNRLVRPRDGTRSYFGAFMVGGSGNPAAHLTGANASLIGIGGPLLWETRLGLAFLR
GLTYHDGALVTMEPGYYYVYSKVQLSGVGCPQGLANGLPITHGLYKRTSRYPKELE
LLVSRRSPCGRANSSRVWWDSSFLGGVVHLEAGEEVVVRVPGNRLVRPRDGTRSYF
GAFMVGGSGGGGSEPRGPTIKPCPPCKCPAPNLLGGPSVFIFPPKIKDVLMISLSPIVTC
VVVDVSEDDPDVQISWFVNNVEVHTAQTQTHREDYNSTLRVVSALPIQHQDWMSG
KEFKCKVNNKDLPAPIERTISKPKGSVRAPQVYVLPPPEEEMTKKQVTLSCAVTDFM
PEDIYVEWTNNGKTELNYKNTEPVLDSDGSYFMVSKLRVEKKNWVERNSYSCSVV
HEGLHNHHTTKSFSRTPGK
**[seq27] mu scLIGHT(225-230)3-mu IgG2a Fc hole DANG** 
NPAAHLTGANASLIGIGGPLLWETRLGLAFLRGLTYHDGALVTMEPGYYYVYSKVQ
LSGVGCPQGLANGLPITHGLYKRTSRYPKELELLVSRRSPCGRANSSRVWWDSSFLG
GVVHLEAGEEVVVRVPGNRLVDYTKEDRSYFGAFMVGGSGNPAAHLTGANASLIGI
GGPLLWETRLGLAFLRGLTYHDGALVTMEPGYYYVYSKVQLSGVGCPQGLANGLPI
THGLYKRTSRYPKELELLVSRRSPCGRANSSRVWWDSSFLGGVVHLEAGEEVVVRV
PGNRLVDYTKEDRSYFGAFMVGGSGNPAAHLTGANASLIGIGGPLLWETRLGLAFL
 
RGLTYHDGALVTMEPGYYYVYSKVQLSGVGCPQGLANGLPITHGLYKRTSRYPKEL
ELLVSRRSPCGRANSSRVWWDSSFLGGVVHLEAGEEVVVRVPGNRLVDYTKEDRSY
FGAFMVGGSGGGGSEPRGPTIKPCPPCKCPAPNLLGGPSVFIFPPKIKDVLMISLSPIVT
CVVVAVSEDDPDVQISWFVNNVEVHTAQTQTHREDYGSTLRVVSALPIQHQDWMS
GKEFKCKVNNKDLPAPIERTISKPKGSVRAPQVYVLPPPEEEMTKKQVTLSCAVTDF
MPEDIYVEWTNNGKTELNYKNTEPVLDSDGSYFMVSKLRVEKKNWVERNSYSCSV
VHEGLHNHHTTKSFSRTPGK
**[seq28] mu scLIGHT(194-197)3-mu IgG2a Fc hole DANG** 
NPAAHLTGANASLIGIGGPLLWETRLGLAFLRGLTYHDGALVTMEPGYYYVYSKVQ
LSGVGCPQGLANGLPITHGLYKRTSRYPKELELLVSRRSPCGRANSSNNWFDSSFLG
GVVHLEAGEEVVVRVPGNRLVRPRDGTRSYFGAFMVGGSGNPAAHLTGANASLIGI
GGPLLWETRLGLAFLRGLTYHDGALVTMEPGYYYVYSKVQLSGVGCPQGLANGLPI
THGLYKRTSRYPKELELLVSRRSPCGRANSSNNWFDSSFLGGVVHLEAGEEVVVRVP
GNRLVRPRDGTRSYFGAFMVGGSGNPAAHLTGANASLIGIGGPLLWETRLGLAFLR
GLTYHDGALVTMEPGYYYVYSKVQLSGVGCPQGLANGLPITHGLYKRTSRYPKELE
LLVSRRSPCGRANSSNNWFDSSFLGGVVHLEAGEEVVVRVPGNRLVRPRDGTRSYF
GAFMVGGSGGGGSEPRGPTIKPCPPCKCPAPNLLGGPSVFIFPPKIKDVLMISLSPIVTC
VVVAVSEDDPDVQISWFVNNVEVHTAQTQTHREDYGSTLRVVSALPIQHQDWMSG
KEFKCKVNNKDLPAPIERTISKPKGSVRAPQVYVLPPPEEEMTKKQVTLSCAVTDFM
PEDIYVEWTNNGKTELNYKNTEPVLDSDGSYFMVSKLRVEKKNWVERNSYSCSVV
HEGLHNHHTTKSFSRTPGK
**[seq29] anti-mu FAP HC mu IgG2a Fc knob DANG-mu scLIGHT2** 
QVQLQQSGAELARPGASVNLSCKASGYTFTNNGINWLKQRTGQGLEWIGEIYPRSTN
TLYNEKFKGKATLTADRSSNTAYMELRSLTSEDSAVYFCARTLTAPFAFWGQGTLV
TVSAAKTTAPSVYPLAPVCGDTTGSSVTLGCLVKGYFPEPVTLTWNSGSLSSGVHTF
PAVLQSDLYTLSSSVTVTSSTWPSQSITCNVAHPASSTKVDKKIEPRGPTIKPCPPCKC
PAPNLLGGPSVFIFPPKIKDVLMISLSPIVTCVVVAVSEDDPDVQISWFVNNVEVHTA
QTQTHREDYGSTLRVVSALPIQHQDWMSGKEFKCKVNNKDLPAPIERTISKPKGSVR
APQVYVLPPPEEEMTKKQVTLWCMVTDFMPEDIYVEWTNNGKTELNYKNTEPVLD
SDGSYFMYSKLRVEKKNWVERNSYSCSVVHEGLHNHHTTKSFSRTPGKGGGGSGG
GGSNPAAHLTGANASLIGIGGPLLWETRLGLAFLRGLTYHDGALVTMEPGYYYVYS
KVQLSGVGCPQGLANGLPITHGLYKRTSRYPKELELLVSRRSPCGRANSSRVWWDSS
FLGGVVHLEAGEEVVVRVPGNRLVRPRDGTRSYFGAFMVGGSGNPAAHLTGANAS
LIGIGGPLLWETRLGLAFLRGLTYHDGALVTMEPGYYYVYSKVQLSGVGCPQGLAN
GLPITHGLYKRTSRYPKELELLVSRRSPCGRANSSRVWWDSSFLGGVVHLEAGEEVV
VRVPGNRLVRPRDGTRSYFGAFMV
**[seq30] anti-mu FAP HC mu IgG2a Fc hole DANG-mu LIGHT** 
QVQLQQSGAELARPGASVNLSCKASGYTFTNNGINWLKQRTGQGLEWIGEIYPRSTN
TLYNEKFKGKATLTADRSSNTAYMELRSLTSEDSAVYFCARTLTAPFAFWGQGTLV
TVSAAKTTAPSVYPLAPVCGDTTGSSVTLGCLVKGYFPEPVTLTWNSGSLSSGVHTF
PAVLQSDLYTLSSSVTVTSSTWPSQSITCNVAHPASSTKVDKKIEPRGPTIKPCPPCKC
 
PAPNLLGGPSVFIFPPKIKDVLMISLSPIVTCVVVAVSEDDPDVQISWFVNNVEVHTA
QTQTHREDYGSTLRVVSALPIQHQDWMSGKEFKCKVNNKDLPAPIERTISKPKGSVR
APQVYVLPPPEEEMTKKQVTLSCAVTDFMPEDIYVEWTNNGKTELNYKNTEPVLDS
DGSYFMVSKLRVEKKNWVERNSYSCSVVHEGLHNHHTTKSFSRTPGKGGGGSGGG
GSNPAAHLTGANASLIGIGGPLLWETRLGLAFLRGLTYHDGALVTMEPGYYYVYSK
VQLSGVGCPQGLANGLPITHGLYKRTSRYPKELELLVSRRSPCGRANSSRVWWDSSF
LGGVVHLEAGEEVVVRVPGNRLVRPRDGTRSYFGAFMV
**[seq31] anti-mu FAP HC mu IgG2a Fc DANG-mu LIGHT** 
QVQLQQSGAELARPGASVNLSCKASGYTFTNNGINWLKQRTGQGLEWIGEIYPRSTN
TLYNEKFKGKATLTADRSSNTAYMELRSLTSEDSAVYFCARTLTAPFAFWGQGTLV
TVSAAKTTAPSVYPLAPVCGDTTGSSVTLGCLVKGYFPEPVTLTWNSGSLSSGVHTF
PAVLQSDLYTLSSSVTVTSSTWPSQSITCNVAHPASSTKVDKKIEPRGPTIKPCPPCKC
PAPNLLGGPSVFIFPPKIKDVLMISLSPIVTCVVVAVSEDDPDVQISWFVNNVEVHTA
QTQTHREDYGSTLRVVSALPIQHQDWMSGKEFKCKVNNKDLPAPIERTISKPKGSVR
APQVYVLPPPEEEMTKKQVTLTCMVTDFMPEDIYVEWTNNGKTELNYKNTEPVLDS
DGSYFMYSKLRVEKKNWVERNSYSCSVVHEGLHNHHTTKSFSRTPGGGGGSGGGG
SNPAAHLTGANASLIGIGGPLLWETRLGLAFLRGLTYHDGALVTMEPGYYYVYSKV
QLSGVGCPQGLANGLPITHGLYKRTSRYPKELELLVSRRSPCGRANSSRVWWDSSFL
GGVVHLEAGEEVVVRVPGNRLVRPRDGTRSYFGAFMV
**[seq32] mu IgG2a Fc hole DANG** 
EPRGPTIKPCPPCKCPAPNLLGGPSVFIFPPKIKDVLMISLSPIVTCVVVAVSEDDPDVQ
ISWFVNNVEVHTAQTQTHREDYGSTLRVVSALPIQHQDWMSGKEFKCKVNNKDLP
APIERTISKPKGSVRAPQVYVLPPPEEEMTKKQVTLSCAVTDFMPEDIYVEWTNNGK
TELNYKNTEPVLDSDGSYFMVSKLRVEKKNWVERNSYSCSVVHEGLHNHHTTKSFS
RTPGK
**[seq33] mu IgG2a Fc knob DANG** 
EPRGPTIKPCPPCKCPAPNLLGGPSVFIFPPKIKDVLMISLSPIVTCVVVAVSEDDPDVQ
ISWFVNNVEVHTAQTQTHREDYGSTLRVVSALPIQHQDWMSGKEFKCKVNNKDLP
APIERTISKPKGSVRAPQVYVLPPPEEEMTKKQVTLWCMVTDFMPEDIYVEWTNNG
KTELNYKNTEPVLDSDGSYFMYSKLRVEKKNWVERNSYSCSVVHEGLHNHHTTKSF
SRTPGK
**[seq34] mu IgG2a Fc knob** 
EPRGPTIKPCPPCKCPAPNLLGGPSVFIFPPKIKDVLMISLSPIVTCVVVDVSEDDPDVQ
ISWFVNNVEVHTAQTQTHREDYNSTLRVVSALPIQHQDWMSGKEFKCKVNNKDLP
APIERTISKPKGSVRAPQVYVLPPPEEEMTKKQVTLWCMVTDFMPEDIYVEWTNNG
KTELNYKNTEPVLDSDGSYFMYSKLRVEKKNWVERNSYSCSVVHEGLHNHHTTKSF
SRTPGK
**[seq35] anti-mu FAP HC mu IgG2a Fc DANG** 
QVQLQQSGAELARPGASVNLSCKASGYTFTNNGINWLKQRTGQGLEWIGEIYPRSTN
TLYNEKFKGKATLTADRSSNTAYMELRSLTSEDSAVYFCARTLTAPFAFWGQGTLV
TVSAAKTTAPSVYPLAPVCGDTTGSSVTLGCLVKGYFPEPVTLTWNSGSLSSGVHTF
PAVLQSDLYTLSSSVTVTSSTWPSQSITCNVAHPASSTKVDKKIEPRGPTIKPCPPCKC
PAPNLLGGPSVFIFPPKIKDVLMISLSPIVTCVVVAVSEDDPDVQISWFVNNVEVHTA
QTQTHREDYGSTLRVVSALPIQHQDWMSGKEFKCKVNNKDLPAPIERTISKPKGSVR
APQVYVLPPPEEEMTKKQVTLTCMVTDFMPEDIYVEWTNNGKTELNYKNTEPVLDS
DGSYFMYSKLRVEKKNWVERNSYSCSVVHEGLHNHHTTKSFSRTPGK
**[seq36] mu scLIGHT-LTb2-mu IgG2a Fc hole DANG** 
NPAAHLTGANASLIGIGGPLLWETRLGLAFLRGLTYHDGALVTMEPGYYYVYSKVQ
LSGVGCPQGLANGLPITHGLYKRTSRYPKELELLVSRRSPCGRANSSRVWWDSSFLG
GVVHLEAGEEVVVRVPGNRLVRPRDGTRSYFGAFMVGGGGSGGGGSGGGGSLPAA
HLIGAWMSGQGLSWEASQEEAFLRSGAQFSPTHGLALPQDGVYYLYCHVGYRGRTP
PAGRSRARSLTLRSALYRAGGAYGRGSPELLLEGAETVTPVVDPIGYGSLWYTSVGF
GGLAQLRSGERVYVNISHPDMVDYRRGKTFFGAVMVGGSGLPAAHLIGAWMSGQG
LSWEASQEEAFLRSGAQFSPTHGLALPQDGVYYLYCHVGYRGRTPPAGRSRARSLTL
RSALYRAGGAYGRGSPELLLEGAETVTPVVDPIGYGSLWYTSVGFGGLAQLRSGER
VYVNISHPDMVDYRRGKTFFGAVMVGGSGGGGSEPRGPTIKPCPPCKCPAPNLLGGP
SVFIFPPKIKDVLMISLSPIVTCVVVAVSEDDPDVQISWFVNNVEVHTAQTQTHREDY
GSTLRVVSALPIQHQDWMSGKEFKCKVNNKDLPAPIERTISKPKGSVRAPQVYVLPP
PEEEMTKKQVTLSCAVTDFMPEDIYVEWTNNGKTELNYKNTEPVLDSDGSYFMVSK
LRVEKKNWVERNSYSCSVVHEGLHNHHTTKSFSRTPGK
**[seq37] mu scLIGHT-LTb2-mu IgG2a Fc hole**

NPAAHLTGANASLIGIGGPLLWETRLGLAFLRGLTYHDGALVTMEPGYYYVYSKVQ
LSGVGCPQGLANGLPITHGLYKRTSRYPKELELLVSRRSPCGRANSSRVWWDSSFLG
GVVHLEAGEEVVVRVPGNRLVRPRDGTRSYFGAFMVGGGSGGGGSGGGGSLPAA
HLIGAWMSGQGLSWEASQEEAFLRSGAQFSPTHGLALPQDGVYYLYCHVGYRGRTP
PAGRSRARSLTLRSALYRAGGAYGRGSPELLLEGAETVTPVVDPIGYGSLWYTSVGF
GGLAQLRSGERVYVNISHPDMVDYRRGKTFFGAVMVGGSGLPAAHLIGAWMSGQG
LSWEASQEEAFLRSGAQFSPTHGLALPQDGVYYLYCHVGYRGRTPPAGRSRARSLTL
RSALYRAGGAYGRGSPELLLEGAETVTPVVDPIGYGSLWYTSVGFGGLAQLRSGER
VYVNISHPDMVDYRRGKTFFGAVMVGGSGGGGSEPRGPTIKPCPPCKCPAPNLLGGP
SVFIFPPKIKDVLMISLSPIVTCVVVDVSEDDPDVQISWFVNNVEVHTAQTQTHREDY
NSTLRVVSALPIQHQDWMSGKEFKCKVNNKDLPAPIERTISKPKGSVRAPQVYVLPP
PEEEMTKKQVTLSCAVTDFMPEDIYVEWTNNGKTELNYKNTEPVLDSDGSYFMVSK
LRVEKKNWVERNSYSCSVVHEGLHNHHTTKSFSRTPGK

### Manufacturing Example 1. Manufacturing of fusion proteins

### Manufacturing Example 1.1. Vector composition and plasmid maxi-prep

Reagents and equipment are described in Tables 3 and 4 below.

**[Table 3]**

| **Reagent** | **Manufacturer** | **Catalog#** |
|---|---|---|
| pTT5 | Chempartner | |
| In-Fusion HD cloning kit | Clontech | 639648 |
| Accuprime pfx DNA polymerase | Invitrogen | 12344-04 |
| Gel DNA fragment purification Kit | TaKaRa | D823A |
| FastDigest^{®}BamHI | Fermentas | FD0055 |
| FastDigest^{®}EcoRI | Fermentas | FD0275 |

**[Table 4]**

| **Equipment and tool** | **Manufacturer** | **Model name** |
|---|---|---|
| Biosafety cabinet | NUAIRE | LabGard class ± |
| Centrifuge | Eppendorf | 5424 |
| Gel Imaging System | Tanon | 2500R |

The synthesized DNA fragment was amplified through PCR, and the PCR product was purified by gel. The pTT5 vector was cleaved by the restriction enzymes EcoRI and BamHI, and then the gel was purified. Each PCR product and the linear vector were ligated using the In-Fusion kit. The produced vector was transformed into ECOS101 DH5α competent cells, and the cells were cultured on 2xYT agar plates containing 100 µg/ml ampicillin. All processes were performed according to standard transformation protocols. Positive recombinant products were identified by colony PCR, and sequence-verification sequencing was performed on the recombinant plasmid. A single colony was selected, and the seed strain was inoculated into 5 mL of 2xYT medium containing 100 µg/mL ampicillin. It was cultured with shaking at 37 °C for 8 hours. Thereafter, the seed strain was diluted in 200 mL of selective 2xYT medium at a ratio of 1:1,000. It was cultured with shaking at 37 °C for 16 hours. Bacterial cells were harvested by centrifugation at 4 °C at 4,700 rpm for 10 minutes. The bacterial pellet was resuspended in 12 mL of RES-EF buffer. Thereafter, 12 mL of LYS-EF buffer was added, and the sealed tube was inverted vigorously to mix thoroughly and then incubated for 5 minutes at room temperature. 12 mL of NEU-EF buffer was added to the lysate, and inverted vigorously to mix thoroughly and rapidly. Before injecting the lysate into the NucleoBond^{®} Xtra column filter, a homogeneous suspension of the precipitate was prepared by inverting the lysate tube 3 times to prevent clogging of the filter. Thereafter, the NucleoBond^{®} Xtra column filter and the NucleoBond^{®} Xtra column were washed with 10 mL of filter wash buffer FIL-EF. The NucleoBond^{®} Xtra column filter was removed by pulling out or inverting the column. The NucleoBond^{®} Xtra column was washed with 90 mL of wash buffer ENDO.

The NucleoBond^{®} Xtra column was washed with 45 mL of wash buffer WASH-EF. Plasmid DNA was eluted with 15 mL of elution buffer ELU. The eluate was collected in a 50 mL centrifugation tube. 10.5 mL of isopropanol at room temperature was added to precipitate the eluted plasmid DNA. After vortexing, the mixture was allowed to stand for 2 minutes.

Thereafter, 5 mL of 70% ethanol was added to the pellet. Ethanol was carefully and completely removed from the tube using a pipette tip. The pellet was dried at room temperature (20 °C to 25 °C). Thereafter, the DNA pellet was dissolved with 1,000 µL of H₂O.

### Manufacturing Example 1.2. Cell transfection and protein expression

Materials and reagents used are described in Table 5 below.

**[Table 5]**

| **Material and reagent** | **Manufacturer (Product #)** |
|---|---|
| 293F cells | Invitrogen (R790-07) |
| OPM 293 | OPM (81075-001) |
| Pluronic^{®} F-68, 10% (100X) | Gibco (24040-032) |
| 1 mg/mL PEI | Polyscience (23966) |
| OPTI MEM I | Gibco (31985088) |
| Peptone (20x) | FLUKA(P0521-1KG) |
| Shaker flask | |
| ISF1-X incubator shaker | Kuhner shaker |

The 293F seed strain containing the complete medium was maintained in an incubator shaker at 130 rpm, 37 °C, and 8% CO₂. The cells were cultured at a density of 0.3 to 0.4 X 10⁶ cells/mL, and the medium was changed every 2 to 3 days. Twenty-four hours before transfection, the freshly subcultured 293F cells were prepared at 2.6 X 10⁶ cells/mL. The prepared cells were cultured in an incubator shaker at 130 rpm, 37 °C, and 8% CO₂. On the day of transfection, a density of cells was adjusted to a density of 5.0 × 10⁶ cells/mL using a fresh medium. It was performed at a total volume of 1 L in a 3 L shaker flask. 0.4 mg of HC and 0.6 mg of LC plasmid were diluted with 50 mL of OPTI MEM^{o} and filtered through a 0.22 µm filter. Thereafter, 2 mg of PEI was diluted with 50 mL of OPTI MEM^{o} to prepare a transfection reagent.

The diluted PEI was added to the DNA mixture and then mixed immediately. Thereafter, it was cultured for 15 minutes at room temperature. The DNA-PEI mixture was added to 293F cells prepared at 2.6 X 10⁶ cells/mL. Thereafter, the cells were continuously cultured for 24 hours in an incubator shaker at 130 rpm, 37 °C, and 8% CO₂. Twenty-four hours after transfection, 10% peptone was added to 1/20 of the culture solution so that the final concentration was 0.5%. Thereafter, the cells were continuously cultured in an incubator shaker at 130 rpm, 37°C, and 8% CO₂. The cell density/viability was measured and recorded daily during the 2 to 5 days period after transfection. The cells were harvested for purification 7 days after transfection or when the cell viability was less than 70%.

### Manufacturing Example 1.3. Protein purification

Reagents, composition of each buffer, and equipment used for protein purification are described in Tables 6 to 8 below.

**[Table 6]**

| **Reagent** | **Manufacturer** | **Catalog#** |
|---|---|---|
| Mabselect SuRe | GE Healthcare | 11003493 |
| Tris | SIGMA | 77-86-1 |
| NaCl | ACROS ORGANIVS | 7647-14-5 |
| Sodium citrate | Adamas-beta | 76198B |
| Citric acid | GENERAL-Reagent | G83162B |
| Arginine | VETEC | V900343-500G |
| Succinic acid | Sigma-Aldrich | S9512-500G |
| Triton X-100 | ABCONE | X10010-1L |
| Triton X-114 | SIGMA-ALDRICH | X114 |
| Millex-GP Filter Unit 0.22 µm Sterile | MILLIPORE | SLGP033RS |
| NaOH | Merck | B146369740 |

**[Table 7]**

| | |
|---|---|
| Buffer A | 25 mM Tris, 150 mM NaCl, pH 8.0 |
| Buffer B | 25 mM Tris, 150 mM NaCl,0.1% Triton X-100, 0.1% Triton X-114 pH 8.0 |
| Buffer C | 100 mM Sodium Citrate, 150 mM NaCl, pH 3.0 |
| Buffer D | 1 M Arginine, 400 mM Succinic acid, pH 9.0 |
| Buffer E | 20 mM PB pH 6.5, 1 M (NH₄)₂SO₄ |
| Buffer F | 20 mM PB pH 6.5, 25% isopropyl alcohol |
| Final buffer | 20 mM HEPES, pH 7.5, 240 mM sucrose or 20 mM his acetate pH 5.5, 240 mM sucrose |

**[Table 8]**

| **Equipment** | **Manufacturer** | **Model name** |
|---|---|---|
| AKTA Pure | GE Healthcare | 29-0182-24 |
| Centrifuge | Beckman | J-26xp |
| Gel Imaging System | Tanon | 2500R |
| Sartopore 2 filter | Sartorius | 5445307H9-OO-A |

The proteins were purified using a MabSelect Sure column. Specifically, the supernatant was harvested by centrifugation at 2,000×g at 4 °C for 20 minutes. Thereafter, the supernatant was filtered with a Sartopore 2 filter. A 5 mL MabSelect Sure column equilibrated with Buffer A was loaded with the clarified supernatant. Thereafter, the column was washed with Buffer A until the A280 absorbance reached the baseline. The column was washed with 10 CV Buffer B. The column was washed with 10 CV Buffer A. the bound proteins were eluted with 6 CV Buffer C, and 1/6 volume of Buffer D was added to neutralize the eluted substance. SDS-PAGE and SEC-HPLC analyses were performed.

The proteins were purified using a HIC column. Thereafter, the proteins were dialyzed against Buffer E at 4 °C overnight. A HIC column equilibrated with Buffer E was loaded with the supernatant. Thereafter, the column was washed with Buffer E until the A280 absorbance reached the baseline. The bound proteins were eluted by gradient elution (10 CV Buffer F 0% to 40%). The bound proteins were eluted with 2 CV 100% Buffer F. SDS-PAGE analysis was performed. In some cases, the proteins were purified using a SEC column. Specifically, the supernatant was loaded onto the SEC column equilibrated with the final buffer, and then the proteins were eluted with the final buffer.

The purified proteins were pooled, and then the proteins were dialyzed against the final buffer at 4 °C overnight. Thereafter, SDS-PAGE and SEC-HPLC analysises were performed.

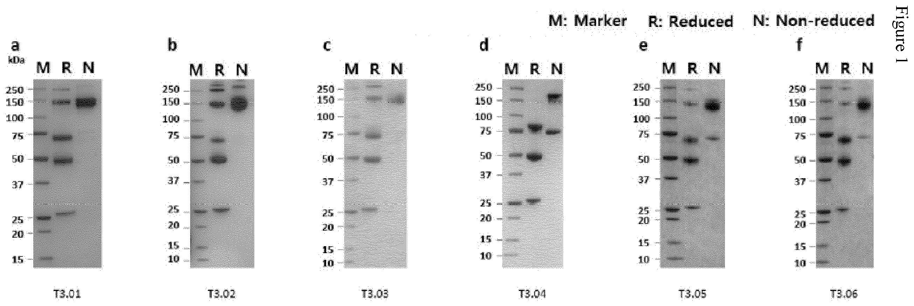

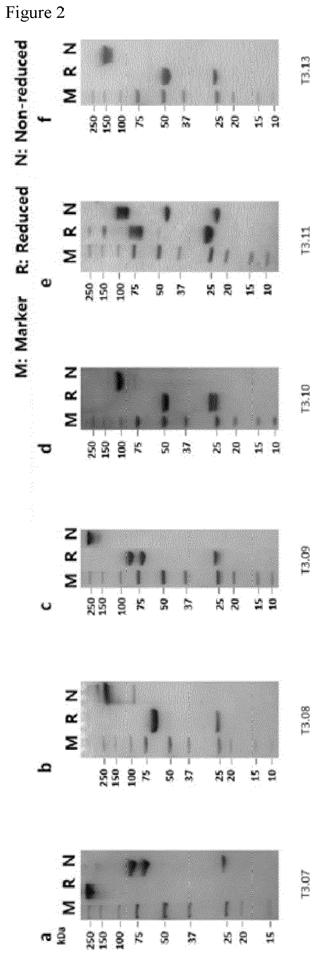

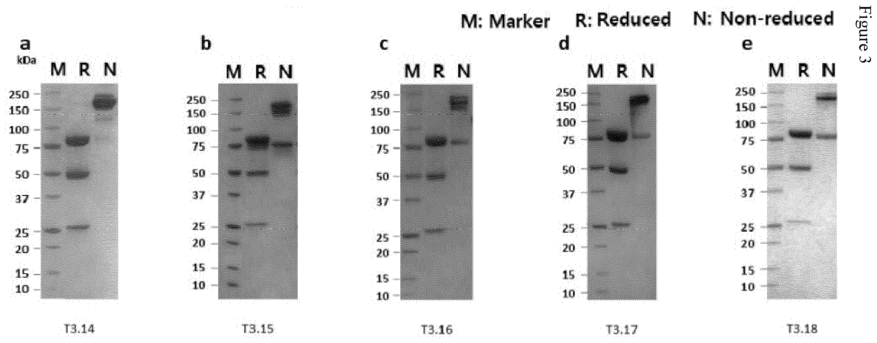

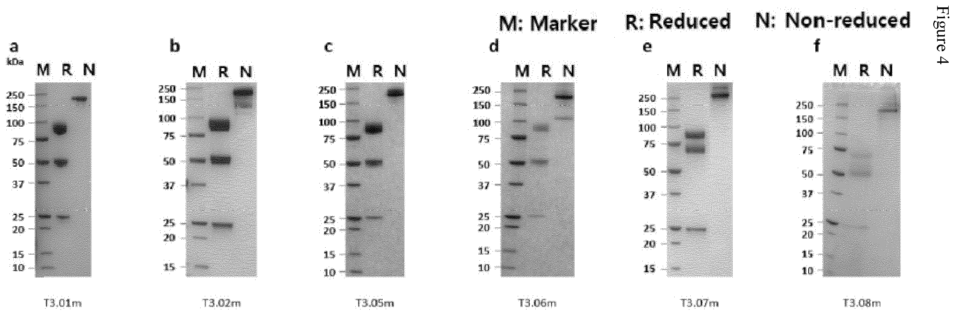

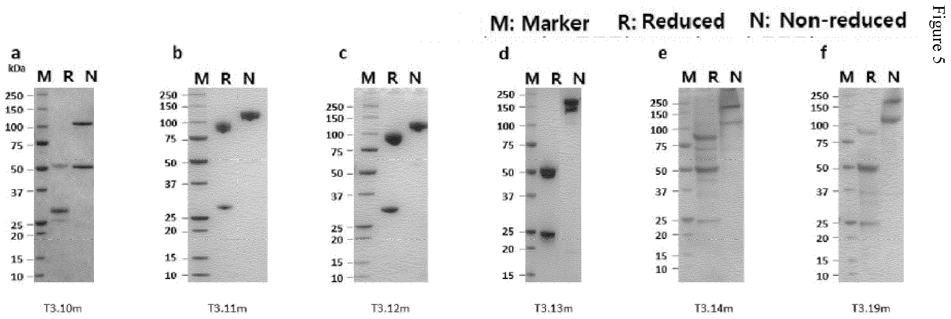

As a result, as shown in Figures 1 to 5, it was found that the human protein and the mouse protein of one embodiment were purified.

### Manufacturing Example 2. Improvement in production of bispecific antibody

### Manufacturing Example 2.1. Confirmation of changes in improvement in production of a fusion protein comprising human LIGHT

Table 9 below shows changes in the production of a human anti-FAP/LIGHT bispecific antibody according to changes in DNA ratio during cell line and cell transfection. The parentheses indicate the production scale of the protein, and the unit is L. The number in front of parentheses is the production per liter obtained by dividing the amount of the purified proteins by the production scale.

**[Table 9]**

| **PROTEIN** | **Description** | **Cell line** | **DNA ratio** | **Amount of protein produced (mg) (production scale, L)** |
|---|---|---|---|---|
| T3.01 | anti-hu FAP/hu scLIGHT3, hu IgG1 DANG | Expi293 | 1:1:2 | 1.2 (1) |
| T3.01 | anti-hu FAP/hu scLIGHT3, hu IgG1 DANG | Expi293 | 1:1:20 | 6.76 (1) |
| T3.01 | anti-hu FAP/hu scLIGHT3, hu IgG1 DANG | CHO | 1:1:5 | 41 (0.03) |

As shown in Table 9 above, the amount of protein produced was increased as the ratio of the DNA of the human anti-FAP sequence and the LIGHT region injected into the cell during protein expression and the cell line were changed. It was found that the amount of T3.01 produced was improved by approximately 5 times as the ratio of the DNA of the LIGHT region was increased by 10 times. It was found that the production yield of T3.01 was improved by approximately 34 times as the ratio of the DNA of the LIGHT region was increased by 2.5 times and a CHO cell line was also used.

### Manufacturing Example 2.2. Confirmation of changes in improvement in production of a fusion protein comprising mouse LIGHT

Table 10 below shows changes in the production of a mouse anti-FAP/LIGHT bispecific antibody according to changes in DNA ratio during cell line and cell transfection.

**[Table 10]**

| **PROTEIN** | **Description** | **Cell line** | **DNA ratio** | **Amount of protein produced (mg) (production scale, L)** |
|---|---|---|---|---|
| T3.01m | anti-mu FAP/mu scLIGHT3, mu IgG2a DANG | Expi293 | 1:1:1 | 1.2 (1) |
| T3.01m | anti-mu FAP/mu scLIGHT3, mu IgG2a DANG | Expi293 | 1:1:1 | 1.6 (10) |
| T3.01m | anti-mu FAP/mu scLIGHT3, mu IgG2a DANG | CHO | 1:1:5 | 5.67 (1) |

As shown in Table 10 above, the amount of protein produced was increased as the ratio of the DNA of the mouse anti-FAP sequence and the LIGHT region injected into the cell during protein expression and the cell line were changed. It was found that the production yield of T3.01m was improved by approximately 4 times as the ratio of the DNA of the LIGHT region was increased by 5 times and a CHO cell line was also used.

### Manufacturing Example 3. Cell lines and culture of cell lines

The human embryonic kidney fibroblast cell line HEK293, the human malignant melanoma cell line A375, the mouse colorectal cancer cell line CT26-WT, the mouse lung cancer cell line LLC1, the mouse melanoma cell line B16F10, and the mouse fibroblast cell line NIH-3T3 were supplied and used from the ATCC (American Type Culture Collection, Manassas, VA, USA). The HEK293 cells, the B16F10 cells, and the NIH-3T3 cells were maintained in DMEM (GIBCO) containing 10% FBS (GIBCO). The cell line (HEK293-hFAP) that overexpressed human FAP (fibroblast activation protein alpha) in the HEK293 cells was constructed using lentivirus that can deliver the human FAP gene. The HEK293-hFAP cells were maintained in DMEM (GIBCO) containing 10% FBS (GIBCO) and 5 µg/mL puromycin.

The cell lines (CT26-mFAP, B16F10-mFAP, and NIH-3T3-mFAP) that overexpressed mouse FAP (fibroblast activation protein alpha) in the CT26-WT cells, the B16F10 cells, and the NIH-3T3 cells were constructed using lentivirus that can deliver the mouse FAP gene. The CT26-mFAP cells were maintained in RPMI-1640 (GIBCO) containing 10% FBS (GIBCO) and 10 µg/mL puromycin. The B16F10-mFAP cells were maintained in DMEM (GIBCO) containing 10% FBS (GIBCO) and 10 µg/mL puromycin. The NIH-3T3-mFAP cells were maintained in DMEM (GIBCO) containing 10% FBS (GIBCO) and 5 µg/mL puromycin.

The NIH-3T3-NFxB (luc) cell line, which is the NIH-3T3 cell line, a mouse fibroblast cell line, transfected with an NFκB-inducible luciferase reporter gene, was supplied and used from BPS Bioscience (San Diego, USA). The NIH-3T3-NFκB(luc) cell line was maintained in DMEM (GIBCO) containing 10% calf serum (GIBCO) and 600 µg/mL Geneticin.

### Manufacturing Example 4. Isolation and activation of human immune cells

A blood pack was supplied from the Korean Red Cross (Korea) with the approval of the Institutional Review Board (IRB), and peripheral blood mononuclear cells (PBMCs) were isolated and frozen. The thawed PBMCs were subjected to the negative selection method, and human T cells were isolated with an Easy sep (Stem cell) kit. The human T cells were maintained in RPMI-1640 (GIBCO) containing 10% FBS (GIBCO).

### Example 1. Confirmation of binding affinity of fusion proteins

### Example 1.1. Confirmation of binding of fusion proteins to recombinant human FAP

The binding of T3.01, T3.07, and T3.14 to recombinant human FAP was confirmed by surface plasmon resonance (SPR).

Specifically, the surface of the CM5 chip was activated with a 1:1 mixture of 50 nM NHS (N-hydroxysuccinimide) and 200 nM EDC (1-ethyl-3-(3-dimethylaminopropyl)carbodiimide), and 20 µg/mL of anti-human IgG (Fc) antibody was immobilized for 400 seconds at a rate of 10 µL/min. The remaining active ester groups were blocked with 1 M ethanolamine. T3.01, T3.07, and T3.14 were diluted to 2 µg/mL, 1 µg/mL, and 2 µg/mL, respectively, and reacted on the CM5 chip immobilized with the anti-human IgG (Fc) antibody. The recombinant human FAP was diluted to 50 nM or 100 nM in 1x HBS-EP + buffer solution and diluted by serial dilution. The diluted recombinant human FAP was reacted at a rate of 30 µL/min. The association and dissociation times were 180 seconds and 400 seconds, respectively. After the dissociation step, stabilization was performed for 60 seconds, and then the regeneration step was performed for 30 seconds with a 10 mM glycine pH 1.5 solution at a rate of 30 µL/min.

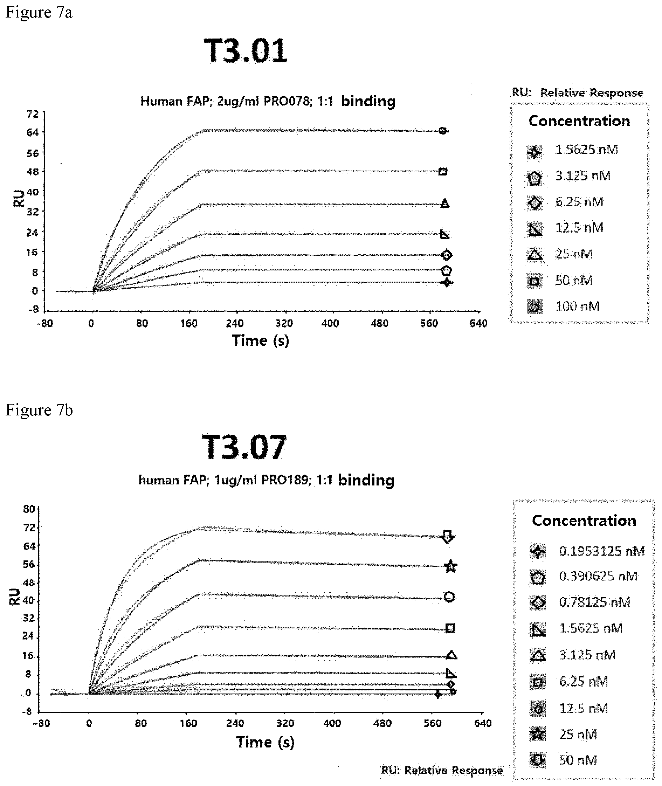

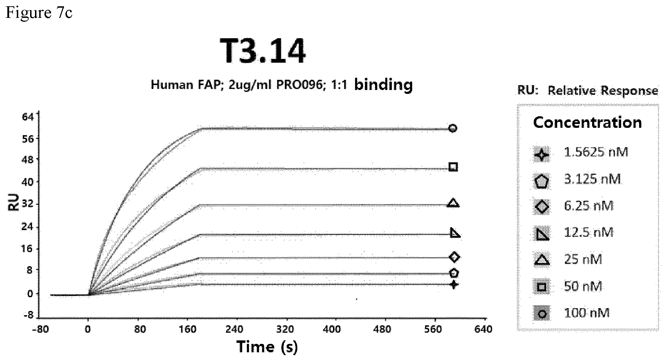

As a result, as shown in Table 11 and Figure 7, it was found that T3.01, T3.07, and T3.14 can specifically bind to recombinant human FAP and specifically target FAP.

**[Table 11]**

| **Kinetics model** | **Capture 1 Solution** | **Analyte 1 Solution** | **Kinetics Chi² (RU²)** | **ka (1/Ms)** | **kd (1/s)** | **KD (M)** |
|---|---|---|---|---|---|---|
| 1:1 binding | 2 µg/mL T3.01 | human FAP | 2.18E-01 | 1.07E+05 | 3.89E-06 | 3.64E-11 |
| 1:1 binding | 1 µg/mL T3.07 | human FAP | 4.98E-01 | 3.91E+05 | 1.18E-04 | 3.03E-10 |
| 1:1 binding | 2 µg/mL T3.14 | human FAP | 3.24E-01 | 1.24E+05 | 1.53E-06 | 1.23E-11 |

### Example 1.2. Confirmation of binding of fusion proteins to LIGHT receptor human LTβR

The binding of T3.01, T3.07, T3.09, T3.14, and T3.18 to human LTβR was confirmed by surface plasmon resonance.

Specifically, the surface of the CM5 chip was activated with a 1:1 mixture of 50 nM NHS and 200 nM EDC, and 5 µg/mL of human LTβR was immobilized for 60 seconds at a rate of 10 µL/min. The remaining active ester groups were blocked with 1 M ethanolamine. T3.01, T3.07, T3.09, T3.14, and T3.18 were each diluted to one of 12.5 nM, 25 nM, and 100 nM in 1x HBS-EP + buffer solution and diluted by serial dilution. The diluted fusion proteins were reacted at a rate of 30 µL/min. The association and dissociation times were 180 seconds and 400 seconds, respectively. After the dissociation step, stabilization was performed for 60 seconds, and then the regeneration step was performed for 30 seconds with a 10 mM glycine pH 1.5 solution at a rate of 30 µL/min.

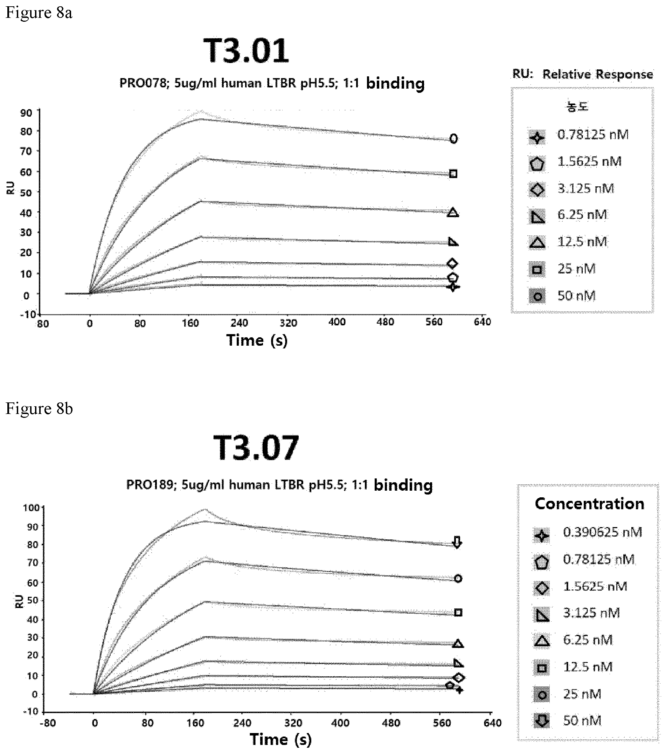

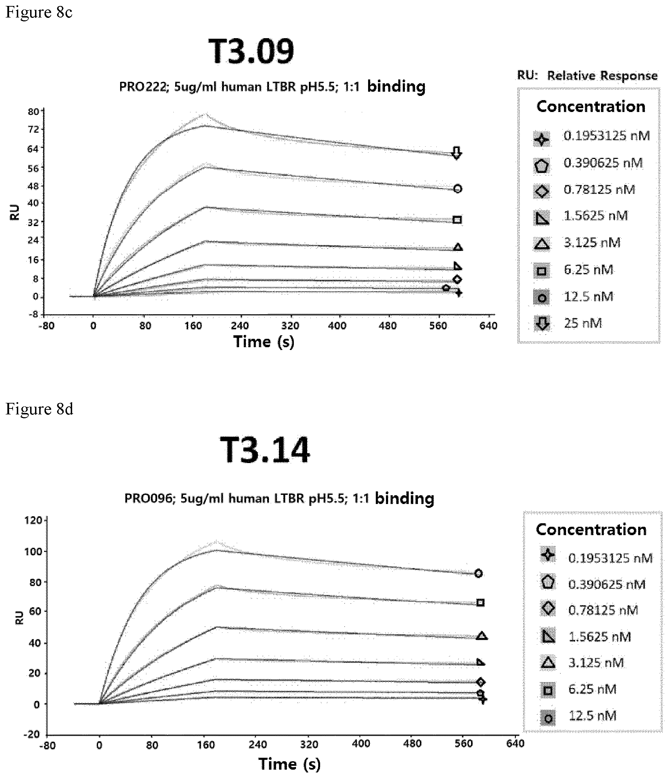

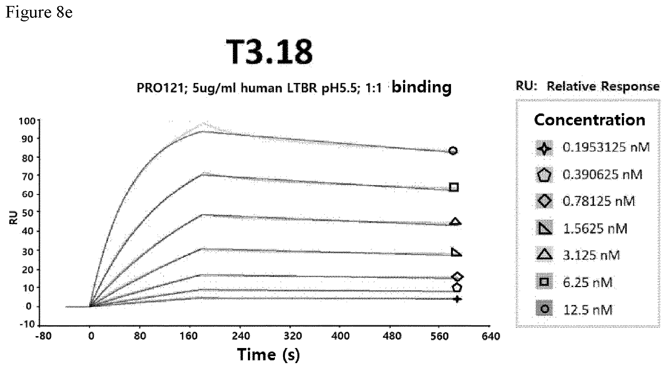

As a result, as shown in Table 12 and Figure 8, it was found that T3.01, T3.07, T3.09, T3.14, and T3.18 bind to human LTβR.

**[Table 12]**

| **Kinetics model** | **Capture 1 Solution** | **Analyte 1 Solution** | **Kinetics Chi² (RU²)** | **ka (1/Ms)** | **kd (1/s)** | **KD (M)** |
|---|---|---|---|---|---|---|
| 1:1 binding | 5 µg/mL human LTβR | T3.01 | 4.66E-01 | 3.46E+05 | 3.21E-04 | 9.28E-10 |
| 1:1 binding | 5 µg/mL human LTβR | T3.07 | 8.89E-01 | 4.09E+05 | 3.82E-04 | 9.35E-10 |
| 1:1 binding | 5 µg/mL human LTβR | T3.09 | 5.40E-01 | 7.70E+05 | 4.83E-04 | 6.28E-10 |
| 1:1 binding | 5 µg/mL human LTβR | T3.14 | 6.14E-01 | 1.50E+06 | 4.34E-04 | 2.90E-10 |
| 1:1 binding | 5 µg/mL human LTβR | T3.18 | 3.76E-01 | 1.29E+06 | 3.15E-04 | 2.45E-10 |

### Example 1.3. Confirmation of binding of fusion proteins to LIGHT receptor human HVEM

The binding of T3.01, T3.07, T3.09, T3.14, and T3.18 to human HVEM was confirmed by surface plasmon resonance.

Specifically, the surface of the CM5 chip was activated with a 1:1 mixture of 50 nM NHS and 200 nM EDC, and 5 to 6 µg/mL of human HVEM was immobilized for 60 seconds at a rate of 10 µL/min. The remaining active ester groups were blocked with 1 M ethanolamine. T3.01, T3.07, T3.09, T3.14, and T3.18 were each diluted to 100 nM in 1x HBS-EP + buffer solution and diluted by serial dilution. The diluted fusion proteins were reacted at a rate of 30 µL/min. The association and dissociation times were 180 seconds and 400 seconds, respectively. After the dissociation step, stabilization was performed for 60 seconds, and then the regeneration step was performed for 30 seconds with a 10 mM glycine pH 1.5 solution at a rate of 30 µL/min.

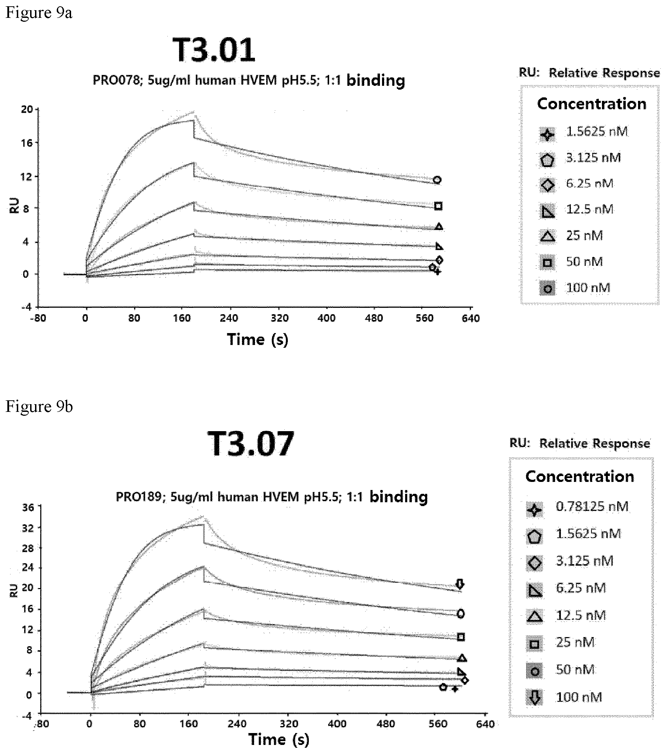

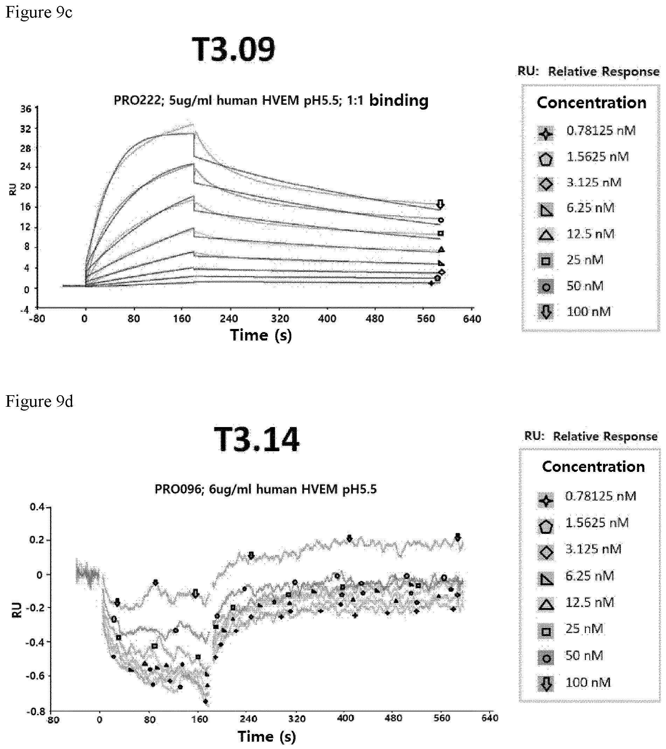

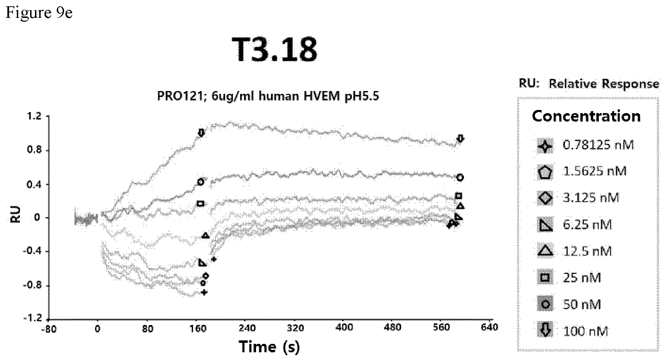

As a result, as shown in Table 13 and Figure 9, it was found that T3.01, T3.07, and T3.09 bind to human HVEM.

**[Table 13]**

| **Kinetics model** | **Capture 1 Solution** | **Analyte 1 Solution** | **Kinetics Chi² (RU²)** | **ka (1/Ms)** | **kd (1/s)** | **KD (M)** |
|---|---|---|---|---|---|---|
| 1:1 binding | 5 µg/mL human HVEM | T3.01 | 6.65E-02 | 2.81E+05 | 1.19E-03 | 4.24E-09 |
| 1:1 binding | 5 µg/mL human HVEM | T3.07 | 2.16E-01 | 2.85E+05 | 1.14E-03 | 3.99E-09 |
| 1:1 binding | 5 µg/mL human HVEM | T3.09 | 2.40E-01 | 4.01E+05 | 1.67E-03 | 4.17E-09 |
| 1:1 binding | 6 µg/mL human HVEM | T3.14 | N/A | N/A | N/A | N/A |
| 1:1 binding | 6 µg/mL human HVEM | T3.18 | N/A | N/A | N/A | N/A |

### Example 1.4. Confirmation of binding of fusion proteins to LIGHT receptor human DcR3

The binding of T3.01, T3.07, T3.09, T3.14, and T3.18 to human DcR3 was confirmed by surface plasmon resonance.

Specifically, the surface of the CM5 chip was activated with a 1:1 mixture of 50 nM NHS and 200 nM EDC, and 5 to 6 µg/mL of human DcR3 was immobilized for 60 seconds at a rate of 10 µL/min. The remaining active ester groups were blocked with 1 M ethanolamine. T3.01, T3.07, T3.09, T3.14, and T3.18 were each diluted to 50 nM or 200 nM in 1x HBS-EP + buffer solution and diluted by serial dilution. The diluted fusion proteins were reacted at a rate of 30 µL/min. The association and dissociation times were 180 seconds and 400 seconds, respectively. After the dissociation step, stabilization was performed for 60 seconds, and then the regeneration step was performed for 30 seconds with a 10 mM glycine pH 1.5 solution at a rate of 30 µL/min.

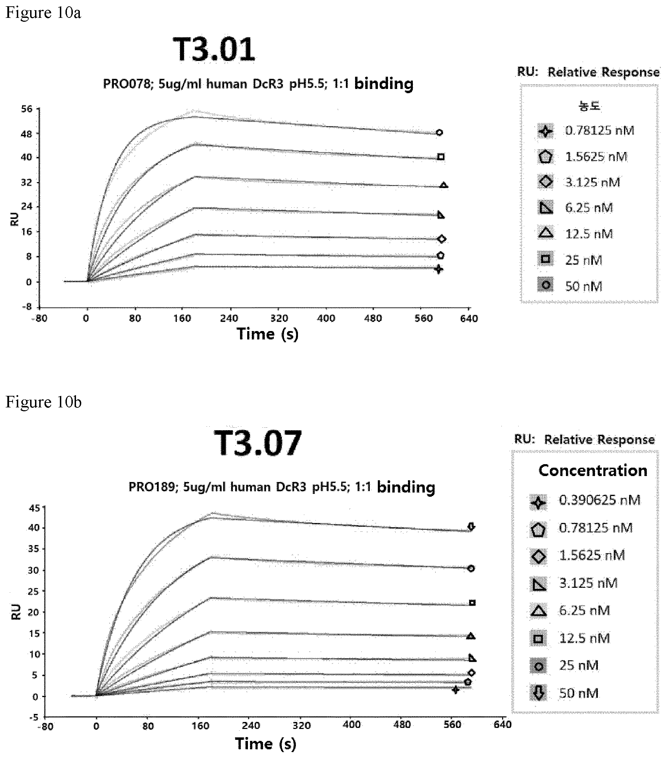

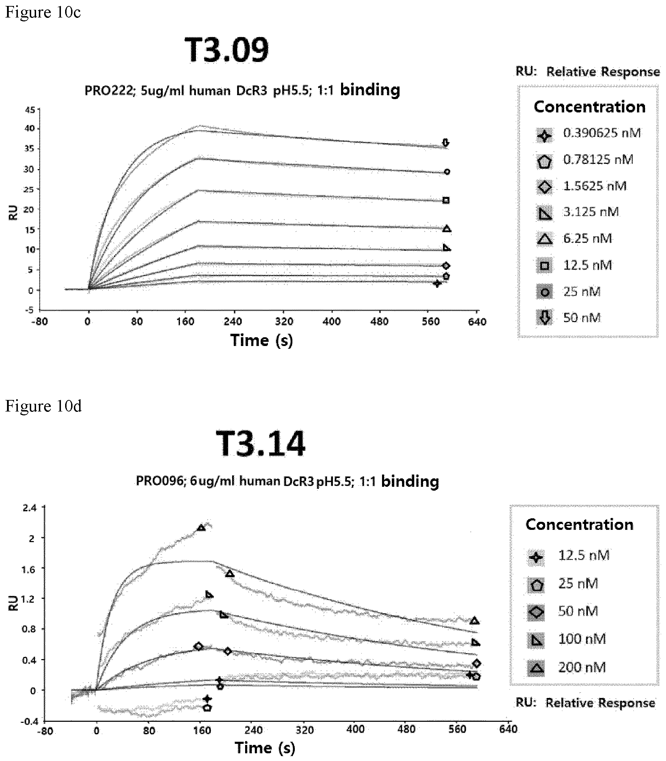

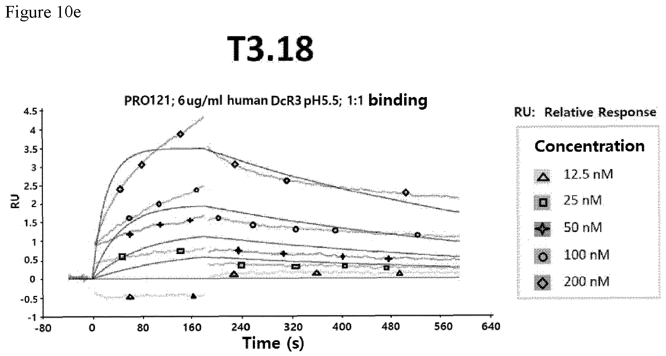

As a result, as shown in Table 14 and Figure 10, it was found that T3.01, T3.07, T3.09, T3.14, and T3.18 bind to human DcR3.

**[Table 14]**

| **Kinetics model** | **Capture 1 Solution** | **Analyte 1 Solution** | **Kinetics Chi² (RU²)** | **ka (1/Ms)** | **kd (1/s)** | **KD (M)** |
|---|---|---|---|---|---|---|
| 1:1 binding | 5 µg/mL human DcR3 | T3.01 | 1.51E-01 | 6.62E+05 | 2.36E-04 | 3.57E-10 |
| 1:1 binding | 5 µg/mL human DcR3 | T3.07 | 1.55E-01 | 3.27E+05 | 1.95E-04 | 5.98E-10 |
| 1:1 binding | 5 µg/mL human DcR3 | T3.09 | 1.84E-01 | 4.00E+05 | 2.85E-04 | 7.11E-10 |
| 1:1 binding | 6 µg/mL human DcR3 | T3.14 | 2.10E-02 | 2.04E+05 | 1.98E-03 | 9.72E-09 |
| 1:1 binding | 6 µg/mL human DcR3 | T3.18 | 6.95E-02 | 2.14E+05 | 1.70E-03 | 7.93E-09 |

### Example 1.5. Confirmation of binding to FAP-expressing cells

The degree of binding of T3.10, T3.01, T3.05, T3.06, T3.14, and T3.18 to HEK293 and HEK293-hFAP cells was confirmed.

Specifically, the cell line was prepared by suspending in a FACS buffer solution at 1 × 10⁵ cells/100 µL, and treated with 1 µg each of T3.10, T3.01, T3.05, T3.06, T3.14, and T3.18. The cells were washed twice with a FACS buffer solution. The cells were stained with an anti-human IgG antibody (Biolegend). The negative control sample was stained only with an anti-human IgG antibody (Biolegend). The expression ratio of the stained cells was measured using BD LSR and analyzed using FlowJo software.

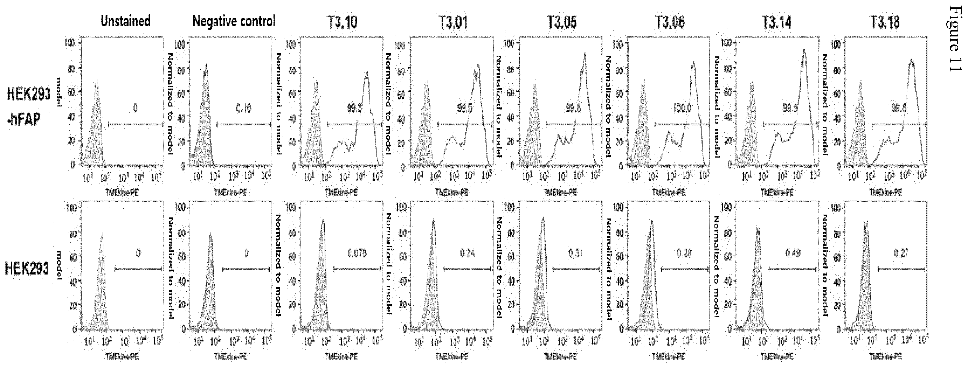

As a result, as shown in Figure 11, it was found that T3.10, T3.01, T3.05, T3.06, T3.14, and T3.18 bind to the human FAP-expressing cell line by 99% or more. These results indicate that the fusion protein of one embodiment can bind to a human FAP-expressing cell line and specifically target FAP. On the other hand, the fusion protein did not bind to the negative control HEK293 cell line.

In addition, the degree of binding of T3.10m and T3.01m to B16F10 and B16F10-mFAP cells was confirmed.

Specifically, the cell line was prepared by suspending in a FACS buffer solution at 1 x 10⁵ cells/100 µL, and treated with 1 µg each of T3.10m and T3.01m. The cells were washed twice with a FACS buffer solution. The cells were stained with an anti-mouse IgG2a antibody (Biolegend). The negative control sample was stained only with an anti-mouse IgG2a antibody (Biolegend). The expression ratio of the stained cells was measured using BD LSR and analyzed using FlowJo software.

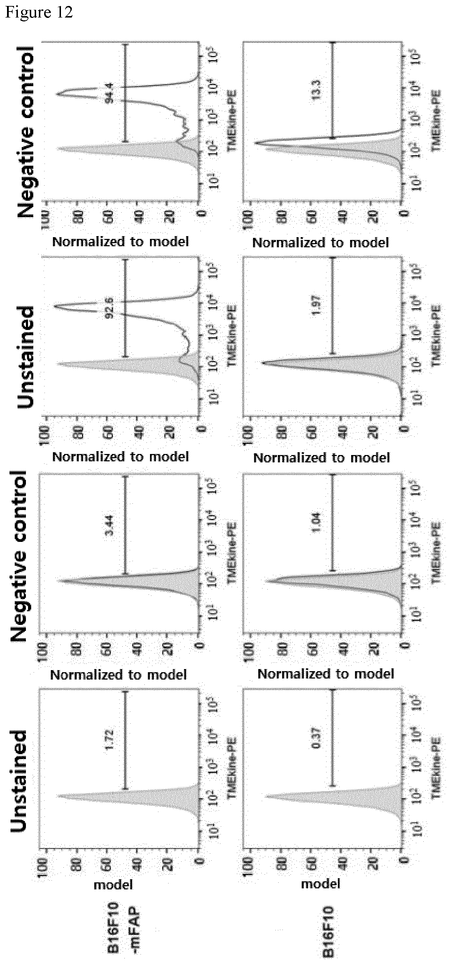

As a result, as shown in Figure 12, it was found that T3.10m and T3.01m bind to the FAP-expressing cell line by 99% or more. These results indicate that the fusion protein of one embodiment can bind to a mouse FAP-expressing cell line and specifically target FAP. On the other hand, the fusion protein did not bind to the negative control B16F10 cell line.

### Example 1.6. Confirmation of binding to LTβR expressing cells

The degree of binding of T3.01, T3.07, T3.04, and T3.18 to LTβR expressing A375 cells was confirmed.

Specifically, the A375 cell line was prepared by suspending in a FACS buffer solution at 1 x 10⁵ cells/100 µL, and treated with 100 µL each of T3.01, T3.07, T3.04, and T3.18 prepared by suspending at 500 nM and diluting by serial dilution. The cells were washed twice with a FACS buffer solution. The cells were stained with an anti-human IgG antibody (Biolegend). The expression ratio of the stained cells was measured using BD LSR and analyzed using FlowJo software.

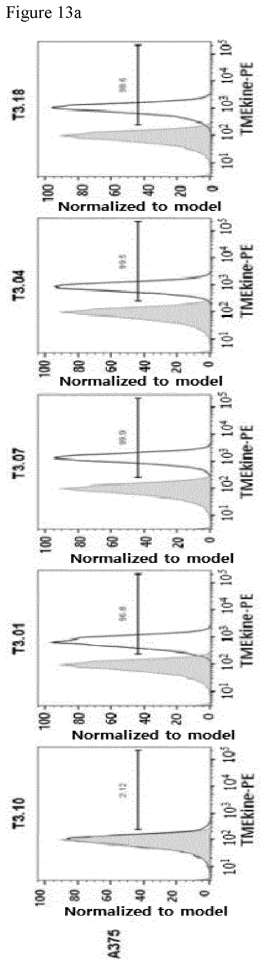

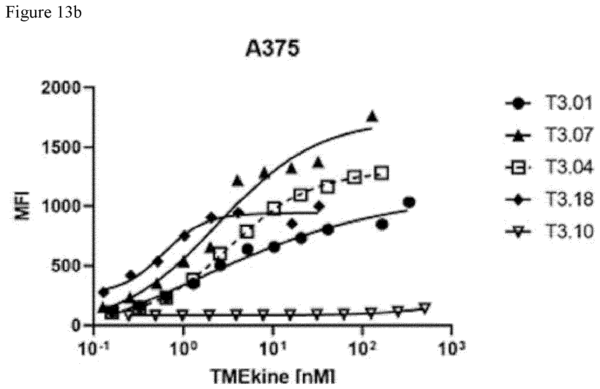

As a result, as shown in Figures 13a and 13b, it was found that T3.01, T3.07, T3.04, and T3.18 bind to the LTβR expressing cell line by 96% or more and bind in a concentration-dependent manner. These results indicate that the fusion protein of one embodiment can bind to a LTβR expressing cell line and specifically target LTβR. T3.10 was used as a negative control.

### Example 1.7. Confirmation of binding of the fusion protein to recombinant human herpes virus entry mediator

Considering that LIGHT of the fusion protein must bind to the herpes virus entry mediator (HVEM) for the action, it was confirmed that T3.01 binds to the recombinant human HVEM, as follows:
(i) The recombinant human HVEM protein (R&D systems) was suspended at 1 µg/mL, dispensed in a 96-well immune plate, and treated for 24 hours.
(ii) The wells were washed with a wash solution, and then blocking was performed for 1 hour with 1% BSA (bovine serum albumin, Sigma) solution.
(iii) The wells were washed with a wash solution, and then T3.01, T3.14, T3.16, T3.17, T3.18 and T3.10, or T3.05, T3.06, T3.04, T3.14 and T3.10 were suspended at 1 nM and diluted by serial dilution. The wells were treated with the diluted T3.01, T3.14, T3.16, T3.17, T3.18 and T3.10, or T3.05, T3.06, T3.04, T3.14 and T3.10 for 2 hours.
(iv) The wells were washed with a wash solution, and then treated with the human recombinant FAP-biotin diluted at 0.5 µg/mL for 2 hours.
(v) The wells were washed with a wash solution, and then treated with streptavidin-HRP for 20 minutes.
(vi) The wells were washed with a wash solution, and then treated with a substrate solution for 20 minutes.
(vii) The wells were treated with a stop solution, and the absorbance was measured at 450 nm.

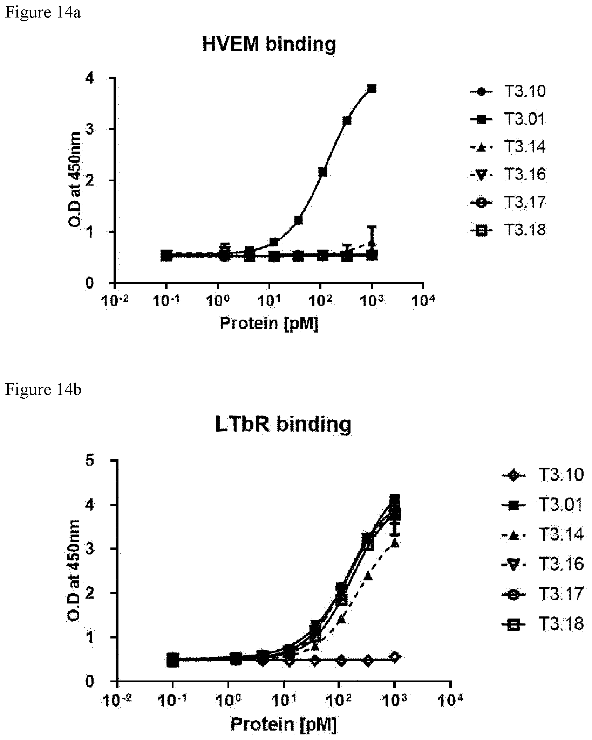

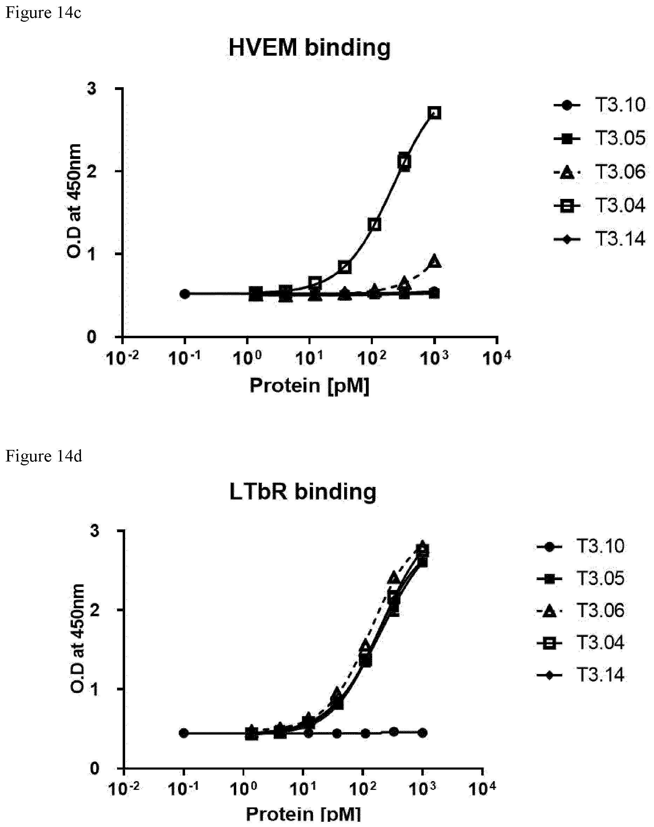

As a result, as shown in Figures 14a and 14c, it was found that T3.01 and T3.04 bind to the recombinant human HVEM protein in a concentration dependent manner. T3.10 was used as a negative control.

### Example 1.8. Confirmation of binding of the fusion protein to recombinant human lymphotoxin beta receptor (LTβR)

Considering that LIGHT of the fusion protein must bind to the lymphotoxin beta receptor (LTβR) for the action, it was confirmed that T3.01, T3.14, T3.16, T3.17, T3.18 and T3.10, or T3.05, T3.06, T3.04, T3.14 and T3.10 bind to the recombinant human LTβR, as follows:
(i) The recombinant human LTβR protein was suspended at 1 µg/mL, dispensed in a 96-well immune plate, and treated for 24 hours.
(ii) The wells were washed with a wash solution, and then blocking was performed for 1 hour with 1% BSA (bovine serum albumin, Sigma) solution.
(iii) The wells were washed with a wash solution, and then T3.01, T3.14, T3.16, T3.17, T3.18 and T3.10, or T3.05, T3.06, T3.04, T3.14 and T3.10 were suspended at 1 nM and diluted by serial dilution. The wells were treated with the diluted T3.01, T3.14, T3.16, T3.17, T3.18 and T3.10, or T3.05, T3.06, T3.04, T3.14 and T3.10 for 2 hours.
(iv) The wells were washed with a wash solution, and then treated with the human recombinant FAP-biotin diluted at 0.5 µg/mL for 2 hours.
(v) The wells were washed with a wash solution, and then treated with streptavidin-HRP for 20 minutes.
(vi) The wells were washed with a wash solution, and then treated with a substrate solution for 20 minutes.
(vii) The wells were treated with a stop solution, and the absorbance was measured at 450 nm.

As a result, as shown in Figures 14b and 14d, it was found that T3.01, T3.14, T3.16, T3.17, T3.18, T3.05, T3.06, T3.04, and T3.14 bind to the recombinant human LTβR protein in a concentration dependent manner. T3.10 was used as a negative control.

### Example 2. Confirmation of cytokine secretion ability of fusion proteins

### Example 2.1. Enzyme-linked immunoprecipitation assay for cytokine measurement

For cytokine measurement, enzyme-linked immunoprecipitation assay (enzyme-linked immunosorbent assay, ELISA, R&D systems) was performed on the supernatant stored at - 80 °C or -20 °C in the following steps:
(i) The capture antibody was diluted according to the Certificate of Analysis (CoA) and coated on a 96-well plate for 24 hours.
(ii) The wells were washed with a wash solution, and then blocking was performed for 1 hour with 1% BSA solution.
(iii) The wells were washed with a wash solution, and then treated with the samples for 2 hours.
(iv) The wells were washed with a wash solution, and then the detection antibody was diluted according to the Certificate of Analysis and dispensed in the wells, and then the wells were treated therewith for 2 hours.
(v) The wells were washed with a wash solution, and then treated with streptavidin-HRP for 20 minutes.
(vi) The wells were washed with a wash solution, and then treated with a substrate solution for 20 minutes.
(vii) The wells were treated with a stop solution, and the absorbance was measured at 450 nm.

### Example 2.2. Confirmation of cytokine secretion ability in the A375 cell line

Considering that IL-8 secretion occurs through NFκB signaling by LTβR-mediated reaction when the A375 cell line is treated with LIGHT, IL-8 secretion ability was evaluated when the A375 cell line was treated with the recombinant human LIGHT, T3.01, T3.04, T3.14, T3.15 and T3.10, or the recombinant human LIGHT, T3.01, T3.07, T3.18 and T3.10.

Specifically, the A375 cell line was diluted to 1 x 10⁵ cells/mL and dispensed in a 96-well plate at 100 µL/well. After 24 hours, the recombinant human LIGHT, T3.01, T3.04, T3.14, T3.15 and T3.10, or the recombinant human LIGHT (R&D systems), T3.01, T3.07, T3.18 and T3.10 were diluted to 10 nM and dispensed at 100 µL/well. After 6 hours of treatment, the cell supernatant was collected, and the samples were stored at -80°C.

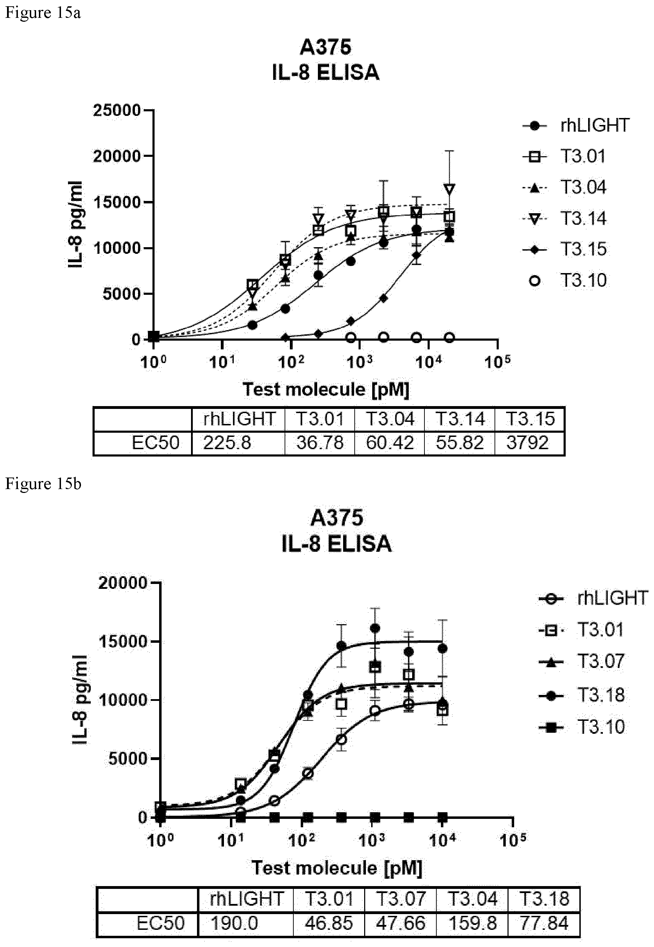

As a result, as shown in Figure 15a, it was found that the sample treated with the recombinant human LIGHT had a half maximal effective concentration (EC50) of 225.8 pM; the sample treated with T3.01 had an EC50 of 36.78 pM; the sample treated with T3.04 had an EC50 of 60.42 pM; the sample treated with T3.14 had an EC50 of 55.82 pM; and the sample treated with T3.15 had an EC50 of 3792 pM. In contrast, IL-8 was not detected in the sample treated with the control, T3.10. As shown in Figure 15b, , it was found that the sample treated with the recombinant human LIGHT had an EC50 of 190 pM; the sample treated with T3.01 had an EC50 of 46.85 pM; the sample treated with T3.07 had an EC50 of 47.66 pM; and the sample treated with T3.18 had an EC50 of 77.84 pM. In contrast, IL-8 was not detected in the sample treated with the control, T3.10.

These results indicate that T3.01, T3.04, T3.14, T3.15, T3.07, and T3.18 act on the A375 cells to induce IL-8 secretion.

### Example 2.3. Confirmation of cytokine secretion ability in human T cells

Considering that IFN-γ secretion, which is a major immune response, occurs when human T cells are treated with LIGHT, IFN-γ secretion ability was evaluated when human T cells were treated with T3.01, T3.04, T3.14, T3.15 and T3.10, or T3.01, T3.07, T3.04, T3.18 and T3.10.

Specifically, the human T cells were diluted to 5 x 10⁵ cells/mL and dispensed in a 96-well plate coated with 1 µg/mL anti-CD3 antibody (OKT3, Invitrogen) at 200 µL/well. T3.01, T3.04, T3.14, T3.15, and T3.10 were diluted to 20 nM or 10 nM and dispensed at 20 µL/well.

After 48 hours of treatment, the cell supernatant was collected, and the samples were stored at -80 °C.

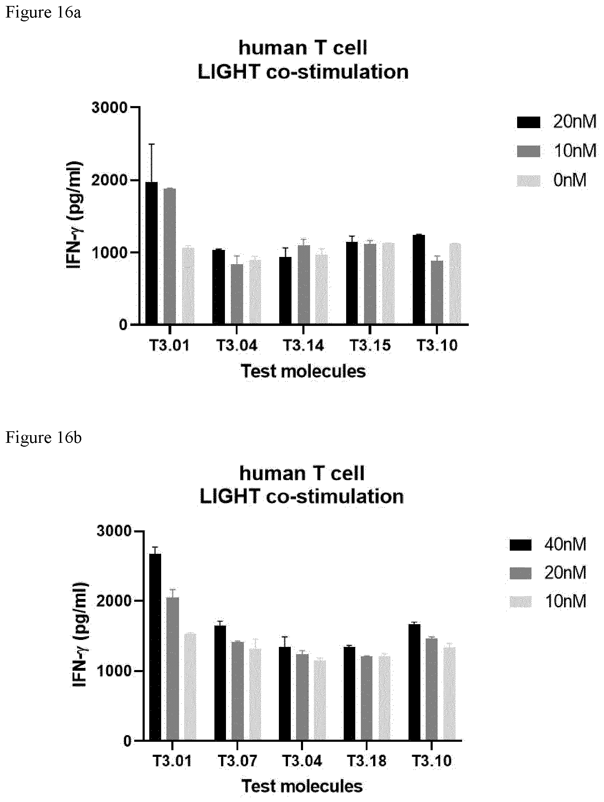

As a result, as shown in Figure 16a, an average of 1,977.5 pg/mL IFN-γ was measured in the sample treated with T3.01 at a concentration of 20 nM. In contrast, it was found that the amount of IFN-γ in the samples treated with T3.04, T3.14, and T3.15 was not different from the amount of IFN-γ determined in the sample treated with T3.10, which was the control.

Specifically, the human T cells were diluted to 5 x 10⁵ cells/mL and dispensed in a 96-well plate coated with 1 µg/mL anti-CD3 antibody (OKT3, Invitrogen) at 200 µL/well. T3.01, T3.07, T3.04, T3.18, and T3.10 were diluted to 40 nM, 20 nM, or 10 nM and dispensed at 20 µL/well. After 48 hours of treatment, the cell supernatant was collected, and the samples were stored at -80 °C.

As a result, as shown in Figure 16b, an average of 2,671 pg/mL IFN-γ was measured in the sample treated with T3.01 at a concentration of 40 nM. In contrast, it was found that the amount of IFN-γ in the samples treated with T3.07, T3.04, and T3.18 was not different from the amount of IFN-γ determined in the sample treated with T3.10, which was the control.

These results indicate that T3.01 acts on human T cells to induce IFN-γ secretion.

### Example 3. Confirmation of ability of fusion proteins to induce signaling

### Example 3.1. Confirmation of signaling ability in LIGHT recognizing cells

Considering that LIGHT is known to induce the signal transduction of NFκB, a signaling mediator, when LIGHT binds to LTβR, the signaling ability of the fusion protein of one embodiment in the LTβR expressing NIH-3T3-NFκB (Luc) cells was evaluated.

Specifically, the NIH-3T3-NFκB (Luc) cell line was diluted to 5 x 10⁵ cells/mL and dispensed at 50 µL/well. The recombinant mouse TNF-α (R&D systems), T3.01m, and T3.10m were suspended at 4 µg/mL and diluted by serial dilution. The NIH-3T3-NFκB (Luc) cells were treated with the diluted recombinant mouse TNF-α, T3.01m, and T3.10m. After 6 hours of treatment, the cell supernatant was collected and mixed with a One-step^{™} Luciferase assay reagent (BPS bioscience) in a 96-well plate. After 30 minutes of mixing, the luminescence degree was measured using a Lumenometer (Thermo Fisher).

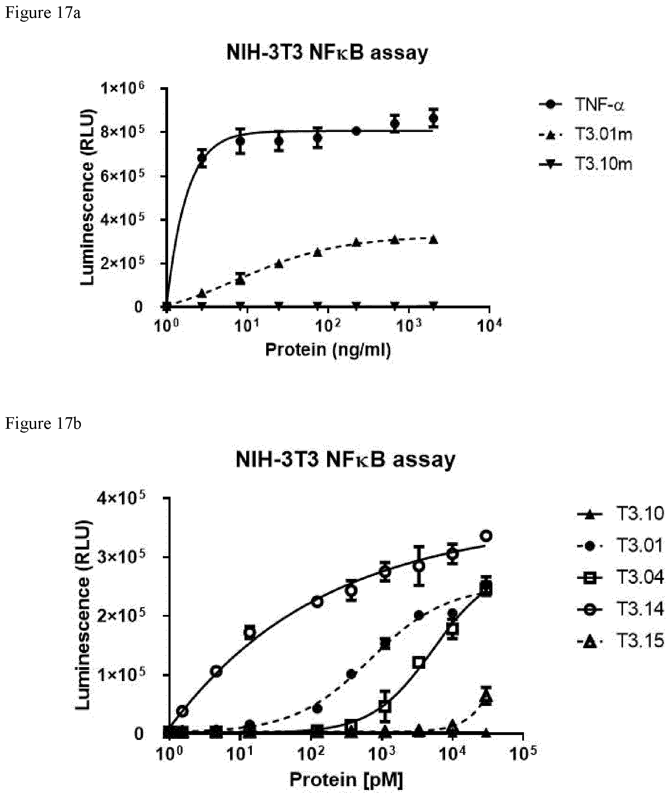

As a result, as shown in Figure 17a, in the case of the group treated with T3.01m, it was confirmed by luminescence degree that LIGHT bound to NFκB and transmitted the NFκB signal. In contrast, it was confirmed that the control, T3.10m, did not bind to LIGHT when the NIH-3T3-NFκB (Luc) cells were treated with T3.10m. These results indicate that the fusion protein of one embodiment specifically binds to LTβR and induces signal transduction.

### Example 3.2. Confirmation of cross-species response of human LIGHT

In order to confirm whether human LIGHT has cross-species response with mouse LIGHT, the signaling ability of the fusion protein of one embodiment in the LTβR expressing NIH-3T3-NFκB (Luc) cells was evaluated.

Specifically, the NIH-3T3-NFκB (Luc) cell line was diluted to 5 x 10⁵ cells/mL and dispensed at 50 µL/well. T3.01, T3.04, T3.14, and T3.15 were suspended at 30 nM and diluted by serial dilution. The NIH-3T3-NFκB (Luc) cells were treated with T3.01, T3.04, T3.14, and T3.15. After 6 hours of treatment, the cell supernatant was collected and mixed with a One-step^{™} Luciferase assay reagent (BPS bioscience) in a 96-well plate. After 30 minutes of mixing, the luminescence degree was measured using a Lumenometer (Thermo Fisher).

As a result, as shown in Figure 17b, in the case of the groups treated with T3.14, T3.01, and T3.04, it was confirmed by luminescence degree that human LIGHT bound to mouse LTβR and transmitted the NFκB signal. In contrast, it was confirmed that in the group treated with T3.15, luminescence degree was weak only at a high concentration of 10⁴ pM or higher, and it was confirmed that the control, T3.10, did not bind to LIGHT when the NIH-3T3-NFκB (Luc) cells were treated with T3.10. These results indicate that the fusion protein of one embodiment specifically binds to mouse LTβR through a cross-species response and induces signal transduction.

### Example 4. Evaluation of anti-cancer efficacy of the fusion protein in colorectal cancer animal model

### Example 4.1. Evaluation of anti-cancer efficacy in FAP-expressing colorectal cancer animal model

Considering that FAP expression is high in the tumor cells themselves in the case of colorectal cancer, the tumor growth inhibition ability was evaluated in a tumor model in which mouse FAP overexpression was induced in CT26 cells, a mouse colorectal cancer cell line, in order to evaluate the anti-cancer effect.

In order to construct a FAP-expressing tumor animal model, mouse FAP was overexpressed in CT26, a mouse colorectal cancer cell line. Specifically, the CT26-mFAP cell line overexpressing mouse FAP in culture was resuspended in Hanks' Balanced salt solution (HBSS, Gibco), and then 1 x 10⁶ tumor cells were transplanted into the left dorsal subcutaneous region of 6-week-old BALB/c mice using a 1 cc syringe (25G) at 100 µL per mouse to obtain a FAP-expressing colorectal cancer animal model. The tumor size was measured along the short axis and the long axis of the tumor using a digital vernier caliper, and the tumor size was measured twice a week using the calculation formula: (short axis, mm)² x (long axis, mm) x 0.5.

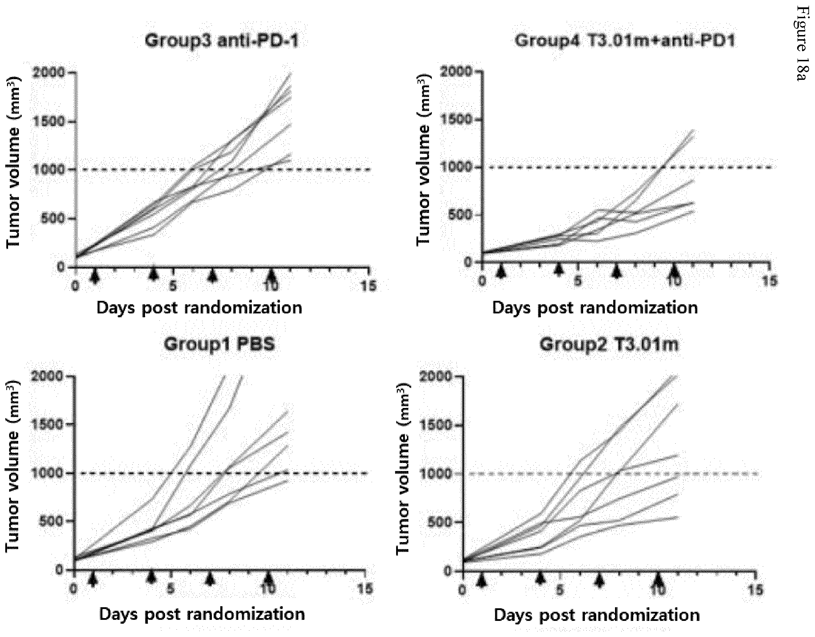

Next, for efficacy evaluation, when the tumor size reached 100 mm³, the mice were randomly divided into groups. Each experimental group consisted of 7 mice, and PBS was administered intraperitoneally once every 3 days for a total of 4 times as a control group. The group administered with T3.01m alone, the group administered with aPD-1 (Clone RMP1-14, BioXCell), immune checkpoint inhibitor, alone, and the group administered with T3.01m and aPD-1 in combination were used as experimental groups to evaluate the tumor growth inhibition ability. T3.01m at a dose of 100 µg and aPD-1 at a dose of 200 µg were administered intraperitoneally once every 3 days for 4 times, and the tumor growth inhibition ability was observed. The measured tumor size was displayed as a graph for each subject (Figure 18a), and it was found that tumor growth was inhibited in the group administered with the fusion protein and the anti-PD-1 antibody in combination.

Specifically, in the above experimental groups, tumor tissues were harvested from 4 or 5 mice in each group on the 11th day after the first intraperitoneal injection using a treatment method according to the IACUC regulations. Immune cells were isolated from the harvested tumor tissues using a tumor dissociation kit (Miltenyi, Germany). The cells were stained using anti-mouse CD45 antibody (Biolegend), anti-mouse CD3 antibody (BD), anti-mouse CD4 antibody (Biolegend), anti-mouse CD8 antibody (eBioscience), anti-mouse CCR7 antibody (Biolegend), anti-mouse CD19 antibody (Biolegend), anti-mouse CD11c antibody (Biolegend), anti-mouse CD49b antibody (Biolegend), anti-mouse CD11b antibody (Biolegend), anti-mouse Gr-1 antibody (Biolegend), Live/Dead (eBioscience), and Counting beads (eBioscience). The expression ratio of the stained cells was measured using BD LSR and analyzed using FlowJo software.

As a result, as shown in Figure 18a, at 11 days after administration, the more excellent tumor growth inhibition ability was observed in the group administered with T3.01m and anti-PD-1 antibody in combination compared to the control, the group administered with T3.01m, and the group administered with aPD-1. These results indicate that when T3.01m, a fusion protein of anti-FAP and LIGHT, is administered in combination with an anti-PD-1 antibody, a synergistic effect is remarkable in a mouse model of colorectal cancer.

In addition, it was confirmed, through analysis of the distribution of immune cells in tumor tissue, whether the above effects were anti-cancer effects resulting from intratumoral infiltration of immune cells by administration of the fusion protein and the anti-PD-1 antibody in combination.

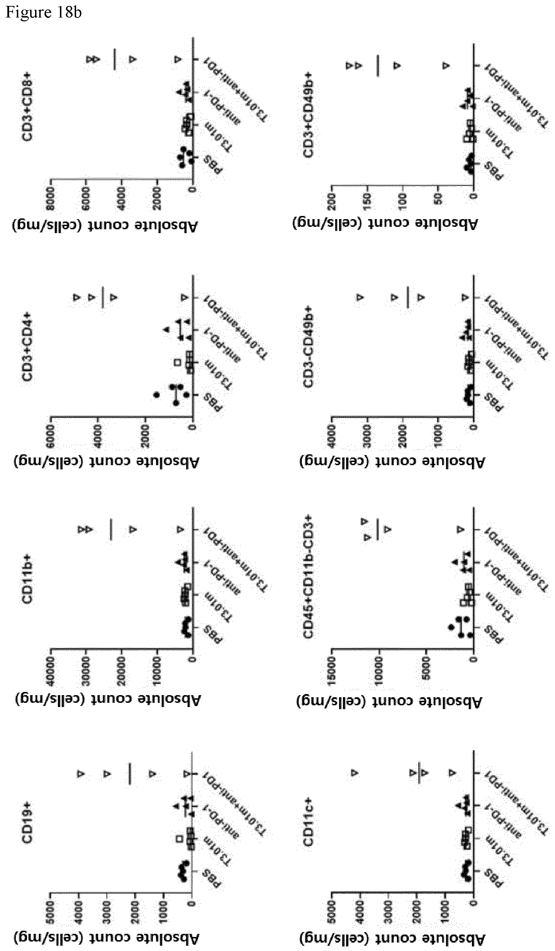

As a result, as shown in Figure 18b, it was found that the absolute number of immune cells, CD19+ cells, CD11b+ cells, CD3+ cells, CD4+ cells, CD8+ cells, CD11c+ cells, CD3-CD49b+ cells, and CD3+CD49b+ cells, was increased in the group administered with the fusion protein and the anti-PD-1 antibody in combination. These results indicate that the above results are anti-cancer effects resulting from increased infiltration of immune cells by administration of the fusion protein and the anti-PD-1 antibody in combination.

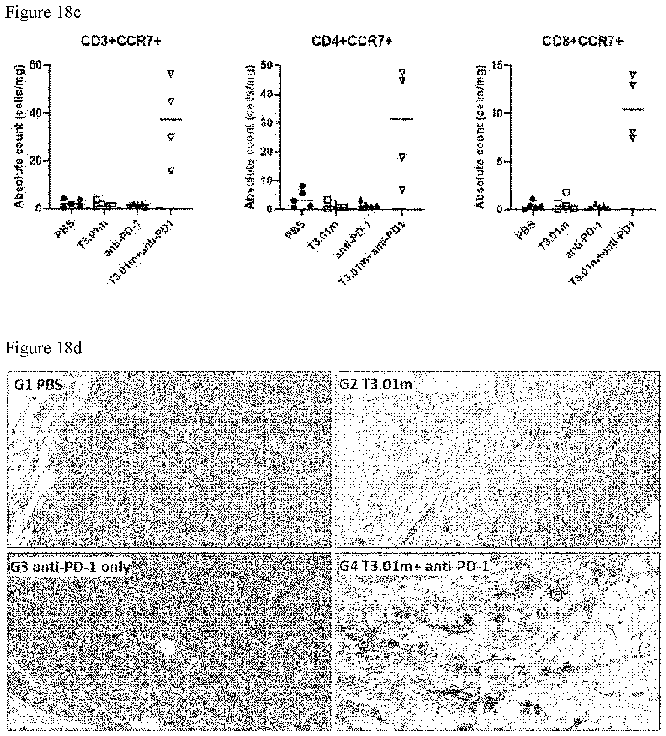

In addition, as shown in Figure 18c, the analysis of the distribution of CCR7+ cells in immune cells in tumor tissues implied that the above effects were due to the formation of high endothelial venules (HEV) by the fusion protein, which induced chemotaxis and increased infiltration of CCR7+ cells.

Next, in the above experimental groups, tumor tissues were harvested from three mice in each group on the 11th day after the first intraperitoneal injection using a treatment method according to the IACUC regulations. The harvested tumor tissues were fixed in 10% neutral buffered formalin for 24 hours and then dehydrated in a Vacuum Tissue Processor (HistoCorePEARL, Leica), and then FFPE blocks were prepared in a Tissue embedding center (EG1150, Leica). In order to confirm immune cell infiltration and HEV formation in tumor tissues, immunohistochemistry (IHC) was performed. The tissues were stained with anti-mouse CD3 antibody (Abcam), anti-mouse CD19 antibody (Cell signaling), and anti-mouse MECA-79 antibody (Thermo), respectively.

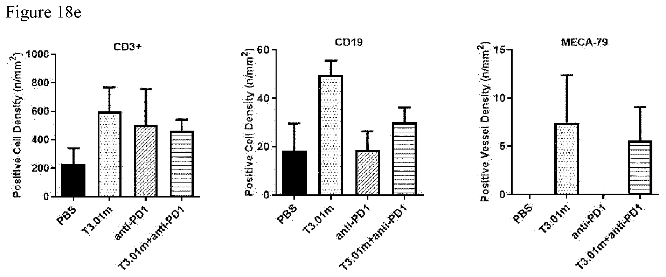

As a result, as shown in Figure 18d, it was found that HEV formation was induced by the fusion protein. When the IHC results were quantified and displayed as a graph (Figure 18e), it was found that HEV formation and immune cell infiltration increased in the group treated with the fusion protein. These results indicate that the anti-cancer effect of the fusion protein is due to increased immune cell infiltration by inducing the formation of tertiary lymphoid structures (TLS).

### Example 4.2. Evaluation of dose dependent anti-cancer efficacy in a FAP-expressing colorectal cancer animal model

In order to construct a FAP-expressing tumor animal model, mouse FAP was overexpressed in CT26, a mouse colorectal cancer cell line. Specifically, the CT26-mFAP cell line overexpressing mouse FAP in culture was resuspended in Hanks' Balanced salt solution (HBSS, Gibco), and then 1 x 10⁶ tumor cells were transplanted into the left dorsal subcutaneous region of 6-week-old BALB/c mice using a 1 cc syringe (25G) at 100 µL per mouse to obtain a FAP-expressing colorectal cancer animal model. The tumor size was measured along the short axis and the long axis of the tumor using a digital vernier caliper, and the tumor size was measured using the calculation formula: (short axis, mm)² x (long axis, mm) x 0.5.

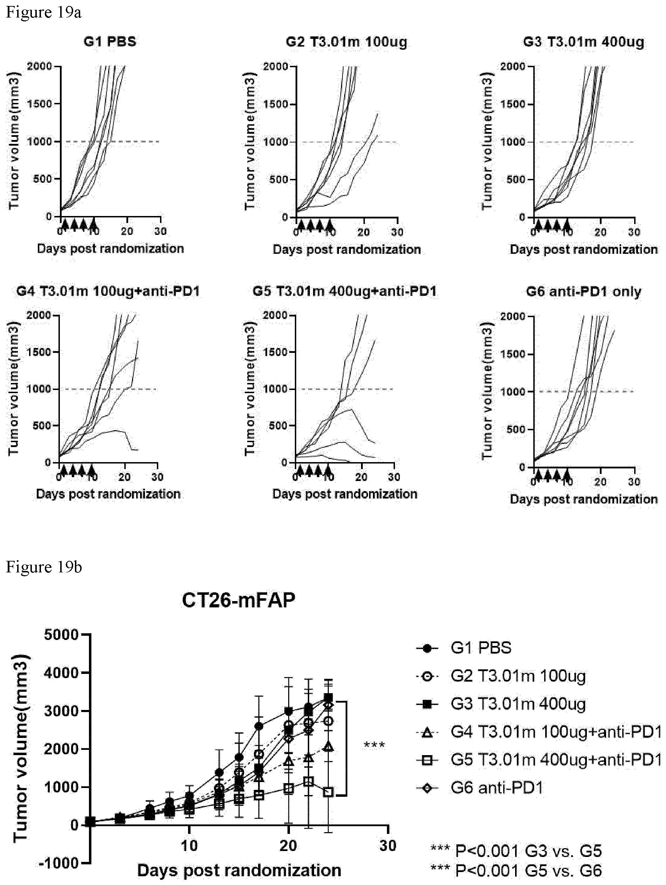

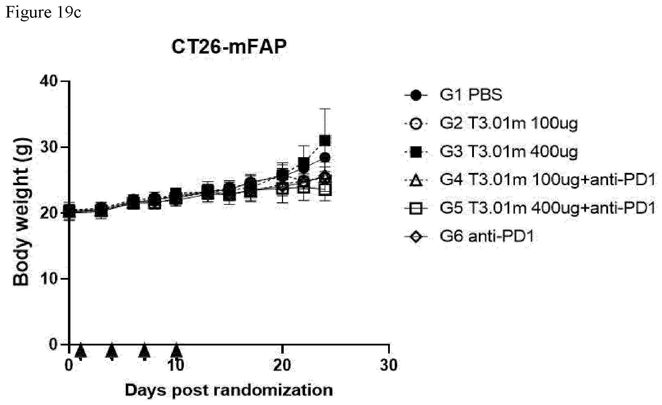

Next, for efficacy evaluation, when the tumor size reached 100 mm³, the mice were randomly divided into groups. Each experimental group consisted of 7 mice, and PBS was administered intraperitoneally once every 3 days for a total of 4 times as a control group. The group administered with 100 µg of T3.01m alone, the group administered with 400 µg of T3.01m alone, the group administered with 200 µg of aPD-1 (Clone RMP1-14), an immune checkpoint inhibitor, alone, the group administered with 100 µg of T3.01m and 200 µg of aPD-1 in combination, and the group administered with 400 µg of T3.01m and 200 µg of aPD-1 in combination were used as experimental groups to evaluate the tumor growth inhibition ability. All experimental substances were administered intraperitoneally once every 3 days for 4 times, and the tumor growth inhibition ability was observed. The tumor size and body weight were measured twice a week. The measured tumor size was displayed as a graph (Figures 19a and 19b), and it was found that tumor growth was inhibited in the group administered with the fusion protein and the anti-PD-1 antibody in combination as the dosage of the fusion protein increased. The side effects due to the experimental substance were expressed as body weight changes (Figure 19c). It was found that there were no side effects after intraperitoneal injection of the experimental substance.

### Example 4.3. Evaluation of anti-cancer efficacy of the fusion protein in a melanoma animal model

In order to confirm the anti-cancer effect of the fusion protein through the induction of tertiary lymphoid structure (TLS) formation, mouse FAP was overexpressed in B16F10, a mouse melanoma cell line known to have low immune cell infiltration, and then B16F10 was transplanted into C57BL/6J mice to evaluate the tumor growth inhibition ability.

Specifically, the B16F10-mFAP cells in culture were resuspended in Hanks' Balanced salt solution (HBSS, Gibco), and then 2 x 10⁵ tumor cells were transplanted into the left dorsal subcutaneous region of 6-week-old C57BL/6J mice at 100 µL per mouse.

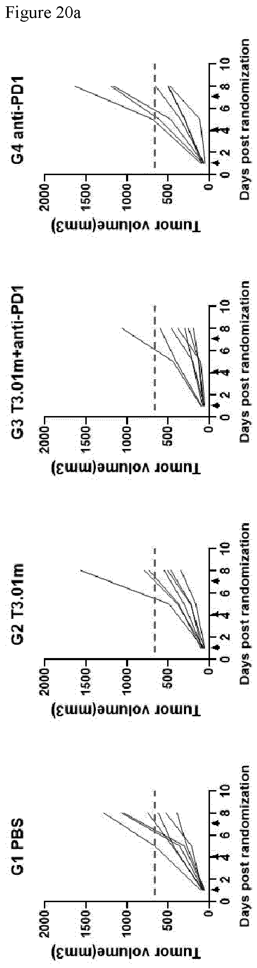

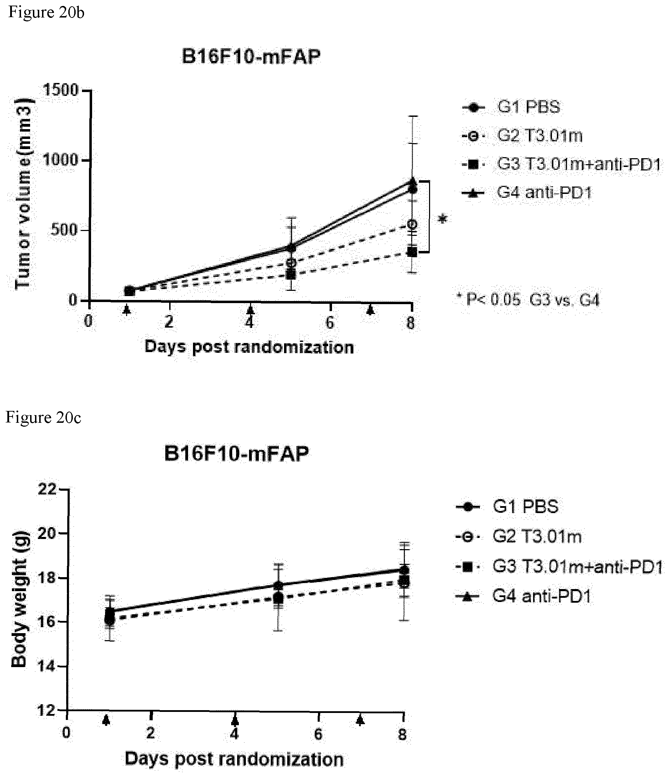

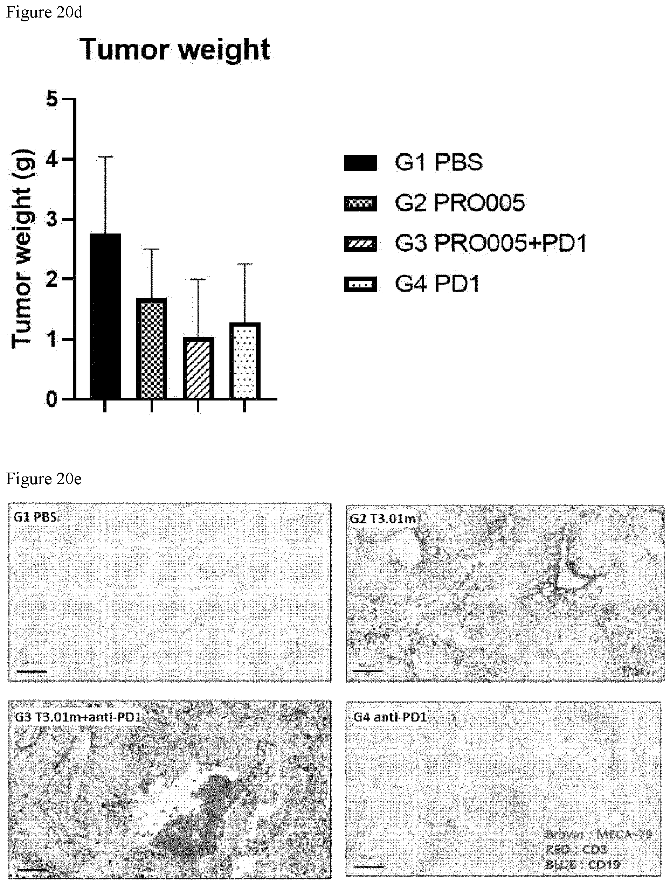

Next, for efficacy evaluation, when the tumor size reached 100 mm³, the mice were randomly divided into groups. Each experimental group consisted of 7 mice, and PBS was administered intraperitoneally once every 3 days for a total of 4 times as a control group. The group administered with 300 µg of T3.01m alone, the group administered with 200 µg of aPD-1 (Clone RMP1-14), an immune checkpoint inhibitor, alone, and the group administered with 300 µg of T3.01m and 200 µg of aPD-1 in combination were used as experimental groups to evaluate the tumor growth inhibition ability. All experimental substances were administered intraperitoneally once every 3 days for 4 times, and the tumor growth inhibition ability was observed. The tumor size and body weight were measured twice a week. The measured tumor size was displayed as a graph (Figures 20a and 20b), and it was found that tumor growth was inhibited in the group administered with the fusion protein and the anti-PD-1 antibody in combination. The side effects due to the experimental substance were expressed as body weight changes (Figure 20c). It was found that there were no side effects after intraperitoneal injection of the experimental substance. On the 13th day after intraperitoneal injection of the experimental substance, the tumor tissues from the subjects excluding the subjects that had terminated according to the humane termination point were isolated, and the relative weights by group were compared (Figure 20d). It was found that the tumor tissue weight was reduced in the group administered with the fusion protein and the anti-PD-1 antibody in combination, and it has been shown to have excellent anti-cancer effects. The harvested tumor tissue was fixed in 10% neutral buffered formalin.

Next, in order to confirm immune cell infiltration and HEV formation in tumor tissues, immunohistochemistry was performed. The tissues were simultaneously stained with anti-mouse CD3 antibody (Abcam), anti-mouse CD19 antibody (Cell signaling), and anti-mouse MECA-79 antibody (Thermo).

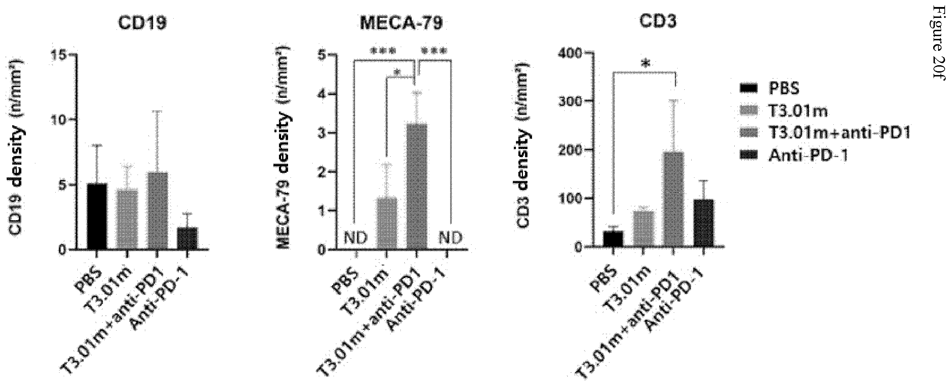

As a result, as shown in Figure 20e, it was found that HEV formation was induced by the fusion protein. When the IHC results were quantified and displayed as a graph (Figure 20f), it was found that HEV formation and immune cell infiltration increased in the group treated with the fusion protein and the anti-PD-1 antibody in combination. These results indicate that the anti-cancer effect of the fusion protein is due to increased immune cell infiltration by inducing the formation of tertiary lymphoid structures in a model with low immune cell infiltration.

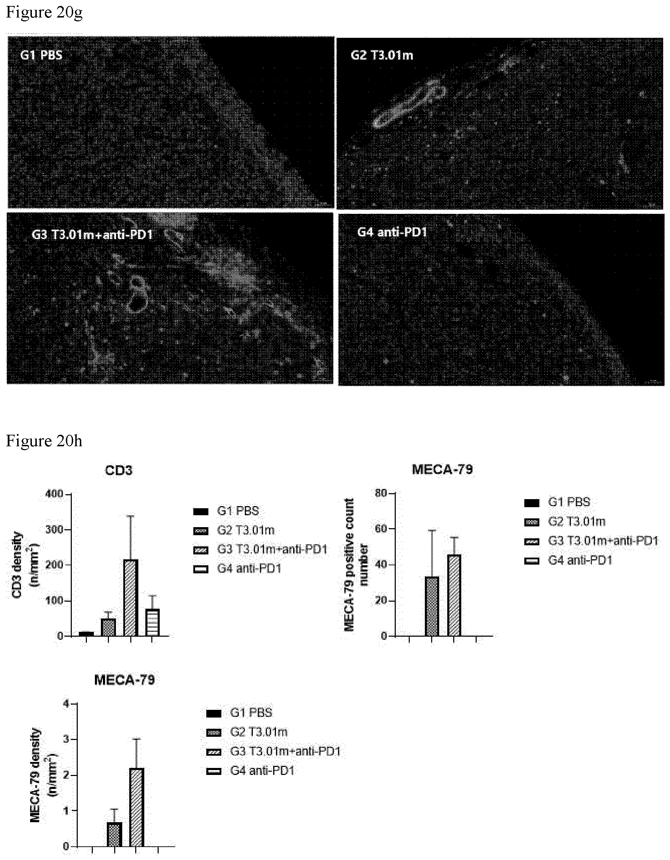

Next, in order to confirm immune cell infiltration and HEV formation in tumor tissues, fluorescent immunohistochemical staining (immunofluorescence, IF) was performed. The tissues were simultaneously fluorescently stained with anti-mouse CD3 antibody (Abcam) and anti-mouse MECA-79 antibody (Thermo). As a result, as shown in Figure 20g, it was found that HEV formation was induced by the fusion protein. When the IF results were quantified and displayed as a graph (Figure 20h), it was found that HEV formation and T cell infiltration increased in the group treated with the fusion protein and the anti-PD-1 antibody in combination. These results indicate that the anti-cancer effect of the fusion protein is due to increased immune cell infiltration by inducing the formation of tertiary lymphoid structures in a model with low immune cell infiltration.

### Example 4.4. Evaluation of synergistic efficacy of cancer targeting in a lung cancer animal model co-injected with fibroblasts

In order to compare the anti-cancer efficacy of a bispecific antibody-type fusion protein in which anti-FAP sequence structure and LIGHT sequence structure form a knob-into-hole structure according to cancer targeting, the tumor growth inhibition ability was confirmed in an animal model in which NIH-3T3-mFAP cells, a mouse fibroblast cell line that overexpresses mouse FAP, and LLC1, a mouse lung cancer cell line, were co-injected at a 1:1 ratio.

Specifically, the NIH-3T3-mFAP cell line overexpressing mouse FAP in culture and the LLC1 cell line were each resuspended in Hanks' Balanced salt solution at a concentration of 5 x 10⁵ cells/50 µL, and the cells were mixed at a 1:1 ratio and then were transplanted into the left dorsal subcutaneous region of 6-week-old C57BL/6J mice at 100 µL per mouse.

Next, for efficacy evaluation, when the tumor size reached 70 to 100 mm³, the mice were randomly divided into groups. Each experimental group consisted of 7 mice, and PBS was administered intraperitoneally once every 3 days for a total of 5 times as a control group. The group administered with T3.13m, T3.11m, and aPD-1 in combination; the group administered with T3.13m and aPD-1 in combination; the group administered with T3.01m and aPD-1 in combination; and the group administered with aPD-1 were used as experimental groups to evaluate the tumor growth inhibition ability. All experimental substances were administered intraperitoneally once every 3 days for 5 times, and the tumor growth inhibition ability was observed. The tumor size and body weight were measured twice a week.

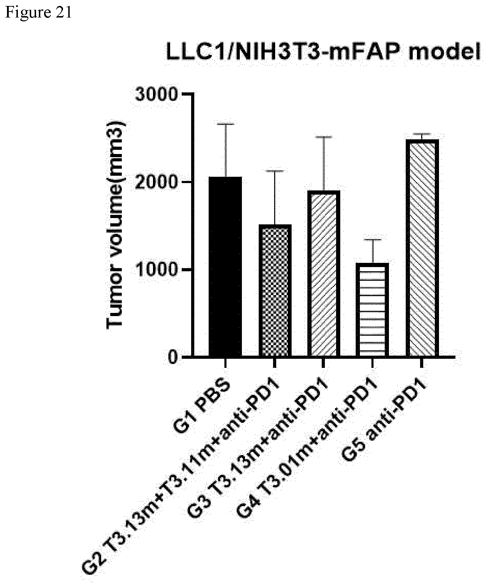

As a result, as shown in Figure 21, at 15 days after administration, excellent tumor growth inhibition ability was observed in the group administered with T3.01m and aPD-1 in combination compared to the control, and excellent tumor growth inhibition ability was confirmed in the group administered with T3.01m and aPD-1 in combination, compared to the group administered with T3.13m and aPD-1 in combination and the group administered with T3.13m, T3.11m, and aPD-1 in combination. These results indicate that the fusion protein of one embodiment has a remarkable synergistic effect when compared to when only targeting FAP or when compared to LIGHT without cancer targeting.

## Claims

1. A fusion protein comprising an antigen binding domain that specifically binds to FAP (fibroblast activation protein alpha); and a LIGHT protein, a fragment thereof, or a variant thereof.

2. The fusion protein according to claim 1, wherein the fusion protein comprises a first monomer comprising an antigen binding domain that specifically binds to FAP; and a second monomer comprising a LIGHT protein, a fragment thereof, or a variant thereof.

3. The fusion protein according to claim 2, wherein the first monomer further comprises a LIGHT protein, a fragment thereof, or a variant thereof.

4. The fusion protein according to claim 2, wherein the second monomer further comprises an antigen binding domain that specifically binds to FAP.

5. The fusion protein according to claim 2, wherein the LIGHT protein or a fragment thereof comprises the amino acid sequence of SEQ ID NO: 78 or SEQ ID NO: 93.

6. The fusion protein according to claim 1, wherein the variant of the LIGHT protein comprises an amino acid sequence in which at least one amino acid selected from the group consisting of the 118th, 119th, 195th, 196th, 198th, and 226th to 23 1st amino acids in the amino acid sequence of SEQ ID NO: 78 is substituted with another amino acid; or
an amino acid sequence in which at least one amino acid selected from the group consisting of the 194th, 195th, 197th, and 225th to 230th amino acids in the amino acid sequence of SEQ ID NO: 93 is substituted with another amino acid.

7. The fusion protein according to claim 1, wherein the variant of the LIGHT protein comprises at least one amino acid sequence selected from the group consisting of SEQ ID NO: 81, SEQ ID NO: 83, SEQ ID NO: 85, SEQ ID NO: 96, and SEQ ID NO: 98.

8. The fusion protein according to claim 1, wherein the variant of the LIGHT protein is a homomultimer of the LIGHT protein or a fragment thereof.

9. The fusion protein according to claim 8, wherein the homomultimer comprises at least one amino acid sequence selected from the group consisting of SEQ ID NO: 79, SEQ ID NO: 80, SEQ ID NO: 82, SEQ ID NO: 84, SEQ ID NO: 86, SEQ ID NO: 97, and SEQ ID NO: 99.

10. The fusion protein according to claim 1, wherein the LIGHT protein, a fragment thereof, or a variant thereof is additionally bound to an LT (lymphotoxin) protein or a variant thereof.

11. The fusion protein according to claim 10, wherein the LT protein or a variant thereof comprises an amino acid sequence selected from the group consisting of SEQ ID NO: 88 and SEQ ID NO: 290.

12. The fusion protein according to claim 10, wherein the LT protein or a variant thereof is a homodimer.

13. The fusion protein according to claim 12, wherein the homodimer of the LT protein or a variant thereof comprises the amino acid sequence of SEQ ID NO: 288 or SEQ ID NO: 291.

14. The fusion protein according to claim 1, wherein the antigen binding domain that specifically binds to FAP comprises
a heavy chain variable region comprising HCDR1 of SEQ ID NO: 101, HCDR2 of SEQ ID NO: 102, and HCDR3 of SEQ ID NO: 103; and a light chain variable region comprising LCDR1 of SEQ ID NO: 104, LCDR2 of SEQ ID NO: 105, and LCDR3 of SEQ ID NO: 106;
a heavy chain variable region comprising HCDR1 of SEQ ID NO: 109, HCDR2 of SEQ ID NO: 110, and HCDR3 of SEQ ID NO: 111; and a light chain variable region comprising LCDR1 of SEQ ID NO: 112, LCDR2 of SEQ ID NO: 113, and LCDR3 of SEQ ID NO: 114;
a heavy chain variable region comprising HCDR1 of SEQ ID NO: 127, HCDR2 of SEQ ID NO: 128, and HCDR3 of SEQ ID NO: 129; and a light chain variable region comprising LCDR1 of SEQ ID NO: 130, LCDR2 of SEQ ID NO: 131, and LCDR3 of SEQ ID NO: 132;
a heavy chain variable region comprising HCDR1 of SEQ ID NO: 133, HCDR2 of SEQ ID NO: 134, and HCDR3 of SEQ ID NO: 135; and a light chain variable region comprising LCDR1 of SEQ ID NO: 136, LCDR2 of SEQ ID NO: 137, and LCDR3 of SEQ ID NO: 138;
a heavy chain variable region comprising HCDR1 of SEQ ID NO: 139, HCDR2 of SEQ ID NO: 140, and HCDR3 of SEQ ID NO: 141; and a light chain variable region comprising LCDR1 of SEQ ID NO: 142, LCDR2 of SEQ ID NO: 143, and LCDR3 of SEQ ID NO: 144;
a heavy chain variable region comprising HCDR1 of SEQ ID NO: 145, HCDR2 of SEQ ID NO: 146, and HCDR3 of SEQ ID NO: 147; and a light chain variable region comprising LCDR1 of SEQ ID NO: 148, LCDR2 of SEQ ID NO: 149, and LCDR3 of SEQ ID NO: 150;
a heavy chain variable region comprising HCDR1 of SEQ ID NO: 151, HCDR2 of SEQ ID NO: 152, and HCDR3 of SEQ ID NO: 153; and a light chain variable region comprising LCDR1 of SEQ ID NO: 154, LCDR2 of SEQ ID NO: 155, and LCDR3 of SEQ ID NO: 156;
a heavy chain variable region comprising HCDR1 of SEQ ID NO: 157, HCDR2 of SEQ ID NO: 158, and HCDR3 of SEQ ID NO: 159; and a light chain variable region comprising LCDR1 of SEQ ID NO: 160, LCDR2 of SEQ ID NO: 161, and LCDR3 of SEQ ID NO: 162;
a heavy chain variable region comprising HCDR1 of SEQ ID NO: 163, HCDR2 of SEQ ID NO: 164, and HCDR3 of SEQ ID NO: 165; and a light chain variable region comprising LCDR1 of SEQ ID NO: 166, LCDR2 of SEQ ID NO: 167, and LCDR3 of SEQ ID NO: 168;
a heavy chain variable region comprising HCDR1 of SEQ ID NO: 169, HCDR2 of SEQ ID NO: 170, and HCDR3 of SEQ ID NO: 171; and a light chain variable region comprising LCDR1 of SEQ ID NO: 172, LCDR2 of SEQ ID NO: 173, and LCDR3 of SEQ ID NO: 174;
a heavy chain variable region comprising HCDR1 of SEQ ID NO: 175, HCDR2 of SEQ ID NO: 176, and HCDR3 of SEQ ID NO: 177; and a light chain variable region comprising LCDR1 of SEQ ID NO: 178, LCDR2 of SEQ ID NO: 179, and LCDR3 of SEQ ID NO: 180;
a heavy chain variable region comprising HCDR1 of SEQ ID NO: 181, HCDR2 of SEQ ID NO: 182, and HCDR3 of SEQ ID NO: 183; and a light chain variable region comprising LCDR1 of SEQ ID NO: 184, LCDR2 of SEQ ID NO: 185, and LCDR3 of SEQ ID NO: 186;
a heavy chain variable region comprising HCDR1 of SEQ ID NO: 187, HCDR2 of SEQ ID NO: 188, and HCDR3 of SEQ ID NO: 189; and a light chain variable region comprising LCDR1 of SEQ ID NO: 190, LCDR2 of SEQ ID NO: 191, and LCDR3 of SEQ ID NO: 192;
a heavy chain variable region comprising HCDR1 of SEQ ID NO: 193, HCDR2 of SEQ ID NO: 194, and HCDR3 of SEQ ID NO: 195; and a light chain variable region comprising LCDR1 of SEQ ID NO: 196, LCDR2 of SEQ ID NO: 197, and LCDR3 of SEQ ID NO: 198;
a heavy chain variable region comprising HCDR1 of SEQ ID NO: 199, HCDR2 of SEQ ID NO: 200, and HCDR3 of SEQ ID NO: 201; and a light chain variable region comprising LCDR1 of SEQ ID NO: 202, LCDR2 of SEQ ID NO: 203, and LCDR3 of SEQ ID NO: 204;
a heavy chain variable region comprising HCDR1 of SEQ ID NO: 205, HCDR2 of SEQ ID NO: 206, and HCDR3 of SEQ ID NO: 207; and a light chain variable region comprising LCDR1 of SEQ ID NO: 208, LCDR2 of SEQ ID NO: 209, and LCDR3 of SEQ ID NO: 210;
a heavy chain variable region comprising HCDR1 of SEQ ID NO: 211, HCDR2 of SEQ ID NO: 212, and HCDR3 of SEQ ID NO: 213; and a light chain variable region comprising LCDR1 of SEQ ID NO: 214, LCDR2 of SEQ ID NO: 215, and LCDR3 of SEQ ID NO: 216;
a heavy chain variable region comprising HCDR1 of SEQ ID NO: 217, HCDR2 of SEQ ID NO: 218, and HCDR3 of SEQ ID NO: 219; and a light chain variable region comprising LCDR1 of SEQ ID NO: 220, LCDR2 of SEQ ID NO: 221, and LCDR3 of SEQ ID NO: 222;
a heavy chain variable region comprising HCDR1 of SEQ ID NO: 223, HCDR2 of SEQ ID NO: 224, and HCDR3 of SEQ ID NO: 225; and a light chain variable region comprising LCDR1 of SEQ ID NO: 226, LCDR2 of SEQ ID NO: 227, and LCDR3 of SEQ ID NO: 228;
a heavy chain variable region comprising HCDR1 of SEQ ID NO: 229, HCDR2 of SEQ ID NO: 230, and HCDR3 of SEQ ID NO: 231; and a light chain variable region comprising LCDR1 of SEQ ID NO: 232, LCDR2 of SEQ ID NO: 233, and LCDR3 of SEQ ID NO: 234;
a heavy chain variable region comprising HCDR1 of SEQ ID NO: 235, HCDR2 of SEQ ID NO: 236, and HCDR3 of SEQ ID NO: 237; and a light chain variable region comprising LCDR1 of SEQ ID NO: 238, LCDR2 of SEQ ID NO: 239, and LCDR3 of SEQ ID NO: 240;
or
a heavy chain variable region comprising HCDR1 of SEQ ID NO: 241, HCDR2 of SEQ ID NO: 242, and HCDR3 of SEQ ID NO: 243; and a light chain variable region comprising LCDR1 of SEQ ID NO: 244, LCDR2 of SEQ ID NO: 245, and LCDR3 of SEQ ID NO: 246.

15. The fusion protein according to claim 1, wherein the antigen binding domain that specifically binds to FAP comprises
a heavy chain variable region of SEQ ID NO: 107 and a light chain variable region of SEQ ID NO: 108;
a heavy chain variable region of SEQ ID NO: 115 and a light chain variable region of SEQ ID NO: 116;
a heavy chain variable region of SEQ ID NO: 248 and a light chain variable region of SEQ ID NO: 249;
a heavy chain variable region of SEQ ID NO: 250 and a light chain variable region of SEQ ID NO: 251;
a heavy chain variable region of SEQ ID NO: 252 and a light chain variable region of SEQ ID NO: 253;
a heavy chain variable region of SEQ ID NO: 254 and a light chain variable region of SEQ ID NO: 255;
a heavy chain variable region of SEQ ID NO: 256 and a light chain variable region of SEQ ID NO: 257;
a heavy chain variable region of SEQ ID NO: 258 and a light chain variable region of SEQ ID NO: 259;
a heavy chain variable region of SEQ ID NO: 260 and a light chain variable region of SEQ ID NO: 261;
a heavy chain variable region of SEQ ID NO: 262 and a light chain variable region of SEQ ID NO: 263;
a heavy chain variable region of SEQ ID NO: 264 and a light chain variable region of SEQ ID NO: 265;
a heavy chain variable region of SEQ ID NO: 266 and a light chain variable region of SEQ ID NO: 267;
a heavy chain variable region of SEQ ID NO: 268 and a light chain variable region of SEQ ID NO: 269;
a heavy chain variable region of SEQ ID NO: 270 and a light chain variable region of SEQ ID NO: 271;
a heavy chain variable region of SEQ ID NO: 272 and a light chain variable region of SEQ ID NO: 273;
a heavy chain variable region of SEQ ID NO: 274 and a light chain variable region of SEQ ID NO: 275;
a heavy chain variable region of SEQ ID NO: 276 and a light chain variable region of SEQ ID NO: 277;
a heavy chain variable region of SEQ ID NO: 278 and a light chain variable region of SEQ ID NO: 279;
a heavy chain variable region of SEQ ID NO: 280 and a light chain variable region of SEQ ID NO: 281;
a heavy chain variable region of SEQ ID NO: 282 and a light chain variable region of SEQ ID NO: 283;
a heavy chain variable region of SEQ ID NO: 284 and a light chain variable region of SEQ ID NO: 285; or
a heavy chain variable region of SEQ ID NO: 286 and a light chain variable region of SEQ ID NO: 287.

16. The fusion protein according to claim 2, wherein the first monomer is composed of the following structural formula (I) or (II):
N'-X-[linker (3)]q-Fc domain-[linker (4)]r-(Y)c-C' (I)
N'-(Y)d-[linker (3)]q-Fc domain-[linker (4)]r-X-C' (II)
in the structural formulas (I) and (II),
N' is the N-terminus of the fusion protein,
C' is the C-terminus of the fusion protein,
X is the antigen binding domain that specifically binds to FAP,
Y is the LIGHT protein, a fragment thereof, or a variant thereof,
the linkers (3) and (4) are peptide linkers, and
c, d, q, and r are each independently 0 or 1.

17. The fusion protein according to claim 2, wherein the second monomer is composed of the following structural formula (III) or (IV):
N'-(X)e-[linker (5)]s-Fc domain-[linker (6)]t-Y'-C' (III)
N'-Y'-[linker (5)]s-Fc domain-[linker (6)]t-(X)f-C' (IV)
in the structural formulas (III) and (IV),
N' is the N-terminus of the fusion protein,
C' is the C-terminus of the fusion protein,
X is the antigen binding domain that specifically binds to FAP,
Y' is the LIGHT protein, a fragment thereof, or a variant thereof; or a heteromultimer of the LIGHT protein and the LT protein,
the linkers (5) and (6) are peptide linkers, and
e, f, s, and t are each independently 0 or 1.

18. The fusion protein according to claim 16 or 17, wherein the linker comprises a (G4S)n, GGGS(G4S)n, GGS(G3S)n, or GGSG(G3S)n linker, and n is an integer from 0 to 10.

19. The fusion protein according to claim 18, wherein the linker comprises at least one amino acid sequence selected from the group consisting of SEQ ID NO: 117 to SEQ ID NO: 126.

20. The fusion protein according to claim 16 or 17, wherein the Fc region is derived from human IgG1 or mouse IgG2a.

21. The fusion protein according to claim 20, wherein the Fc region comprises an amino acid sequence selected from the group consisting of SEQ ID NO: 14, SEQ ID NO: 15, SEQ ID NO: 32 to SEQ ID NO: 34, and SEQ ID NO: 292 to SEQ ID NO: 298.

22. The fusion protein according to claim 2, wherein the first monomer comprises a heavy chain consisting of the amino acid sequence of SEQ ID NO: 75 and a light chain consisting of the amino acid sequence of SEQ ID NO: 2; or a heavy chain consisting of the amino acid sequence of SEQ ID NO: 76 and a light chain consisting of the amino acid sequence of SEQ ID NO: 23.

23. The fusion protein according to claim 2, wherein the fusion protein is composed of a first monomer of structural formula (I); and a second monomer of structural formula (III).

24. The fusion protein according to claim 23, wherein in the structural formula (I), q, r and c are 1, and in the structural formula (III), e, s, and t are 1.

25. The fusion protein according to claim 2, wherein the fusion protein is composed of a first monomer of structural formula (I); and a second monomer of structural formula (IV).

26. The fusion protein according to claim 25, wherein in the structural formula (I), q is 1 and r and c are 0, and in the structural formula (IV), s is 1 and t and f are 0.

27. A pharmaceutical composition for preventing or treating cancer, comprising the fusion protein according to any one of claims 1 to 26 as an active ingredient.

28. The pharmaceutical composition for preventing or treating cancer according to claim 27, wherein the cancer is any one selected from the group consisting of gastric cancer, liver cancer, lung cancer, colorectal cancer, breast cancer, prostate cancer, skin cancer, bone cancer, multiple myeloma, glioma, ovarian cancer, pancreatic cancer, cervical cancer, thyroid cancer, laryngeal cancer, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer, and lymphoma.

29. The pharmaceutical composition for preventing or treating cancer according to claim 27, wherein the pharmaceutical composition further comprises an anti-PD-1 antibody.

30. A polynucleotide encoding the first monomer of structural formula (I) of claim 16.

31. A polynucleotide encoding the first monomer of structural formula (II) of claim 16.

32. A polynucleotide encoding the second monomer of structural formula (III) of claim 17.

33. A polynucleotide encoding the second monomer of structural formula (IV) of claim 17.

34. A vector comprising the polynucleotide according to any one of claims 30 to 33.

35. A vector comprising the polynucleotide according to claim 33.

36. A transformed cell into which the vector according to claim 35 has been introduced.

37. A method of producing a fusion protein, comprising the steps of:
i) culturing the transformed cell according to claim 36; and
ii) recovering a fusion protein comprising an antigen binding domain that specifically binds to FAP; and a LIGHT protein, a fragment thereof, or a variant thereof.

38. A method for preventing or treating cancer, comprising administering the pharmaceutical composition according to claim 27 to a subject.

39. A use of the fusion protein according to any one of claims 1 to 26 for the prevention or treatment of cancer.
